(19) Europäisches Patentamt
European Patent Office
Office européen des brevets

(11) EP 0 512 901 B1

(12) **FASCICULE DE BREVET EUROPEEN**

(45) Date de publication et mention de la délivrance du brevet:
**23.06.1999 Bulletin 1999/25**

(21) Numéro de dépôt: **92401235.4**

(22) Date de dépôt: **30.04.1992**

(51) Int. Cl.[6]: **C07D 211/76**, C07D 211/52, C07D 211/26, C07D 211/22, C07D 405/12, C07D 401/06, C07D 207/08, A61K 31/445

(54) **Composés polycycliques aminés et leurs énantiomères, procédé pour leur préparation et compositions pharmaceutiques en contenant**

Aminierte polyzyklische Verbindung und ihre Enantiomere, Verfahren zu ihrer Herstellung und diese enthaltende pharmazeutische Zusammensetzungen

Aminated polycyclic compounds and their enantiomers, process for their preparation and pharmaceutical compositions containing them

(84) Etats contractants désignés:
**AT BE CH DE DK ES FR GB GR IT LI LU NL PT SE**

(30) Priorité: **03.05.1991 FR 9105487**

(43) Date de publication de la demande:
**11.11.1992 Bulletin 1992/46**

(73) Titulaire: **SANOFI**
**75013 Paris (FR)**

(72) Inventeurs:
- **Goulaouic, Pierre**
  **décédé (FR)**
- **Emonds-Alt, Xavier**
  **F-34980 Combaillaux (FR)**
- **Gueule, Patrick**
  **F-34820 Teyran (FR)**
- **Proietto, Vincenzo**
  **F-34680 St Georges d'Orques (FR)**

(74) Mandataire:
**Gillard, Marie-Louise et al**
**Cabinet Beau de Loménie**
**158, rue de l'Université**
**75340 Paris Cédex 07 (FR)**

(56) Documents cités:
**EP-A- 0 347 802**
**EP-A- 0 412 542**
**DE-A- 2 345 192**
**GB-A- 2 056 439**
**US-A- 3 718 743**

- **CHEMISCHE BERICHTE vol. 108, 1975, pages 3475 - 3482; H. BOCHOW ET AL: '4-Phenyl-2-chinuclidinol'**

EP 0 512 901 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

## Description

La présente invention a pour objet de nouveaux dérivés aromatiques substitués par un groupement amino et par des fonctions amines ou amides, ainsi que leurs énantiomères.

La présente invention concerne également le procédé d'obtention des composés, qui peut être énantiosélectif et l'utilisation des composés selon l'invention dans des compositions à usage thérapeutique et plus particulièrement dans les phénomènes pathologiques qui impliquent le système des neurokinines comme : la douleur (D. Regoli et al., Life Sciences, 1987, 40, 109-117), l'allergie et l'inflammation (J,E. Morlay et al., Life Sciences, 1987, 41) 527-544), l'insuffisance circulatoire (J. Losay et al., 1977, Substance P, Von Euler, U.S. and Pernow ed., 287-293, Raven Press, New York), les troubles gastro-intestinaux (D. Regoli ct al., Trends Pharmacol. Sci., 1985, 6, 481-484), les troubles respiratoires (J. Mizrahi et al., Pharmacology, 1982, 25, 39-50).

Des ligands endogènes aux récepteurs des neurokinines ont été décrits, telles la substance P (SP), la neurokinine A ($NK_A$) (S.J. Bailey et al., 1983, Substance P, P. Skrabanck cd., 16-17 Boole Press, Dublin) et la neurokinine B ($NK_B$) (S.P. Watson, Life Sciences, 1983, 25, 797-808).

Les récepteurs aux neurokinines ont été reconnus sur de nombreuses préparations et sont actuellement classés en trois types , $NK_1$, $NK_2$ et $NK_3$. Alors que la plupart des préparations étudiées jusqu'à maintenant, présentent plusieurs types de récepteurs, tel l'iléon de cobaye ($NK_1$, $NK_2$ et $NK_3$), certaines d'entre elles n'en possèderaient qu'un seul, telles l'artère carotide de chien ($NK_1$), l'artère pulmonaire de lapin dépourvue d'endothélium ($NK_2$) et la veine porte de rat ($NK_3$) (D. Regoli et al., Trends Pharmacol. Sci., 1988, 9, 290-295 et Pharmarcology, 1989, 38, 1-15).

Une caractérisation plus précise des différents récepteurs est rendue possible par la synthèse récente d'agonistes sélectifs. Ainsi, la [$Sar^9$, Met-$(O_2)^{11}$] SP, la [$Nle^{10}$] $NKA_{4-10}$, et la [Me $Phe^7$] -$NK_B$ présenteraient une sélectivité respective pour les récepteurs $NK_1$, $NK_2$ et $NK_3$ (cf D. Regoli, 1988 et 1989 précédemment cité).

On a maintenant trouvé que certains composés aromatiques aminés possèdent des propriétés pharmacologiques intéressantes, en tant qu'antagonistes des recepteurs des neurokinines et sont notamment utiles pour le traitement de toute pathologie substance P et neurokinine dépendante.

Ainsi selon un de ses aspects, la présente invention concerne des dérivés aromatiques aminés de formule :

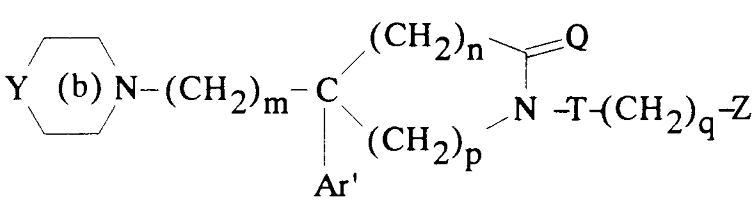

(I)

dans laquelle

- Y représente
  - soit un groupe Cy-N ou Cy-$CH_2$-N dans lequel
    . Cy représente un phényle non substitué ou substitué une ou plusieurs fois par un des substituants choisis parmi : un atome d'halogène, un hydroxyle, un alcoxy en $C_1$-$C_4$, un alkyle en $C_1$-$C_4$, un trifluorométhyle, lesdits substituants étant identiques ou différents ; un groupe cycloalkyle en $C_3$-$C_7$ ; un groupe pyrimidyle ou un groupe pyridyle ;
  - soit un groupe

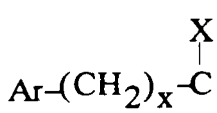

dans lequel

. Ar représente un phényle non substitué ou substitué une ou plusieurs fois par un des substituants choisis parmi : un atome d'halogène, un hydroxyle, un alcoxy en $C_1$-$C_4$, un trifluorométhyle, un alkyle en $C_1$-$C_4$,

lesdits substituants étant identiques ou différents ; un groupe pyridyle ; un groupe thiényle ;

. x est zéro ou un ;

. X représente un hydrogène, un hydroxyle, un alcoxy en $C_1$-$C_4$ ; un acyloxy en $C_1$-$C_4$ ; un carboxy ; un carbalcoxy en $C_1$-$C_4$ ; un cyano ; un groupe-$N(X_1)_2$ dans lequel les groupes $X_1$ représentent indépendamment l'hydrogène, un alkyle en $C_1$-$C_4$, un hydroxyalkyle en $C_1$-$C_4$, un acyle en $C_1$-$C_4$, ou bien -$(X_1)_2$ constitue avec l'atome d'azote auquel il est lié, un hétérocycle choisi parmi pyrrolidine, pipéridine ou morpholine ; un groupe-S-$X_2$ dans lequel $X_2$ représente l'hydrogène ou un groupe alkyle en $C_1$-$C_4$;

ou bien X forme, avec l'atome de carbone auquel il est lié et avec l'atome de carbone voisin dans l'hétérocycle, une double liaison ;

- m est 2 ou 3;
- Ar' représente un phényle non substitué ou substitué une ou plusieurs fois par un des substituants choisis parmi : un atome d'halogène, de préférence un atome de chlore ou de fluor, un trifluorométhyle, un alcoxy en $C_1$-$C_4$, un alkyle en $C_1$-$C_4$, lesdits substituants étant identiques ou différents ; un thiényle ; un benzothiényle ; un naphtyle ou un indolyle ;
- n est 0,1, 2 ou 3 ;
- p est 1 ou 2 et lorsque p est égal à 2, alors n est égal à 1 et Q représente deux atomes d'hydrogène ;
- Q représente l'oxygène ou deux atomes d'hydrogène ;
- T représente un groupe choisi parmi

$$-\underset{\underset{O}{\parallel}}{C}- \quad \text{et} \quad -CH_2-$$

- q est 0,1, 2 ou 3 ;
- Z représente un groupe aromatique ou hétéroaromatique mono-, di- ou tricyclique éventuellement substitué tel que défini plus loin ; ou encore lorsque T est -C = O, -$(CH_2)_q$-Z peut aussi représenter un groupe benzyle substitué sur le -CH- par un hydroxyle, un alcoxy en $C_1$-$C_4$, un alkyle en $C_1$-$C_4$ et éventuellement substitué sur le cycle aromatique par un halogène, plus particulièrement un atome de chlore ou de fluor, un trifluorométhyle, un alkyle en $C_1$-$C_4$, un hydroxyle, un alcoxy en $C_1$-$C_4$ ;

ou un de ses sels éventuels avec des acides minéraux ou organiques, ou, lorsque

$$Y = Ar -(CH_2)_x \overset{X}{\overset{|}{C}} ,$$

un de leurs sels d'ammonium quaternaire ou dérivés N-oxyde formés avec l'azote (b) de la pipéridine.

La limitation relative à p = 2 existe du fait des contraintes de synthèse chimique. Pour les valeurs $(CH_2)_qZ$ lorsque T = CO, la limitation provient de l'accessibilité chimique des produits utilisés.

Dans la présente description les groupes alkyle ou les groupes alcoxy sont droits ou ramifiés

Les sels des composé s de formule (I) selon la présente invention comprennent aussi bien ceux avec des acides minéraux ou organiques qui permettent une séparation ou une cristallisation convenable des composés de formule (I), tels que l'acide picrique ou l'acide oxalique ou un acide optiquement actif, par exemple un acide mandélique ou camphosulfonique, que ceux qui forment des sels pharmaceutiquement acceptables tels que le chlorhydrate, le bromhydrate, le sulfate, l'hydrogénosulfate, le dihydrogénophosphate, le méthanesulfonate, le méthylsulfate, le maléate, le fumarate, le 2-naphtalènesulfonate, le glycolate, le gluconate, le citrate, l'iséthionate.

Lorsque

$$Y = Ar - (CH_2)_x - \overset{X}{\overset{|}{C}} ,$$

les composés de formule (I) peuvent se trouver sous forme d'un sel d'ammonium quaternaire formé avec l'azote (b) de

la pipéridine ou d'un dérivé N-oxyde formé avec l'azote (b). le groupe

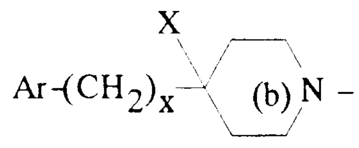

est lors représenté par
le groupe

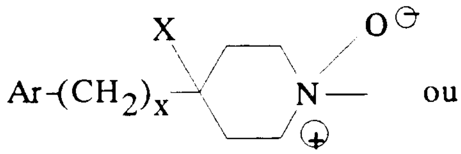

ou

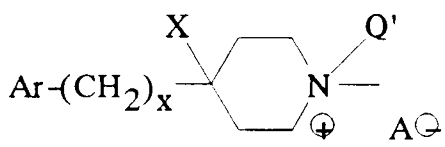

dans lequel

- . Q' représente un groupe alkyle en $C_1$-$C_6$ ou un groupe benzyle et
- . A $^{\ominus}$ représente un anion choisi parmi chlorure, bromure, iodure, acétate, méthanesulfonate ou paratoluènesulfonate.

De façon grossière, dans la formule (I), Z représente un groupe aromatique ou hétéroaromatique mono-, di- ou tricyclique, pouvant porter un ou plusieurs substituants, dont un atome de carbone du carbocycle aromatique ou de l'hétérocycle aromatique est directement lié au groupe T.

Plus particulièrement, le radical Z peut être un groupe phényle, qui peut être non substitué ou éventuellement contenir un ou plusieurs substituants.

Lorsque Z est un groupe phényle, celui-ci peut être de préférence mono- ou disubstitué notamment en position 2,4, mais aussi par exemple en position 2,3, 4,5, 3,4 ou 3,5 ; il peut aussi être trisubstitué, notamment en position 2,4,6 mais aussi par exemple en 2,3,4, 2,3,5 ou 2,4,5 3,4,5 ; tétrasubstitué, par exemple en 2,3,4,5 ; ou pentasubstitué. Les substituants du groupe phényle peuvent être : F ; Cl; Br; I; CN; OH; $NH_2$; NH-$CONH_2$;$NO_2$; $CONH_2$; $CF_3$; alkyle en $C_1$-$C_{10}$, de préférence en $C_1$-$C_4$, méthyle ou éthyle étant préférés, ainsi que par exemple n-propyle, isopropyle, n-butyle, isobutyle, sec-butyle, tert-butyle, pentyle ou n-pentyle, hexyle ou n-hexyle,heptyle ou n-heptyle, octyle ou n-octyle, nonyle ou n-nonyle ainsi que décyle ou n-décyle ; alcényle contenant 2 à 10, de préférence 2-4 atomes de carbone, par exemple vinyle, allyle, 1-propényle, isopropényle, butényle ou 1-butèn-1-, -2-, -3- ou -4-yle, 2-butèn-1-yle, 2-butèn-2-yle, pentényle, hexényle ou décényle ; alcynyle contenant 2 à 10, de préférence 2-4 atomes de carbone, par exemple éthynyle, 1-propyn-1-yle, propargyle, butynyle ou 2-butyn-1-yle, pentynyle, décynyle ; cycloalkyle contenant 3 à 8, de préférence 5 ou 6 atomes de carbone, cyclopentyle ou cyclohexyle étant préférés, ainsi que par exemple cyclopropyle, cyclobutyle, 1-, 2- ou 3-méthylcyclopentyle, 1-, 2-, 3- ou 4-méthylcyclohexyle, cycloheptyle ou cyclooctyle ; bicycloalkyle contenant 4 à 11, de préférence 7 atomes de carbone, exo ou endo 2-norbomyle étant préférés, ainsi que par exemple 2-isobornyle ou 5-camphyle ; hydroxyalkyle contenant 1 à 5, de préférence 1-2 atomes de carbone, hydroxyméthyle et 1- ou 2-hydroxyéthyle étant préférés, ainsi que par exemple 1-hydroxyprop-1-yle, 2-hydroxyprop-1-yle, 3-hydroxyprop-1-yle, 1-hydroxyprop-2-yle, 1-hydroxybut-1-yle, 1-hydroxypent-1-yle ; alcoxy contenant 1 à 10, de préférence 1-4 atomes de carbone, méthoxy, éthoxy ou isopropoxy étant préférés, ainsi que par exemple n-propoxy, n-butoxy, isobutoxy, sec-butoxy, tert-butoxy, pentyloxy, hexyloxy, heptyloxy, octyloxy, nonyloxy ou décyloxy ; alcoxyalkyle contenant 2 à 10, de préférence de 2 à 6 atomes de carbone, par exemple alcoxyméthyle ou alcoxyéthyle, tel que méthoxyméthyle ou 1- ou 2-méthoxyéthyle, 1- ou 2-n-butoxyéthyle, 1- ou 2-n-octyloxyéthyle ; alcoxyalcoxyalkyle contenant jusqu'à 10, de préférence - de 4 à 7 atomes de carbone, par exemple alcoxyalcoxyméthyle,par exemple 2-méthoxyéthoxyméthyle, 2-éthoxyéthoxyméthyle ou 2-isopropoxyéthoxyméthyle, alcoxyalcoxyéthyle par exemple 2-(2-méthoxyéthoxy)éthyle ou 2-(2-éthoxyéthoxy)éthyle ; alcoxyalcoxy contenant de 2 à 10, de préférence de 3 à 6 atomes de carbone, par exemple 2-méthoxyéthoxy, 2-éthoxyéthoxy ou 2-n-butoxyéthoxy ; alcényloxy contenant 2 à 10, de préférence 2 à 4 atomes de carbone, allyloxy étant préféré, ainsi que par exemple vinyloxy, propényloxy, isopropényloxy, buténylоxy tel que 1-butèn-1-, -2-, -3- ou -4-yloxy, 2-butèn-1-yloxy, 2-butèn-yloxy, pentènyloxy, hexényloxy ou décényloxy ; alcényloxyalkyle contenant de 3 à 10, de préférence 3-6 atomes de carbone, par exemple allyloxyméthyle ; alcynyloxy contenant de 2 à 10, de préférence 2 à 4 atomes de carbone, propargyloxy étant préférés, ainsi que par exemple éthynyloxy, 1-propyn-1-yloxy, butynyloxy ou 2-butyn-1-yloxy, pentynyloxy ou décynyloxy ; alcynyloxyalkyle contenant de 3 à 10 de préférence

3 à 6 atomes de carbone, par exemple éthynyloxyméthyle, propargyloxyméthyle ou 2-(2-butyn-1-yloxy)éthyle ; cycloalcoxy contenant 3 à 8, de préférence 5 ou 6 atomes de carbone, cyclopentyloxy ou cyclohexyloxy étant préférés, ainsi que par exemple cyclopropyloxy, cyclobutyloxy, 1-, 2- ou 3-méthylcyclopentyloxy, 1-, 2-, 3- ou 4-méthylcyclohexyloxy, cycloheptyloxy ou cyclooctyloxy ; alkylthio contenant de 1 à 10, de préférence 1 à 4 atomes de carbone, méthylthio ou éthylthio étant préférés, ainsi que par exemple n-propylthio, isopropylthio, n-butylthio, isobutylthio, sec-butylthio, tert-butylthio, pentylthio, hexylthio, octylthio, nonylthio ou décylthio ; alkylthioalkyle contenant de 2 à 10, de préférence 2 à 6 atomes de carbone, par exemple méthylthiométhyle, 2-méthylthioéthyle ou 2-n-butylthioéthyle ; acylamino, à savoir alcanoylamino contenant de 1 à 7, de préférence 1 à 4 atomes de carbone, formylamino et acétylamino étant préférés, ainsi que propionylamino, butyrylamino, isobutyrylamino, valérylamino, caproylamino, heptanoylamino, ainsi que aroylamino ou benzylamino ; acylaminoalkyle, de préférence alcanoylaminoalkyle contenant de 2 à 8, de préférence 3 à 6 atomes de carbone, tel que formylaminoéthyle, acétylaminoéthyle, propionylaminoéthyle, n-butyrylaminoéthyle, formylaminopropyle, acétylaminopropyle, propionylaminopropyle, formylaminobutyle, acétylaminobutyle, ainsi que propionylaminobutyle, butyrylaminobutyle ; acyloxy contenant de 1 à 6, de préférence 2 à 4 atomes de carbone, acétyloxy, propionyloxy ou butyryloxy étant préférés, ainsi que par exemple formyloxy, valéryloxy, caproyloxy ; alcoxycarbonyle contenant de 2 à 5, de préférence 2 ou 3 atomes de carbone, méthoxycarbonyle et éthoxycarbonyle étant préférés, ainsi que par exemple n-propoxycarbonyle, isopropoxycarbonyle, n-butoxycarbonyle, isobutoxycarbonyle, sec-butoxycarbonyle ou tert-butoxycarbonyle ; cycloalcoxycarbonyle contenant de 4 à 8, de préférence 6 ou 7 atomes de carbone, cyclopentyloxycarbonyle, cyclohexyloxycarbonyle étant préférés, ainsi que cyclopropyloxycarbonyle, cyclobutyloxycarbonyle ou cycloheptyloxycarbonyle ; alkylaminocarbonylamino contenant de 2 à 4 atomes de carbone, tel que méthylaminocarbonylamino, éthylaminocarbonylamino, propylaminocarbonylamino ; dialkylaminocarbonylamino contenant de 3 à 7, de préférence 3 à 5 atomes de carbone, de préférence diméthylaminocarbonylamino, ainsi que di-n-propylaminocarbonylamino, diisopropylaminocarbonylamino ; (1-pyrrolidino)-carbonylamino ; cycloalkylaminocarbonylamino contenant de 4 à 8, de préférence 6 ou 7 atomes de carbone, cyclopentylaminocarbonylamino, cyclohexylamino-carbonylamino étant préférés, ainsi que cyclopropylaminocarbonylamino, cyclobutylaminocarbonylamino, cycloheptylaminocarbonyl-amino; alkylamino-carbonylaminoalkyle contenant de 3 à 9, de préférence 4 à 7 atomes de carbone, méthylaminocarbonylaminoéthyle, éthylaminocarbonylaminoéthyle, éthylamino-carbonylaminopropyle, éthylaminocarbonylaminobutyle étant préférés, ainsi que par exemple méthylaminocarbonylaminométhyle, n-propylaminocarbonylamino-butyle, n-butylaminocarbonylaminobutyle ; dialkylaminocarbonylaminoalkyle contenant de 4 à 11 atomes de carbone, par exemple diméthylamino-carbonylaminométhyle, diéthylaminocarbonylaminoéthyle, diéthylaminocarbonylaminopropyle, diéthylaminocarbonylaminobutyle, (1-pyrrolidino) carbonylaminoéthyle, (1-pipéridino)-carbonylaminoéthyle, cycloalkylaminocarbonylaminoalkyle contenant de 5 à 12, de préférence 8 à 11 atomes de carbone, cyclopentylaminocarbonylaminoéthyle, cyclopentylaminocarbonylaminopropyle, cyclopentylaminocarbonylaminobutyle, cyclohexylaminocarbonylaminoéthyle, cyclohexylaminocarbonylaminopropyle et cyclohexylaminocarbonylaminobutyle étant préférés, ainsi que par exemple cyclopropylaminocarbonylaminométhyle, cycloheptylaminocarbonylaminoéthyle ; alcoxycarbonylaminoalkyle contenant de 3 à 12, de préférence 4 à 9 atomes de carbone, méthoxycarbonylaminoéthyle, éthoxycarbonylaminoéthyle, n-propoxycarbonylaminoéthyle, isopropoxycarbonylaminoéthyle, n-butoxycarbonyl-aminoéthyle, isobutoxycarbonylaminoéthyle, sec-butoxycarbonylaminoéthyle, tert-butoxycarbonylaminoéthyle, éthoxycarbonylaminopropyle, n-butoxycarbonylaminopropyle, éthoxycarbonylaminobutyle, n-butoxycarbonylaminobutyle étant préférés, ainsi que par exemple n-propoxycarbonylaminopropyle, n-propoxycarbonylaminobutyle, isopropoxycarbonylaminobutyle ; cycloalcoxycarbonylaminoalkyle contenant de 5 à 12, de préférence 8 à 11 atomes de carbone, cyclopentyloxycarbonylaminoéthyle, cyclopentyloxycarbonylaminopropyle, cyclopentyloxycarbonylaminobutyle, cyclohexyloxycarbonylaminoéthyle, cyclohexyloxy-carbonylaminopropyle, cyclohexyloxycarbonylaminobutyle étant préférés, ainsi que par exemple cyclopropyloxycarbonylaminométhyle, cycloheptyloxycarbonylaminoéthyle ; carbamoylalkyle contenant de 2 à 5, de préférence 2 atomes de carbone, de préférence carbamoylméthyle, ainsi que carbamoyléthyle, carbamoylpropyle, carbamoylbutyle ; alkylamthocarbonylalkyle contenant de 3 à 9, de préférence 3 à 6 atomes de carbone, méthylaminocarbonyléthyle, éthylaminocarbonylméthyle, n-propylaminocarbonylméthyle, isopropylamino-carbonylméthyle, n-butylaminocarbonylméthyle, isobutylaminocarbonylméthyle, sec-butylaminocarbonylméthyle, tert-butylaminocarbonylméthyle étant préférés, ainsi que par exemple éthylaminocarbonyléthyle, éthylaminocarbonylpropyle, éthylaminocarbonylbutyle, n-propylaminocarbonylbutyle, n-butylaminocarbonylbutyle ; dialkylaminocarbonyl-alkyle contenant de 4 à 11, de préférence 4 à 8 atomes de carbone, diméthylamino-carbonylméthyle, diéthylaminocarbonylméthyle, di-n-propylaminocarbonylméthyle ; ainsi que par exemple diéthylaminocarbonyléthyle, diéthylaminocarbonylpropyle, diéthylaminocarbonylbutyle ; (1-pyrrolidino)earbonylméthyle, (1-pipéridino)carbonylméthyle ; (1-pipéridino)carbonyléthyle ; cycloalkylaminocarbonylalkyle contenant de 5 à 12, de préférence 7 ou 8 atomes de carbone, cyclopentylaminocarbonylméthyle et cyclohexylaminocarbonylméthyle étant préférés, ainsi que par exemple cyclopropylaminocarbonylméthyle, cyclobutylaminocarbonylméthyle, cycloheptylaminocarbonylméthyle, cyclohexylaminocarbonyléthyle, cyclohexylaminocarbonylpropyle, cyclohexylaminocarbonylbutyle ; alkylaminocarbonylalcoxy contenant de 3 à 10, de préférence 3 à 5 atomes de carbone, méthylaminocarbonylméthoxy étant préféré, ainsi que par exemple méthylaminocarbonyléthoxy, méthylaminocarbonylpropoxy ;

dialkylamino-carbonylalcoxy contenant de 4 à 10, de préférence 4 à 7 atomes de carbone, tel que diméthylaminocarbonylméthoxy, diéthylaminocarbonyléthoxy, (pipéridinyl-1) carbonylméthoxy ; cycloalkylaminocarbonylalcoxy contenant de 5 à 11, de préférence 7 ou 8 atomes de carbone, tel que cyclopentylaminocarbonylméthoxy, cyclohexylaminocarbonylméthoxy.

Le groupe Z est avantageusement un groupe phényle ; un groupe naphtyle.

Le groupe phényle Z est de préférence mono ou disubstitué par un halogène, ou un alcoxy, le groupe isopropoxy étant préféré.

Le radical Z peut également représenter un groupe aromatique bicyclique tel que le 1- ou 2-naphtyle ; 1-, 2-, 3-, 4-, 5-, 6-, 7-indényle ; dont une ou plusieurs liaisons peuvent être hydrogénées, lesdits groupes pouvant être non substitués ou contenir éventuellement un ou plusieurs substituants choisis parmi : un halogène et plus particulièrement un atome de fluor, les groupes alkyle, phényle, cyano, hydroxyalkyle, hydroxy, oxo, alkylcarbonylamino et alcoxycarbonyle, thioalkyle dans lesquels les alkyles sont en $C_1$-$C_4$.

le radical Z peut être aussi un groupe pyridyle, thiadiazolyle, indolyle, indazolyle, imidazolyle, benzimidazolyle, quinoléyle, benzotriazolyle, benzofuranyle, benzothiényle, benzothiazolyle, benzisothiazolyle, isoquinolyle, benzoxazolyle, benzoxazinyle, benzodioxynyle, isoxazolyle, benzopyranyle, thiazolyle, thiényle, furyle, pyranyle, chroményle, isobenzofuranyle, pyrrolyle, pyrazolyle, pyrazinyle, pyrimidinyle, pyridazinyle, indolizinyle, phtalazinyle, quinazolinyle, acridinyle, isothiazolyle, isochromanyle, chromanyle, dont une ou plusieurs doubles liaisons peuvent être hydrogénées, lesdits groupes pouvant être non substitués ou contenir éventuellement un ou plusieurs substituants choisis parmi : les groupes alkyle, phényle, cyano, hydroxyalkyle, hydroxy, alkylcarbonylamino et alcoxycarbonyle, thioalkyle dans lesquels les alkyles sont en $C_1$-$C_4$.

Selon un autre de ses aspects, la présente invention concerne un procédé pour la préparation des composés de formule (I) et de leurs sels caractérisé en ce que :

- a) on traite un composé de formule :

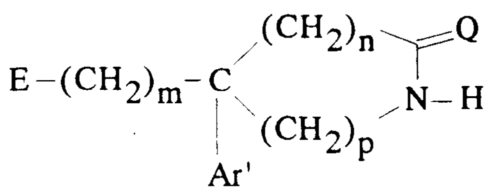

(II)

dans laquelle m, Ar', n, p et Q sont tels que définis précédemment et E représente un hydroxyle ou éventuellement un groupe O-protégé tel que par exemple tétrahydropyranyl-2-oxy ou un groupe

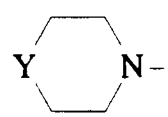

dans lequel Y est tel que défini précédemment étant entendu que : lorsque Y représente le groupe

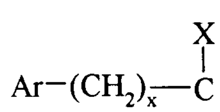

dans lequel X est un hydroxyle, cet hydroxyle peut être protégé,

. soit avec un dérivé fonctionnel d'un acide de formule :

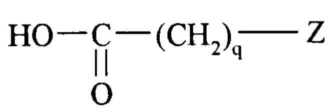

(III)

dans laquelle q et Z sont tels que définis précédemment, lorsqu'on doit préparer un composé de formule (I) où T est -CO-,

. soit avec un dérivé halogéné de formule :

$$Hal\text{-}(CH_2)_{q+1}\text{-}Z \qquad (IV)$$

dans laquelle q et Z sont tels que définis précédemment et où Hal représente un halogène et préférentiellement un atome de brome ou de chlore, lorsqu'on doit préparer un composé de formule (I) où T est $-CH_2-$,

pour obtenir le composé de formule :

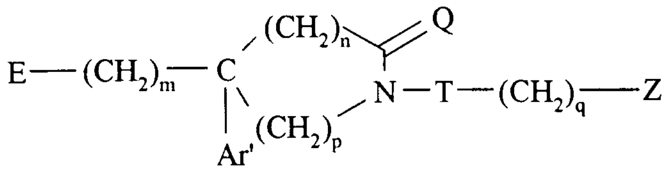

(V)

- b) puis, lorsque E représente un groupe O-protégé, on élimine le groupe O-protecteur par action d'un acide,
- c) on traite l'alcool ainsi obtenu de formule :

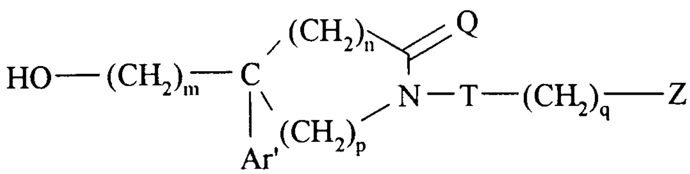

(VI)

avec le chlorure de méthanesulfonyle,

- d) on fait réagir le mésylate ainsi obtenu de formule :

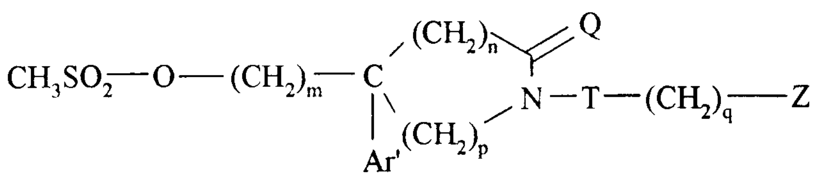

(VII)

ces composés étant des composés nouveaux faisant partie de l'invention, avec une amine secondaire de formule :

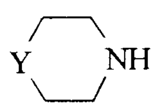

(VIII)

dans laquelle Y est tel que défini précédemment et,

- e) après déprotection éventuelle de l'hydroxyle représenté par X, on transforme éventuellement le produit ainsi obtenu en un de ses sels.

Les composés de formule (II),

. sous réserve que Q représente l'oxygène :

- lorsque E = OH ou ($C_1$-$C_6$)alkylcarbonyloxy, m = 2 ou 3, Ar' représente un phényle substitué par un ($C_1$-$C_4$)alcoxy, n = 3 et p = 1 ;
- lorsque E = ($C_1$-$C_4$)alkylcarbonyloxy, m = 2, n = 1 et Ar' représente un phényle non substitué ou substitué une ou plusieurs fois par un ($C_1$-$C_4$)alkyle, un ($C_1$-$C_4$)alcoxy ou un halogène.

. sous réserve que lorsque E = OH ou un ($C_1$-$C_4$)alcoxy, m = 2, p = 1, Ar' représente un phényle non substitué ou substitué une ou plusieurs fois par un ($C_1$-$C_4$)alkyle, un ($C_1$-$C_4$)alcoxy ou un halogène, n soit différent de 1.
. sous réserve que lorsque E = OH, Q = 2H, m = 2, n = 1, p = 2, Ar' soit différent d'un phényle non substitué

sont nouveaux et font partie de l'invention.
Les composés exclus par cette limitation des composés (II) sont décrit dans la publication H. Bochow et al. Chem. Ber., 1975, 108, 3475 ainsi que dans les demandes DE-A-2 345 192 et GB-A-2 056 439.
Les composés de formule (V),

. sous réserve que lorsque E = OH, Q = 2H, m = 2, n = 1, p = 2, T = -$CH_2$-, q = 0 et Z = phényle, Ar' soit différent d'un phényle non substitué, et
. sous réserve que lorsque E = OH, Q = 2H, m = 2, n = 1, p = 1, T = -$CH_2$-, q = 0 et Z = phényle, Ar' soit différent d'un phényle non substitué ou substitué une ou plusieurs fois par un ($C_1$-$C_4$)alkyle, un ($C_1$-$C_4$)alcoxy ou un halogène, et
. sous réserve que lorsque E = OH ou ($C_1$-$C_6$)alkylcarbonyloxy, Q = 2H, m = 2 ou 3, n = 3, p = 1, T = -$CH_2$-, q = 0, 1, 2 ou 3 et Z est un groupe aryle, Ar' soit différent d'un phényle substitué par un ($C_1$-$C_4$)alcoxy,

sont nouveaux et font partie de l'invention.
Les composés de formule (VI),

. sous réserve que lorsque Q = 2H, m = 2, n = 1, p = 2, T = -$CH_2$-, q = 0 et Z = phényle, Ar' soit différent d'un phényle non substitué, et
. sous réserve que lorsque Q = 2H, m = 2, n = 1, p = 1, T = -$CH_2$-, q = 0 et Z = phényle, Al' soit différent d'un phényle non substitué ou substitué une ou plusieurs fois par un ($C_1$-$C_4$)alkyle, un ($C_1$-$C_4$)alcoxy ou un halogène, et
. sous réserve que lorsque Q = 2H, m = 2 ou 3, n = 3, p = 1, T = -$CH_2$-, q = 0, 1, 2 ou 3 et Z est un groupe aryle, Ar' soit différent d'un phényle substitué par un ($C_1$-$C_4$)alcoxy,

sont nouveaux et font partie de l'invention.
Les composés exclus par ces limitations des composés de formule (V) et (VI) sont décrits dans la publication H. Bochow et al. Chem. Ber., 1975, 108, 3475, dans la demande GB-A-2 056 439 et dans la demande DE-A-2 345 192.
Les sels d'ammonium quaternaires éventuellement formés avec l'azote (b) de la pipéridine lorsque

$$Y = Ar-(CH_2)_x-\overset{X}{\overset{|}{C}}\ ,$$

sont préparés par réaction des bases libres des composés (I), pour lesquelles les fonctions aminés autres, éventuellement présentes sont N-protégées par un groupe N-protecteur habituel, avec un excès d'agent alkylant de formule :

A - Q'

dans lequel A représente un groupe partant et est tel que défini précédemment pour (I), de préférence un chlorure ou un iodure et Q' est tel que défini précédemment pour (I) et on chauffe le mélange réactionnel dans un solvant par exemple choisi parmi le dichlorométhane, le chloroforme, l'acétone ou l'acétonitrile à une température comprise entre la température ambiante et le reflux pendant une à plusieurs heures pour obtenir après traitement selon les méthodes habituelles et après déprotection éventuelle, un mélange des diastéréoisomères axiaux et équatoriaux des sels d'ammonium quaternaires.
De préférence, $A^{\ominus}$ représente un iodure qui peut être échangé par un autre anion ou par un anion pharmacologiquement acceptable, par exemple un chlorure, par élution du composé (I) sur une résine échangeuse d'ion, par exemple l'Amberlite IRA68® ou Duolite A375®.
Les diastéréoisomères sont séparés selon les méthodes habituelles, par exemple par chromatographie ou par recristallisation.
Chacun des diastéréoisomères axiaux ou équatoriaux des composés (I) sous forme de racémiques ou sous

forme d'énantiomères R ou S optiquement purs font partie de l'invention.

Les dérivés N-oxydes éventuellement formés avec l'azote (b) de la pipéridine lorsque

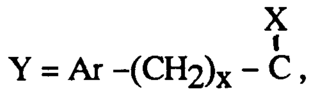

sont préparés par réaction avec un dérivé peroxydé, par exemple l'acide métachloroperbenzoïque ou l'eau oxyénée, selon les méthodes habituelles.

Comme dérivé fonctionnel de l'acide (III), on utilise l'acide lui-même, convenablement activé par exemple par le cyclohexylcarbodiimide ou par l'hexafluorophosphate de benzotriazolyl N-oxy-trisdiméthylaminophosphonium (BOP), ou bien un des dérivés fonctionnels qui réagissent avec les amines, par exemple un anhydride, un anhydride mixte, le chlorure d'acide ou un ester activé.

Lorsque comme produit de départ on utilise un composé de formule (II) où E représente un groupe :

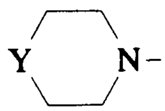

le procédé de la présente invention peut être représenté et illustré en détail par le schéma 1 ci-après :

SCHEMA 1

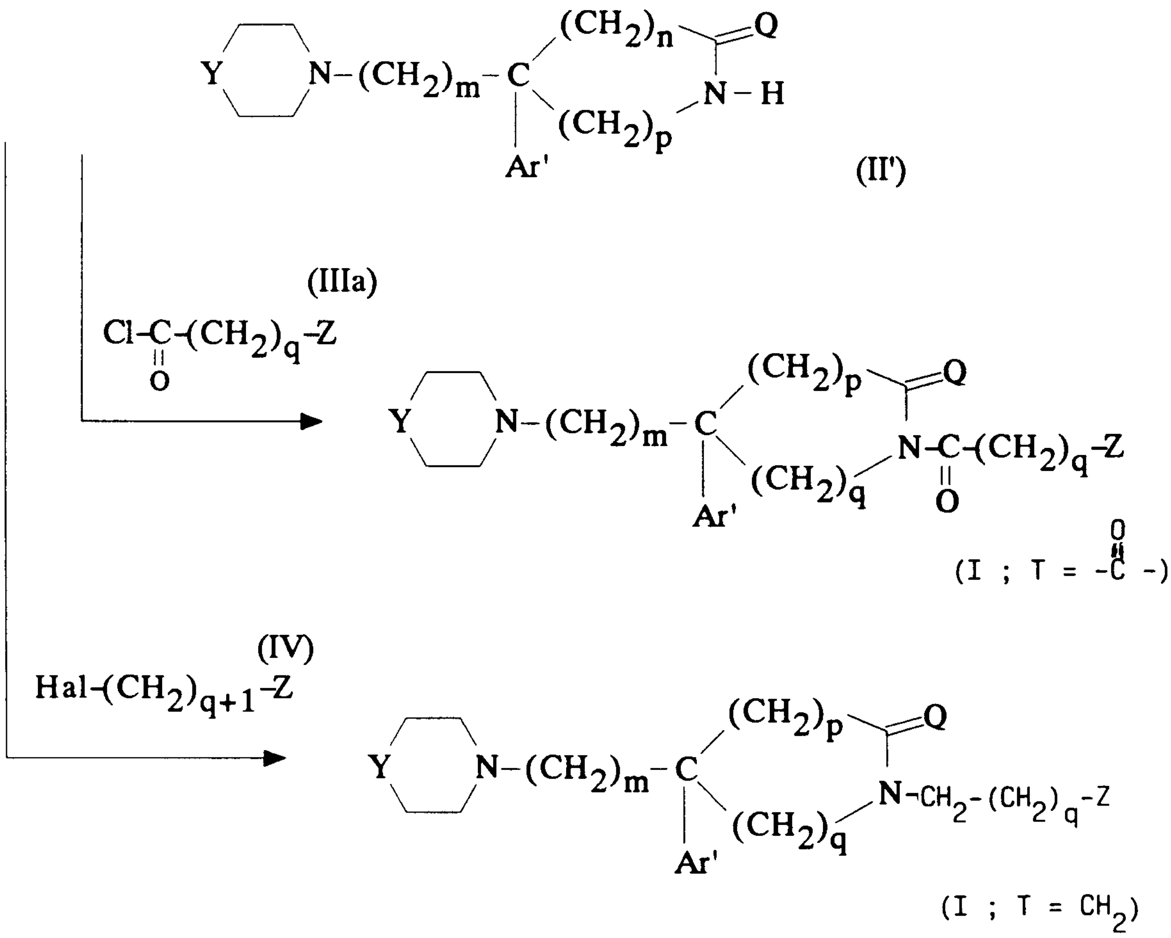


(II')

(I ; T = -C(O)-)

(I ; T = CH2)

Dans la formule (IIIa) ci-dessus, on considère le chlorure d'acide comme dérivé fonctionnel réactif de l'acide (III). On peut cependant utiliser un autre dérivé fonctionnel ou on peut partir de l'acide libre (III) en réalisant un couplage (II') avec le BOP, puis, en additionnant l'acide (III) en présence d'une base organique comme par exemple la triéthylamine, dans un solvant comme le dichlorométhane ou le diméthylformamide, à température ambiante. Les composés (1) obtenus sont isolés et purifiés selon les méthodes habituelles, comme par exemple la chromatographie ou la recristallisation.

Lorsque comme produit de départ on utilise un composé de formule (II) où E représente un groupe tétrahydropyranyloxy (THP-O-), le procédé de la présente invention peut être représenté et illustré à partir du schéma 2.

Les réactions du composé (II) avec les réactifs (IIIA) et (IV) se déroulent comme décrit ci-dessus pour le schéma 1, le chlorure d'acide (IIIA) pouvant être remplacé par un autre dérivé fonctionnel ou par l'acide libre activé par exemple par le BOP.

L'intermédiaire (V) ainsi obtenu est déprotégé par hydrolyse acide douce pour conduire au composé hydroxylé libre (VI) duquel on prépare le mésylate (VII) pour le substituer par une amine secondaire de formule (VIII) pour obtenir finalement les composés (I) selon l'invention.

SCHEMA 2

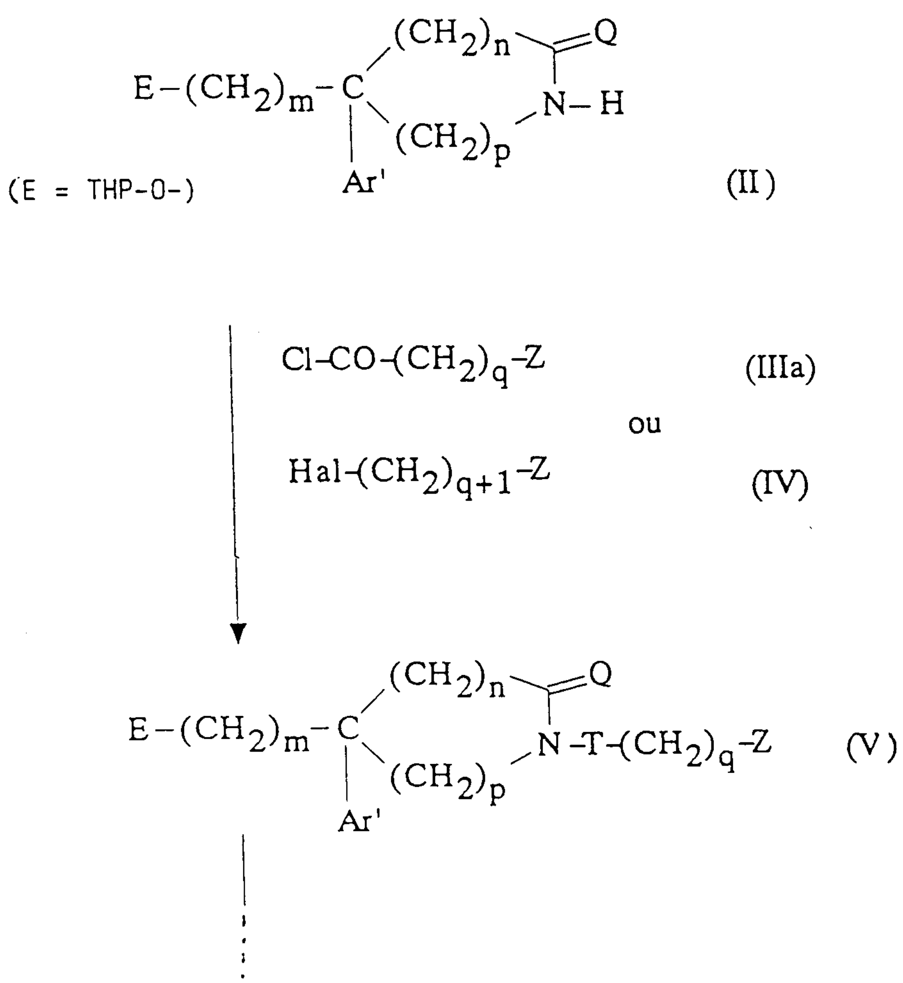

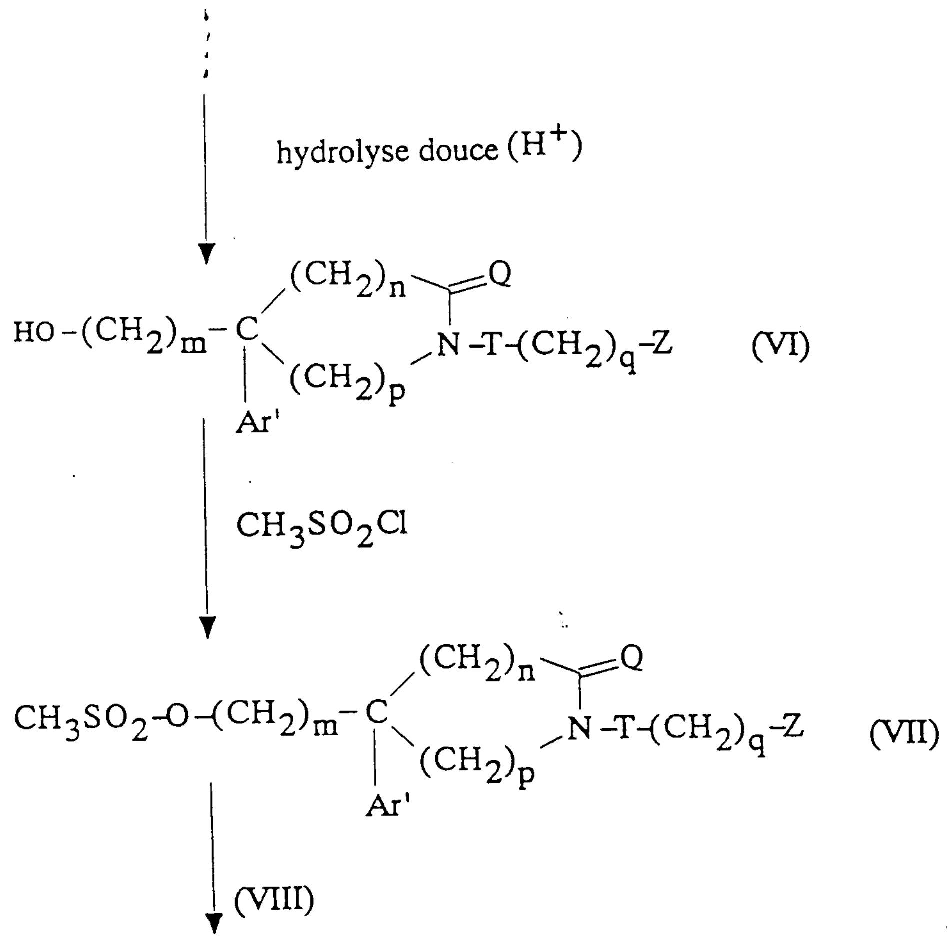


(VI)

(VII)

(I)

Les produits de formule (I) ainsi obtenus sont isolés, sous forme de base libre ou de sel, selon les techniques classiques.

Lorsque le composé de formule (I) est obtenu sous forme de base libre, la salification est effectuée par traitement avec l'acide choisi dans un solvant organique. Par traitement de la base libre, dissoute par exemple dans un alcool tel que l'isopropanol, avec une solution de l'acide choisi dans le même solvant, on obtient le sel correspondant qui est isolé selon les techniques classiques. Ainsi, on prépare par exemple le chlorhydrate, le bromhydrate, le sulfate, l'hydrogénosulfate, le dihydrogénophosphate, le méthanesulfonate, l'oxalate, le maléate, le fumarate, le 2-naphtalènesulfonate.

A la fin de la réaction, les composés de formule (I) peuvent être isolés sous forme d'un de leurs sels, par exemple le chlorhydrate ou l'oxalate ; dans ce cas, si nécessaire, la base libre peut être préparée par neutralisation dudit sel avec une base minérale ou organique, telle que l'hydroxyde de sodium ou la triéthylamine ou avec un carbonate ou bicarbonate alcalin, tel que le carbonate ou bicarbonate de sodium ou de potassium.

On peut aussi préparer à partir des bases libres, les sels d'ammonium quaternaires, par réaction avec un agent alkylant, ou les dérivés N-oxydes, sur l'azote (b) de la pipéridine étant entendu que les autres fonctions aminés éventuellement présentes sur (I) sont N-protégées par des groupes N-protecteurs bien connus de l'homme de l'art.

Les composés de départ de formule (II) sont préparés à partir de nitriles commerciaux ou préparés selon des méthodes connues selon les schémas ci-dessous.

Lorsque n est égal à zéro, le composé (II) est préparé selon D.C. Bishop et al., J. Med. Chem., 1968, 11, 466-470 selon le schéma 3 suivant.

SCHEMA 3

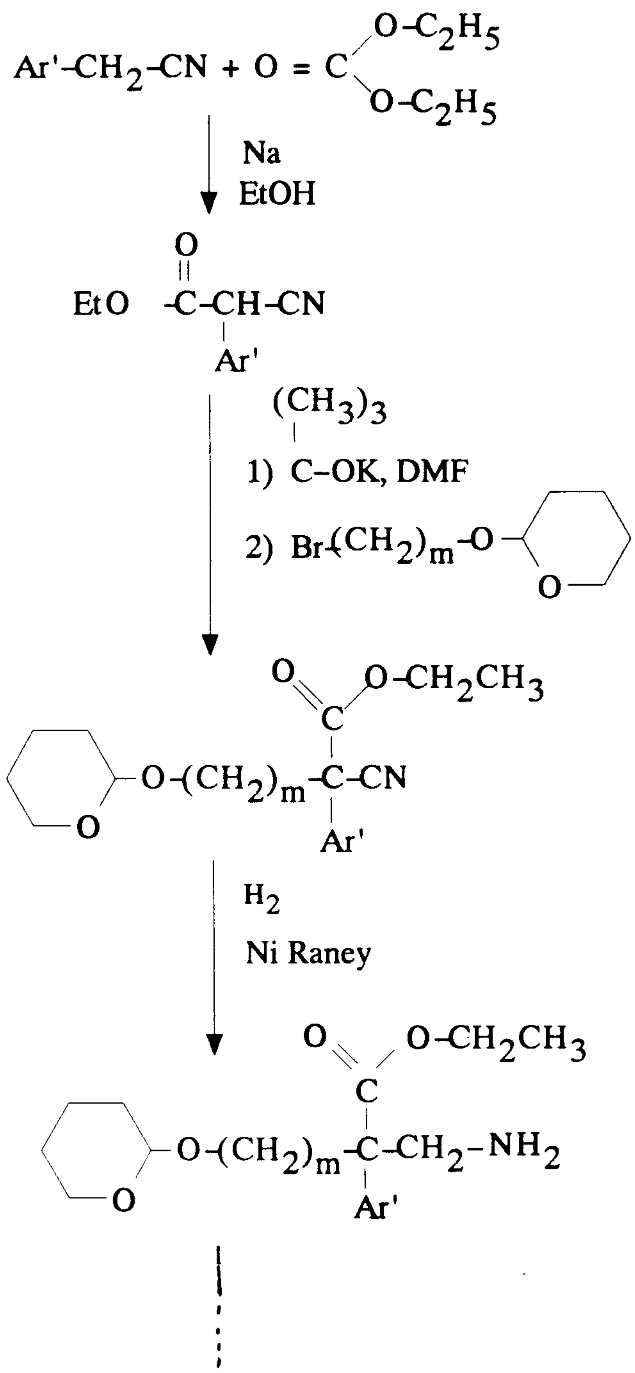

Suite SCHEMA 3

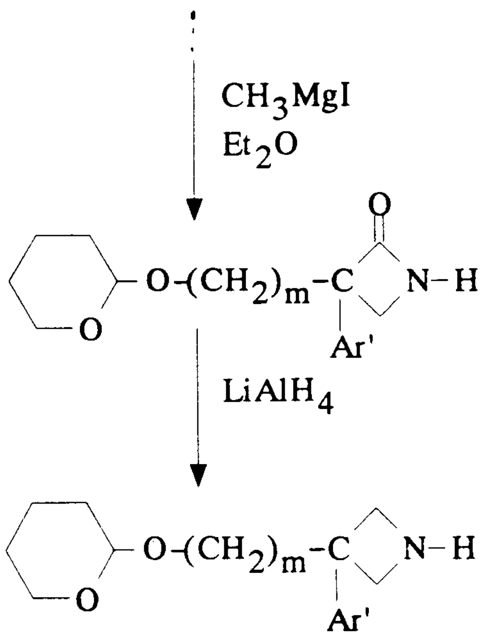


(II ; E = THP-O- et Q = O)

(II ; E = THP-O- et Q = 2H)

Lorsque n = 1, 2 ou 3, les composés (II) correspondant sont préparés selon le schéma 4 suivant.

SCHEMA 4

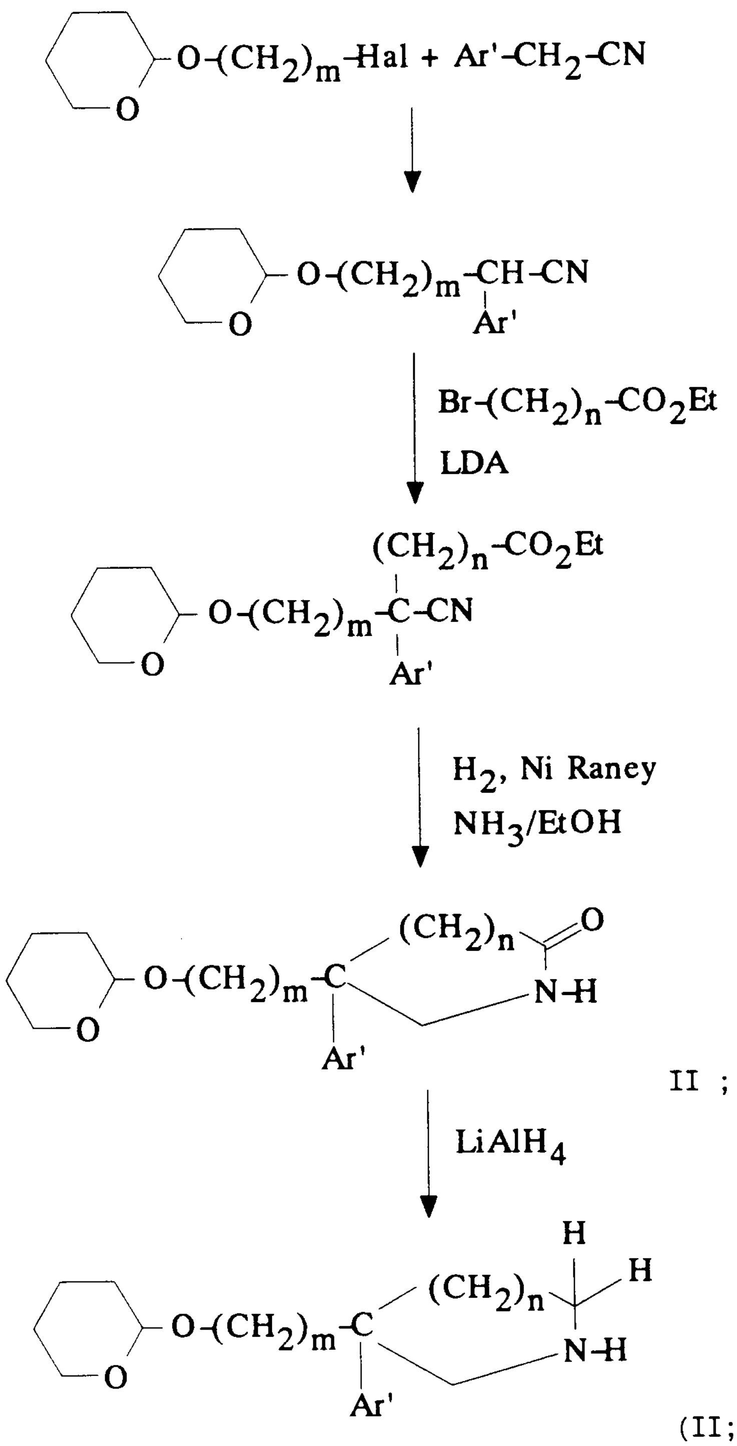


II ; E=THP-O- et Q=O)

(II;E=THP-O- et Q=2H)

Lorsque p = 2, alors n = 1, Q = 2H et m = 2 et l'intermédiaire (II) est préparé selon H. Bochow et al., Chem. Ber., 1975, 108, 3475-3482. La préparation est illustrée par le schéma 5 ci-dessous.

SCHEMA 5

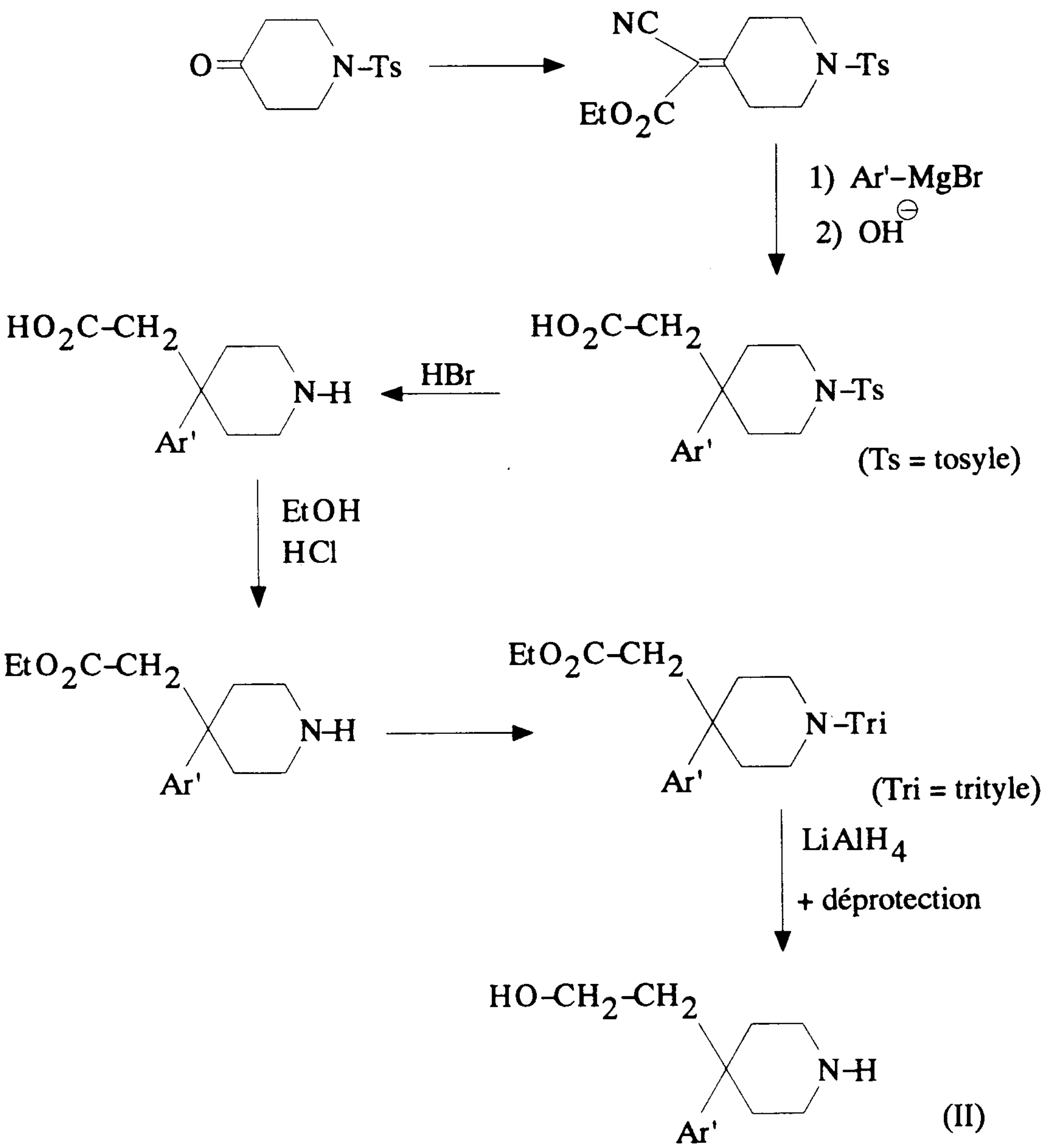


(II)

Les groupes OH et NH peuvent ou non être protégés par des groupes O-protecteurs ou N-protecteurs classiques bien connus de l'homme de l'art.

La résolution des mélanges racémiques (I) permet d'isoler les énantiomères (I*) de formule

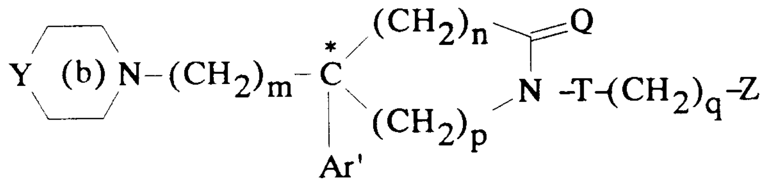

(I*)

dans laquelle

- "*" signifie que l'atome de carbone ainsi marqué à la configuration absolue (+) ou (-) déterminée,
- Y, m, Ar′, n, p, Q, T, q et Z sont tels que définis plus haut pour les dérivés de formule (I), ou un de leurs sels avec des acides minéraux ou organiques, ou encore avec l'atome d'azote (b) un de leurs sels d'ammonium quaternaires ou dérivés N-oxyde.

Lesdits sels ou dérivés N-oxyde sont préparés comme indiqué plus haut pour les sels et dérivés correspondants des dérivés de formule (I).

Les énantiomères de formule (I*) sont des produits nouveaux qui font partie de l'invention.

On peut aussi effectuer le dédoublement des mélanges racémiques des produits de formule (II) dans lesquels m, Ar′, n, et p sont tels que définis pour (I), E représente un hydroxyle et Q représente l'hydrogène, afin de préparer les énantiomères (I*) des produits de formule (I).

Le dédoublement des racémiques est effectué sur les intermédiaires (II) susceptibles de donner des sels avec des acides optiquement actifs. Les énantiomères sont alors séparés par les méthodes classiques telles que la cristallisation ou la chromatographie haute pression préparative chirale.

L'aminoalcool optiquement pur ainsi préparé, est un composé nouveau faisant partie de l'invention, de formule :

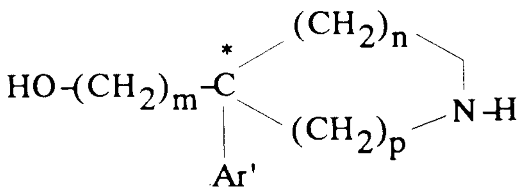

(II*)

dans laquelle "*" signifie que l'atome de carbone ainsi marqué est de configuration (+) ou (-) déterminée.

Les intermédiaires de formules (VI) et (VII) , dans lesquels Q représente l'hydrogène, sous forme optiquement pure, sont des produits nouveaux particulièrement intéressants et représentent un aspect ultérieur de la présente invention. Ces produits peuvent être regroupés dans la formule suivante :

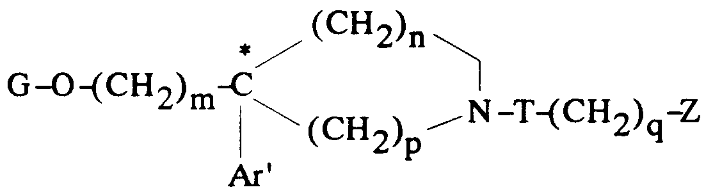

(V*)

dans laquelle :

"*", m, Ar′, n, p, T, q et Z sont tels que définis précédemment et G représente l'hydrogène ou un groupe méthanesulfonyle.

Les mélanges racémiques des composés (II) sont préparés comme indiqué dans les Schéma 3, 4 et 5 ci-dessus.

Les composés (V*) optiquement purs sont préparés selon la suite de réactions indiquées dans le Schéma 2 ci-dessus à partir des composés (II) optiquement purs (II*) pour conduire aux produits finaux selon l'invention sous forme optiquement pure (I*).

Lorsque le substituant $-(CH_2)_q-Z$ représente un groupe benzyle substitué sur le -CH- par un hydroxyle, un alcoxy ou un alkyle en $C_1$-$C_4$ on obtient soit un mélange de deux ou quatre diastéréoisomères selon que l'on fait réagir un dérivé benzylique a-substitué optiquement pur ou non avec un dérivé aminé optiquement pur ou non.

Ces diastéréoisomères font partie de l'invention.

Les composés selon l'invention ont fait l'objet d'essais biochimiques.

Les composés (I) et leurs sels ont montré des propriétés antagonistes de la liaison de la substance P dans des essais réalisés sur des membranes de cortex de rat et de cellules lymphoblastiques IM9, selon M.A. Cascieri et al., J. Biol. Chem., 1983, 258, 5158-5164 et D.D. Paya et al., J. Immunol., 1984, 133, 3260-3265.

Les mêmes composés et leurs sels ont montré des propriétés antagonistes de la liaison de la $NK_A$ dans des

essais réalisés sur des membranes de duodénum de rat, selon L. Bergstom et al., Mol. Pharmacol., 1987, 32, 764-771.

Les mêmes composés et leurs sels ont montré des propriétés antagonistes de la liaison de l'élédoïsine dans des essais réalisés sur des membranes de rat selon A.C. Foster et al., Br. J. Pharmacol., 1988, 94 602-608.

L'élédoïsine est un peptide d'origine batracienne qui est équivalent à la neurokinine B.

Les composés selon l'invention sont des antagonistes de la substance P, de la neurokinine A ou de la neurokinine B.

Ainsi, le composé 2 de l'exemple 2 antagonise la liaison de la substance P avec un Ki de 8,3 nanomolaire, le composé 7 de l'exemple 7 antagonise la liaison de la neurokinine A avec un Ki de 1,3 nanomolaire et le composé 3 de l'exemple 3 antagonise la liaison de l'élédoïsine avec un Ki de 200 nanomolaire.

Les composés de la présente invention sont généralement administrés en unité de dosage. Lesdites unités de dosage sont de préférence formulées dans des compositions pharmaceutiques dans lesquelles le principe actif est mélangé avec un excipient pharmaceutique.

Ainsi, selon un autre de ses aspects, la présente invention concerne des compositions pharmaceutiques renfermant, en tant que principe actif, un composé de formule (I) ou un de ses sels pharmaceutiquement acceptables.

Les composés de formule (I) ci-dessus et leurs sels pharmaceutiquement acceptables penvent être utilisés à des doses journalières de 0,01 à 100 mg par kilo de poids corporel du mammifère à traiter, de préférence à des doses journalières de 0,1 à 50 mg/kg. Chez l'être humain, la dose peut varier de préférence de 0,5 à 4000 mg par jour, plus particulièrement de 2,5 à 1000 mg selon l'âge du sujet à traiter ou le type de traitement : prophylactique ou curatif.

Dans les compositions pharmaceutiques de la présente invention pour l'administration orale, sublinguale, sous-cutanée, intramusculaire, intraveineuse, transdermique, locale ou rectale, les principes actifs pouvant être administrés sous formes unitaires d'administration, en mélange avec des supports pharmaceutiques classiques, aux animaux et aux êtres humains. Les formes unitaires d'administration appropriées comprennent les formes par voie orale telles que les comprimés, les gélules, les poudres, les granules et les solutions ou suspensions orales, les formes d'administration sublinguale et buccale, les formes d'administration sous-cutanée, intramusculaire, intraveineuse, intranasale ou intraoculaire et les formes d'administration rectale.

Lorsque l'on prépare une composition solide sous forme de comprimés, on mélange le principe actif principal avec un véhicule pharmaceutique tel que la gélatine, l'amidon, le lactose, le stéarate de magnésium, le talc, la gomme arabique ou analogues. On peut enrober les comprimés de saccharose ou d'autres matières appropriées ou encore les traiter de telle sorte qu'ils aient une activité prolongée ou retardée et qu'ils libèrent d'une façon continue une quantité prédéterminée de principe actif.

On obtient une préparation en gélules en mélangeant le principe actif avec un diluant et en versant le mélange obtenu dans des gélules molles ou dures.

Une préparation sous forme de sirop ou d'élixir peut contenir le principe actif conjointement avec un édulcorant, acalorique de préférence, du méthylparaben et du propylparaben comme antiseptique, ainsi qu'un agent donnant du goût et un colorant approprié.

Les poudres ou les granules dispersibles dans l'eau peuvent contenir le principe actif en mélange avec des agents de dispersion ou des agents mouillants, ou des agents de mise en suspension, comme la polyvinylpyrrolidone, de même qu'avec des édulcorants ou des correcteurs du goût.

Pour une administration rectale, on recourt à des suppositoires qui sont préparés avec de liants fondant à la température rectale, par exemple du beurre de cacao ou des polyéthylèneglycols.

Pour une administration parentérale, intranasale ou intraoculaire, on utilise des suspensions aqueuses, des solutions salines isotoniques ou des solutions stériles et injectables qui contiennent des agents de dispersion et/ou des agents mouillants pharmacologiquement compatibles, par exemple le propylèneglycol ou le butylèneglycol.

Pour une administration par inhalation on utilise un aérosol contenant par exemple du trioléate de sorbitane ou de l'acide oléique ainsi que du trichlorofluorométhane, du dichlorofluorométhane, du dichlorotétrafluoroéthane ou tout autre gaz propulseur biologiquement compatible.

Le principe actif peut être formulé également sous forme de microcapsules, éventuellement avec un ou plusieurs supports ou additifs.

Les compositions susdites peuvent également renfermer d'autres produits actifs tels que, par exemple, des bronchodilatateurs, des antitussifs ou antihistaminiques.

Les exemples suivants illustrent l'invention sans toutefois la limiter.

Les points de fusion ou de décomposition des produits, F, ont été mesurés au banc chauffant Koffler. Les spectres de résonnance magnétique nucléaire du $^{13}C$ ont été effectués à 50 MHz dans le diméthylsulfoxyde.

EXEMPLE 1

Chlorhydrate de 5-[2-(4-benzyl-1-pipéridinyl)éthyl]-5-(3,4-dichlorophényl)-1-benzylpipéridinone.

(I) : 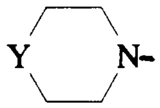 = 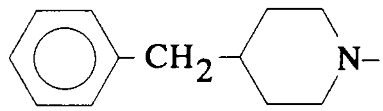 ; m = 2 ; n = 2 ; p = 1 ;

Q = O ; Ar' = 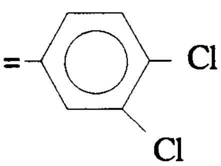 ; $-T-(CH_2)_q-Z$ = 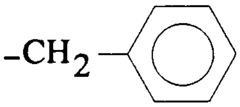

A) 3,4-Dichlorotétrahydropyranyloxyéthyl-α-benzèneacétonitrile.

20 g d'hydrure de sodium à 55-60 % dans l'huile sont mis en suspension dans 200 ml de tétrahydrofurane sec. On ajoute goutte à goutte à 20°C, en 30 minutes, une solution de 85 g de 3,4-dichlorophénylacétonitrile dans 500 ml de tétrahydrofurane puis on agite le mélange réactionnel à température ambiante pendant 2 heures. Le mélange est refroidi à -20°C et on ajoute une solution de 98 g de 2-bromoéthoxytétrahydropyrane dans 100 ml de tétrahydrofurane, on laisse revenir le mélange à température ambiante et après 2 heures on ajoute une solution de 50 g de chlorure d'ammonium dans 3 litres d'eau. On extrait avec 1,5 litres d'éther, lave avec une solution saturée de chlorure de sodium, décante, sèche sur $MgSO_4$ et concentre sous vide.

Le résidu est chromatographié sur gel de silice, éluant : dichlorométhane. Les fractions de produit pur sont concentrées sous vide pour fournir 83,6 g d'une huile.

B) γ-Tétrahydropyranyloxyéthyl-y-cyano-3,4-dichlorobenzylbutanoate d'éthyle.

21 g du nitrile préparé précédemment selon A) sont mis en solution dans 100 ml de tétrahydrofurane, puis on ajoute goutte à goutte et à température ambiante une solution de 0,067 mole de diisopropylamidure de lithium dans 100 ml de tétrahydrofurane et agite le mélange réactionnel pendant une heure à température ambiante. On ajoute alors 12 g de bromopropionate d'éthyle et chauffe à 50°C pendant deux heures. Le mélange est refroidi et on le verse dans une solution saturée de chlorure d'ammonium et extrait à l'éther, lave à l'eau, sépare la phase éthérée par décantation, la sèche sur $Na_2SO_4$ et concentre sous vide. Le résidu est purifié par chromatographie sur gel de silice, éluant : dichlorométhane/acétate d'éthyle 100/1 (v/v). La concentration des fractions pures fournit 13 g du composé attendu.

C) 5-Tétrahydropyranyloxyéthyl-5-(3,4-dichlorophényl) pipéridinone.

13 g du composé préparé précédemment sont mis en solution dans 250 ml d'éthanol et 40 ml d'ammoniaque et sont hydrogénés à température ambiante et pression atmosphérique en présence de nickel de Raney. Lorsque le volume théorique d'hydrogène est absorbé, on filtre le mélange sur célite et on concentre le filtrat sous vide. Le résidu est repris dans l'eau, extrait à l'éther, puis on lave la phase éthérée à l'eau, la sèche sur $MgSO_4$ et concentre sous vide.

m = 9g.

D) 5-Tétrahydropyranyloxyéthyl-5-(3,4-dichlorophényl)-1-benzylpipéridinone.

2,05 g de bromure de benzyle sont additionnés à une solution de 4,5 g du produit préparé précédemment dans 60 ml de diméthylformamide et en présence de 0,3 g d'hydrure de sodium. Le mélange réactionnel est chauffé à 40-50°C pendant deux heures et concentré sous vide. Le résidu est repris dans l'eau, extrait à l'éther et la phase éthérée est lavée à l'eau, séchée sur $MgSO_4$, et concentrée sous vide. Le résidu est chromatographié sur gel de silice, éluant : dichlorométhane/méthanol 100/1 (v/v).

Les fractions de produit pur sont concentrées sous vide.
m = 2g.

E) 5-Méthanesulfonyloxyéthyl-5-(3,4-dichlorophényl)-1-benzylpipéridinone.

2 g du produit préparé précédemment sont mis en solution dans 40 ml de méthanol saturé en acide chlorhydrique gazeux et la solution est agitée pendant deux heures à température ambiante. Les solvants sont concentrés sous vide et le résidu est repris dans un mélange pentane/éther 50-50 puis on filtre le précipité. Le précipité est mis en solution dans 50 ml de dichlorométhane et on ajoute 0,4 g de triéthylamine et 0,45 g de chlorure de mésyle et agite le mélange pendant une demi heure à température ambiante. On concentre sous vide, reprend le résidu dans l'eau, extrait à l'éther, lave la phase éthérée à l'eau, décante, sèche sur $MgSO_4$ et concentre sous vide.
m = 1,6 g.

F) Composé 1

0,68 g du produit préparé précédemment et 0,63 g de 4-benzylpipéridine sont mis en solution dans 2 ml de diméthylformamide et le mélange est chauffé à 80°C pendant deux heures. On refroidit la solution, verse dans l'eau, extrait à l'acétate d'éthyle, décante la phase organique, sèche sur $MgSO_4$ et concentre sous vide. Le résidu est purifié par chromatographie sur gel de silice, éluant : dichlorométhane/méthanol 100/3 (v/v).

Les fractions de produit pur sont concentrées sous vide puis on fait le chlorhydrate qui est concrétisé dans un mélange éther/pentane 50/50.
m = 0,25 g
F = 115°C

EXEMPLE 2

Chlorhydrate de 3-[2-(4-benzyl-1-pipéridinyl)éthyl]-3-(3,4-dichlorophényl)-1-phénylacétylpipéridine.

(I) : 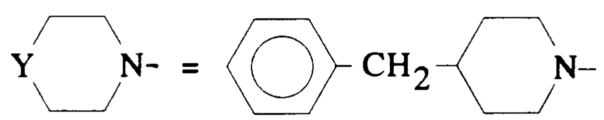 ; m = 2 ; n = 2 ; p = 1 ;

Q = 2H ; Ar' = 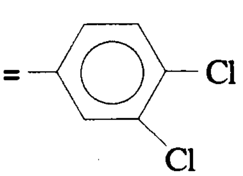 ; 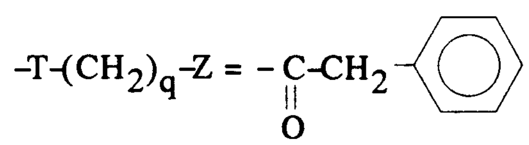

A) 3-Tétrahydropyranyloxyéthyl-3-(3,4-dichlorophényl)pipéridine.

4,5 g de 5-tétrahydropyranyloxyéthyl-5-(3,4-dichlorophényl)pipéridinone préparés selon l'exemple 1 C) sont mis en solution dans 50 ml de tétrahydrofurane et on ajoute la solution à une suspension de 0,9 g d'hydrure de lithium aluminium chauffée à 60°C . Le mélange réactionnel est chauffé pendant une heure à 60°C puis refroidi. On ajoute 1 ml d'eau, 1 ml d'hydroxyde de sodium 4N et 3 ml d'eau. On sépare le minéral par filtration et concentre le filtrat sous vide. Le résidu est repris dans l'éther, séché sur $MgSO_4$ et concentré sous vide pour fournir 3,5 g du produit attendu.

B) 3-Tétrahydropyranyloxyéthyl-3-(3,4-dichlorophényl)-1-phénylacétylpipéridine

0,75 g de chlorure de l'acide phénylacétique sont ajoutés à une solution de 1,7 g du produit préparé précédemment et de 0,9 g de triéthylamine dans 50 ml de dichlorométhane. Le mélange réactionnel est agité pendant une heure à température ambiante et concentré sous vide. Le résidu est repris dans l'acétate d'éthyle, puis lavé à l'eau, la phase organique est séchée sur $MgSO_4$ et concentrée sous vide. Le résidu est purifié par chromatographie sur gel de

silice, éluant : dichlorométhane/méthanol 100/0,5 (v/v).

La concentration des fractions pures fournit 1 g du produit attendu.

C) 3-Méthanesulfonyloxyéthyl-3-(3,4-dichlorophényl)-1-phénylacétylpipéridine.

0,8 g du produit obtenu précédemment sont mis en solution dans 40 ml de méthanol saturé d'acide chlorhydrique et le mélange est agité pendant une demi-heure à température ambiante. On concentre sous vide et le résidu est repris dans 40 ml de dichlorométhane. On ajoute 0,4 g de triéthylamine et 0,23 g de chlorure de mésyle et le mélange réactionnel est agité pendant une heure à température ambiante puis concentré sous vide. Le résidu est repris dans l'acétate d'éthyle, on lave à l'eau, sépare la phase organique par décantation, sèche sur $MgSO_4$ et concentre sous vide.

m = 0,71 g.

D) Composé 2

0,7 g du produit préparé précédemment et 0,52 g de 4-benzylpipéridine en solution dans 2 ml de diméthylformamide sont chauffés à 80°C pendant trois heures. On refroidit le mélange réactionnel, le verse dans l'eau, extrait à l'éther, lave la phase éthérée à l'eau, sèche sur $MgSO_4$ et concentre sous vide puis recristallise le chlorhydrate dans un mélange dichlorométhane/éther.

m = 0,12 g

F = 210-212°C.

En procédant selon l'exemple 1, on prépare les composés 3 à 6 décrits dans le tableau I ci-dessous.

## TABLEAU I

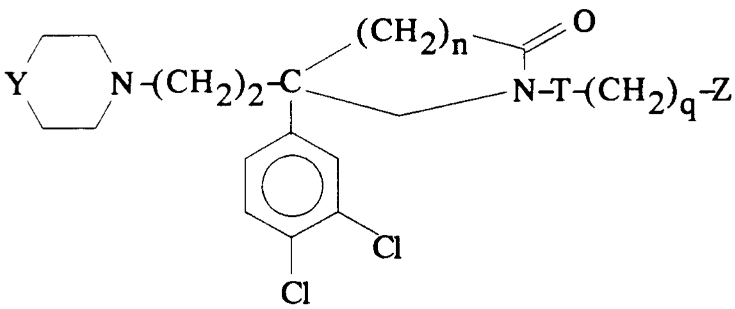

| Exemple n° | 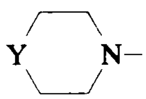 | n | $-T-(CH_2)_q-Z$ | F ; °C Sel |
|---|---|---|---|---|
| 3 | 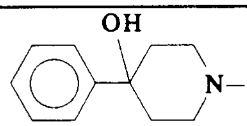 | 2 | 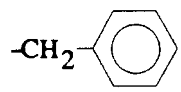 | 250<br>HCl, $0,5H_2O$ |
| 4 | 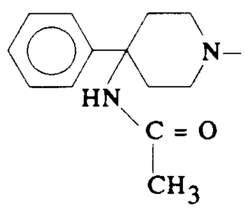 | 1 | 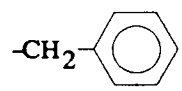 | 168<br>HCl |
| 5 | 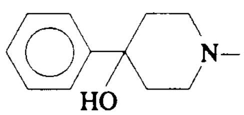 | 1 | 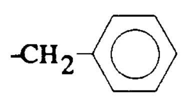 | 142<br>HCl |
| 6 | 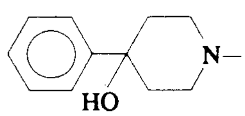 | 2 | 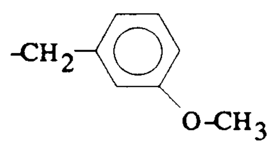 | 136<br>HCl |

En procédant selon l'exemple 2, on prépare les composés 7 à 15 décrits dans le tableau II ci-dessous.

TABLEAU II

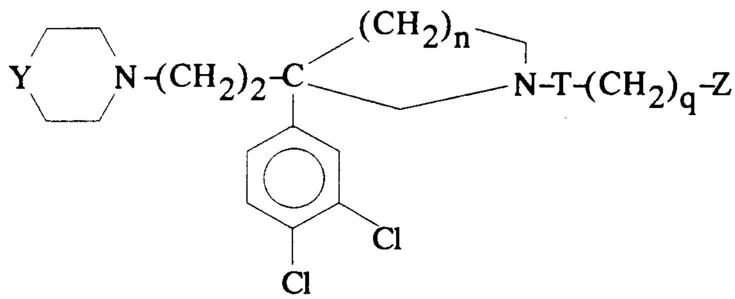

| Exemple n° | 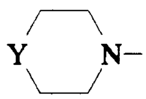 | n | $-T-(CH_2)_q-Z$ | F ; °C Sel |
|---|---|---|---|---|
| 7 | 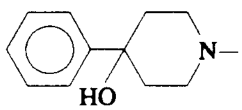 | 1 | 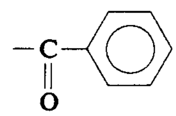 | 139<br>HCl |
| 8 | 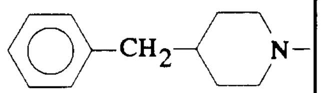 | 1 | 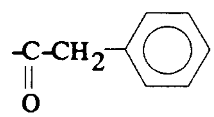 | 112<br>HCl |
| 9 | 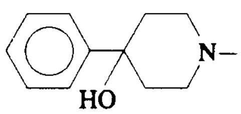 | 2 | 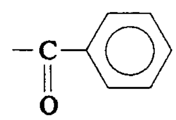 | 160<br>HCl |
| 10 | 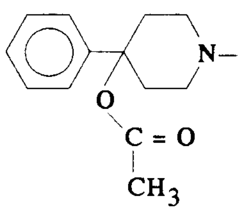 | 2 | 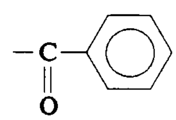 | 142<br>HCl |
| 11 | 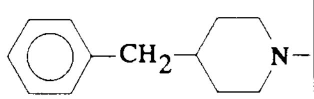 | 2 | 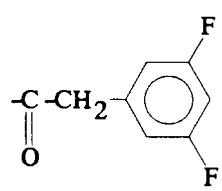 | 114<br>HCl |

TABLEAU II (suite)

| Exemple n° | 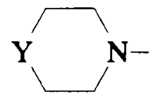 | n | $-T-(CH_2)_q-Z$ | F ; °C Sel |
|---|---|---|---|---|
| 12 | 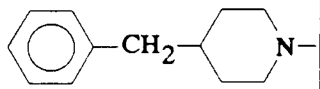 | 2 | 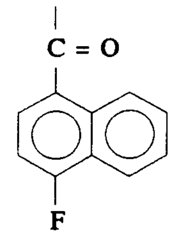 | 168<br>HCl |
| 13 | 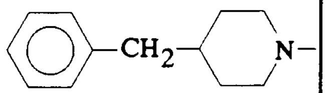 | 2 | 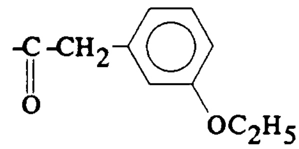 | 102<br>HCl |
| 14 | 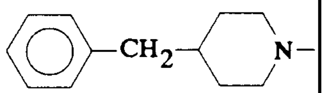 | 2 | 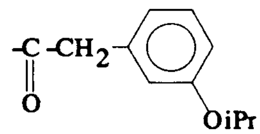 | 169<br>HCl |
| 15 | 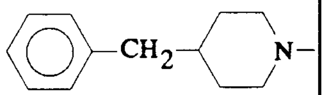 | 2 | 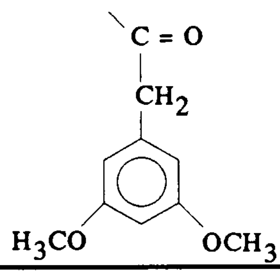 | 110<br>HCl |

iPr = isopropyle

En procédant selon les exemples 1 et 2 ci-dessus et en remplaçant le 3,4-dichlorophénylacétonitrile par l'α-naphtylacétonitrile on obtient les composés 16 à 19 décrits dans le tableau III ci-dessous.

## TABLEAU III

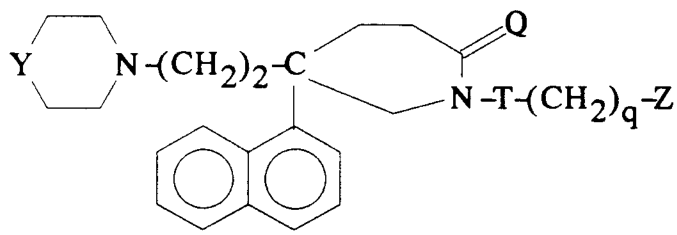

| Exemple n° | 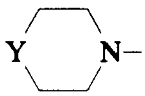 | Q | $-T-(CH_2)_q-Z$ | F ; °C Sel |
|---|---|---|---|---|
| 16 | 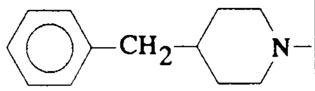 | O | 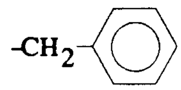 | 104 HCl |
| 17 | 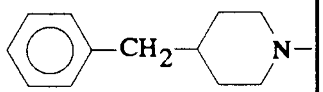 | H | 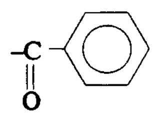 | 115 HCl |
| 18 | 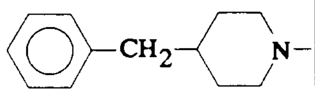 | H | 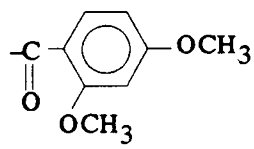 | 105 HCl |
| 19 | 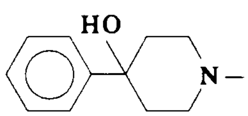 | H | 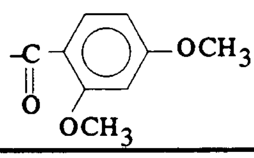 | 110 HCl |

EXEMPLE 20

Chlorhydrate de 3-[2-(4-benzyl-1-pipéridinyl)éthyl]-3-(3,4-dichlorophényl)-1-(3-isopropoxyphényl)acétylazépine.

(I) : 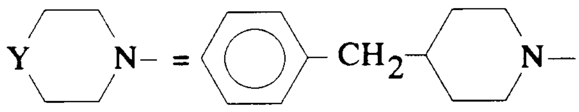 ; m = 2 ; n = 3 ; p = 1 ; Q = 2H ;

Ar' = 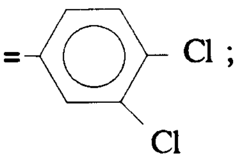 ; $-T-(CH_2)_q-Z =$ 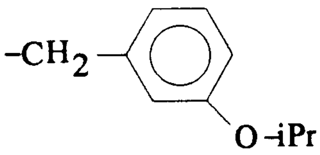

A) δ-Tétrahydropyranyloxyéthyl-δ-cyano-3,4-dichlorobenzylpentanoate d'éthyle.

A 36 g de 3,4-dichloro-α-tétrahydropyranyloxyéthylbenzène acétonitrile (préparé selon l'étape A de l'exemple 1) en solution dans 100 ml de diméthylformamide, on ajoute par petites portions 4,6 g de NaH à 60 %. On agite le mélange réactionnel 3 heures à température ambiante, refroidit à 0°C puis on ajoute 22,4 g de 4-bromobutyrate d'éthyle dans 40 ml de diméthylformamide. On agite le mélange réactionnel pendant 3 heures à température ambiante, on verse sur l'eau, extrait à l'éther, lave avec une solution saturée NaCl, sèche sur $Na_2SO_4$ et concentre sous vide On purifie le résidu obtenu par chromatographie sur gel de silice, éluant : toluène.

m = 24 g.

B) 6-Tétrahydropyranyloxyéthyl-6-(3,4-dichlorophényl)azépinone.

8 g de produit précédemment obtenu sont hydrogénés à pression atmosphérique et température ambiante en présence de nickel de Raney en solution, dans 120 ml d'éthanol.

Quand le volume théorique d'hydrogène est consommé on filtre le catalyseur et concentre sous vide.

L'huile obtenue est ensuite reprise dans 20 ml de xylène et on chauffe le mélange réactionnel à reflux pendant 48 heures. On évapore et purifie le résidu obtenu par chromatographie sur gel de silice, éluant : dichlorométhane/méthanol 100/1 (v/v).

On obtient ainsi 4 g d'une huile.

C) 3-Tétrahydropyranyloxyéthyl-3-(3,4-dichlorophényl) azépine.

A partir de 2 g de produit précédemment obtenu, de 0,49 g d'hydrure de lithium aluminium et en procédant selon l'exemple 2, étape A, on obtient 1,7 g de produit attendu sous forme d'huile.

D) 3-Tétrahydropyranyloxyéthyl-3-(3,4-dichlorophényl)-1-(3-isopropoxyphényl)-acétylazépine.

A partir de 1,7 g du produit précédemment obtenu et en procédant selon l'exemple 2, étape B, on obtient 1,7 g du produit attendu.

E) 3-Méthanesulfonyloxyéthyl-3-(3,4-dichlorophényl)-1-(3-isopropoxyphényl)acétyl azépine.

A partir de 1,7 g du produit précédemment obtenu et de 0,34 g de chlorure de mésyle, en procédant selon l'exemple 2, étape C, on obtient 1,5 g de produit attendu.

F) Composé 20

1,5 g du produit précédemment obtenu et 1,4 g de 4-benzylpipéridine en solution dans 3 ml de diméthylformamide sont chauffés à 80°C pendant 2 heures. On refroidit, verse le mélange réactionnel dans l'eau, extrait à l'éther, lave la phase organique à l'eau, sèche sur $Na_2SO_4$ et concentre sous vide.

On purifie le résidu ainsi obtenu par chromatographie sur gel de silice, éluant : $CH_2Cl_2/CH_3OH$ 100/2 (v/v). On concentre les fractions pures et prépare le chlorhydrate dans l'éther isopropylique, on filtre lave à l'éther et sèche sous vide. On obtient ainsi 1,3 g du produit attendu.

F = 164°C

EXEMPLE 21

Chlorhydrate de 3-[2-(4-benzyl-1-pipéridinyl)éthyl]-3-(3,4-dichlorophényl)-1-(3-méthoxyphényl)acétylazétidine.

(I) : 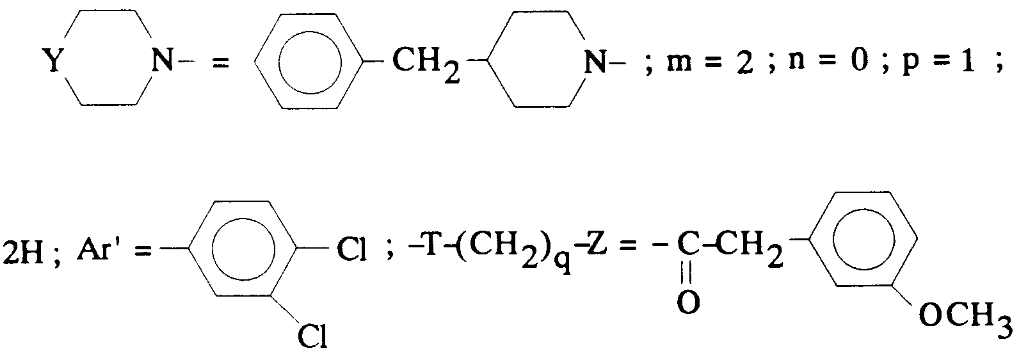 ; m = 2 ; n = 0 ; p = 1 ; Q = 2H ;

Le composé ci-dessus a été préparé selon le Schéma 3 de la description.

A) 3-Tétrahydropyranyloxyéthyl-3-(3,4-dichlorophényl)-1-(3-méthoxyphényl)acétyl azétidine.

A 1 g de 3-tétrahydropyranyloxyéthyl-3-(3,4-dichlorophényl)azétidine en solution dans 50 ml de dichlorométhane en présence de 1 g de triéthylamine et 0,5 g d'acide 3-méthoxyphénylacétique, on ajoute 1,5 g de BOP. On agite le mélange réactionnel pendant 1 heure à température ambiante, évapore à sec, reprend le résidu à l'acétate d'éthyle, lave à l'eau, à la soude diluée, au tampon pH = 2 et finalement à l'eau saturée de NaCl. On sèche la phase organique sur $MgSO_4$ et évapore à sec. On purifie par chromatographie sur gel de silice, éluant : $CH_2Cl_2/CH_3OH$ 100/0,75 (v/v).

On obtient ainsi 0,50 g d'une huile.

B) 3-Méthanesulfonyloxyéthyl-3-(3,4-dichlorophényl)-1-(3-méthoxyphényl)acétyl azétidine.

A 0,50 g de produit préparé précédemment en solution dans 50 ml de méthanol on ajoute de l'éther saturé d'acide chlorhydrique jusqu'à pH = 1. On agite la solution à température ambiante pendant 1 heure, évapore à sec, reprend le résidu à l'eau, extrait à l'AcOEt, lave à l'eau, sèche sur $MgSO_4$ et évapore à sec.

L'huile obtenue est reprise dans 30 ml de dichlorométhane et on ajoute 0,20 g de triéthylamine et 0,12 g de chlorure de mésyle. On agite le mélange réactionnel à température ambiante pendant 1 heure, on évapore à sec, reprend le résidu à l'acétate d'éthyle, lave à l'eau, sèche sur $MgSO_4$ et évapore à sec.

On obtient ainsi 0,50 g d'une huile.

C) Composé 21.

0,50 g de produit précédemment décrit en solution dans 2 ml de diméthylformamide avec 0,40 g de 4-benzylpipéridine sont chauffés à 80°C pendant 3 heures. On refroidit le mélange réactionnel,, verse dans l'eau, extrait à l'acétate d'éthyle, lave à l'eau, sèche sur $MgSO_4$ et évapore à sec.

On purifie le résidu ainsi obtenu par chromatographie sur gel de silice, éluant : $CH_2Cl_2/CH_3OH$ 100/2,5 (v/v)

On concentre les fractions propres sous vide et prépare le chlorhydrate par addition d'éther saturé d'acide chlorhydrique. On reprend le résidu dans le dichlorométhane et précipite le chlorhydrate sur l'éther, filtre, lave à l'éther et sèche sous vide.

On obtient ainsi 0,22 g du produit attendu.

F = 102°C

EXEMPLE 22

Chlorhydrate de 4-[2-(4-benzyl-1-pipéridinyl)éthyl]-4-(3-méthylphényl)-1-(3-chlorophényl)acétylpipéridine.

(I) : 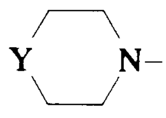 = 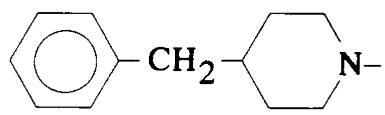 ; m = 2 ; n = 1 ; p = 2 ;

Q = 2H ; Ar' = 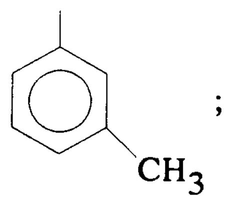 ; $-T-(CH_2)_q-Z$ = 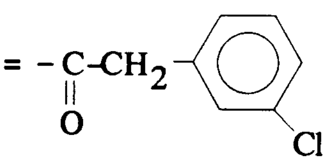

Le composé ci-dessus est préparé selon le Schéma 5 de la description.

A) 4-Méthanesulfonyloxyéthyl-4-(3-méthylphényl)-N-tritylpipéridine.

A 21 g de 4-(2-hydroxyéthyl)-4-(3-méthylphényl)-N-tritylpipéridine (préparé selon le schéma 5) en solution dans 200 ml de dichlorométhane et refroidis à 0°C, on ajoute goutte à goutte 3,8 ml de chlorure de méthanesulfonyle. Le mélange réactionnel est laissé une demi-heure à température ambiante, lavé deux fois à l'eau, séché sur $MgSO_4$ et concentré sous vide.
On obtient ainsi 23,5 g d'une mousse.

B) 4-[2-(4-benzyl-1-pipéridinyl)éthyl]-4-(3-méthylphényl)-N-tritylpipéridine.

18,5 g du mésylate précédemment décrit et 13,5 g de 4-benzylpipéridine en solution dans 40 ml de diméthylformamide sont chauffés pendant 4 heures à 60°C. On verse le mélange réactionnel sur 500 ml d'eau glacée, filtre le précipité et rince à l'eau. On reprend le précipité dans l'éther, lave avec NaOH diluée puis à l'eau, sèche sur $MgSO_4$ et concentre à sec.
On purifie le résidu obtenu par chromatographie sur gel de silice, éluant : $CH_2Cl_2/CH_3OH$ 100/3 (v/v). On obtient ainsi 18 g d'une mousse.

C) Dichlorhydrate de 4-[2-(4-benzyl-1-pipéridinyl)éthyl]-4-(3-méthylphényl)-pipéridine.

18 g du produit précédent en solution dans 150 ml d'acide formique à 50 % sont chauffés à 60°C pendant 30 minutes. On refroidit, sépare le triphénylcarbinol par filtration, rince à l'eau, concentre à sec. Le résidu est repris à l'eau, lavé à l'éther, alcalinisé avec une solution de NaOH, extrait au dichlorométhane, séché sur $MgSO_4$ et concentré à sec.
La base est dissoute dans du dichlorméthane, on additionne de l'éther saturé d'acide chlorhydrique et concentre à sec. On agite le chlorhydrate ainsi préparé dans l'éther, filtre et sèche.

m = 12,7 g
F = 160°C.

D) Composé 22

A 2 g de produit précédemment préparé en solution dans 30 ml de dichlorométhane avec 0,77 g d'acide 3-chlorophénylacétique et 2,2 g de triéthylamine on ajoute 2,4 g de BOP. On agite le mélange réactionnel pendant 30 minutes à température ambiante, concentre à sec, reprend le résidu dans l'acétate d'éthyle, lave à l'eau, puis avec une solution de NaOH diluée, puis à l'eau saturée en NaCl, sèche sur $MgSO_4$ et concentre sous vide. On purifie le résidu

par chromatographie sur gel de silice, éluant : $CH_2Cl_2/CH_3OH$ 100/10 (v/v). On prépare le chlorhydrate par addition d'éther saturé d'acide chlorhydrique et, on concentre à sec. On reprend le résidu dans l'éther isopropylique filtre et sèche sous vide.

m = 2,1 g.

F = 106°C.

En procédant comme indiqué ci-dessus à l'exemple 22 on prépare les composés décrits dans le Tableau IV ci-dessous.

## TABLEAU IV

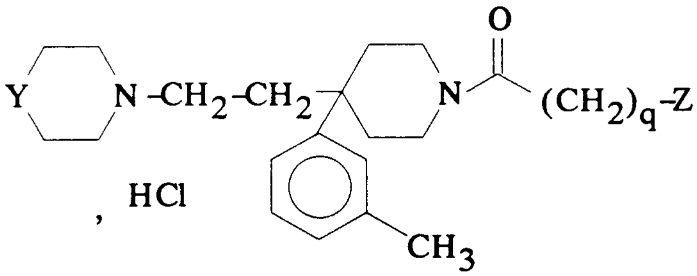

, HCl

| Exemple n° | 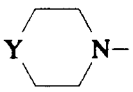 | $-(CH_2)_q-Z$ | F ; °C |
|---|---|---|---|
| 23 | 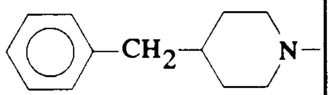 | 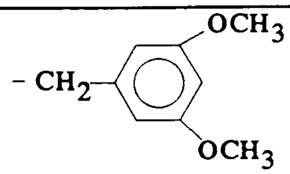 | 105 |
| 24 | 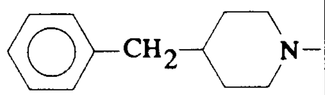 | 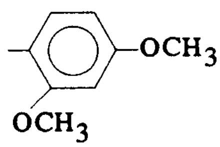 | 146 |
| 25 | 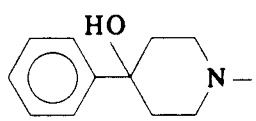 | 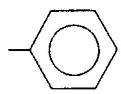 | 143 |

EXEMPLE 26

Chlorhydrate de 3-[3-(4-benzyl-1-pipéridinyl)propyl]-3-(3,4-dichlorophényl)-1-(3-méthoxyphényl)acétylpipéridine.

(I) : 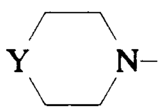 = 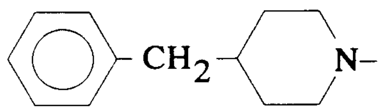 ; m = 3 ; n = 2 ; p = 1 ;

Q = 2H ; Ar' = 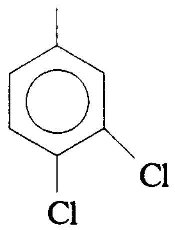 ; $-T-(CH_2)_q-Z$ = 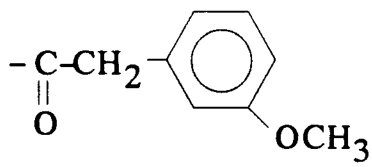

A) 3,4-Dichlorotétrahydropyranyloxypropyl-α-benzèneacétonitrile.

De façon identique à l'étape A selon l'exemple 1 et à partir de 37,2 g de 3,4-dichlorophénylacétonitrile et de 44,6 g de 3-bromopropoxytétrahydropyrane, on obtient 35 de produit attendu.

B) γ-Tétrahydropyranyloxypropyl-γ-cyano-3,4-dichlorobenzylbutanoate d'éthyle.

A partir de 35 g du produit précedemment obtenu et de 19,2 g de bromopropionate d'éthyle, en procédant de façon identique à l'étape B selon l'exemple 1 on obtient 28 g de produit attendu.

C) 5-Tétrahydropyranyloxypropyl-5-(3,4-dichlorophényl)pipéridone.

23 g du produit précédemment obtenu, en solution dans 650 ml d'éthanol, sont hydrogénés à pression atmosphérique et température ambiante en présence de nickel de Raney. Lorsque le volume théorique d'hydrogène est consommé, on filtre le catalyseur, évapore à sec, reprend le résidu à l'éther, lave à l'eau, au tampon pH = 2, sèche sur $Na_2SO_4$ et évapore à sec.
On obtient ainsi 18 de produit attendu.

D) 3-Tétrahydropyranyloxypropyl-3-(3,4-dichlorophényl) pipéridine.

14 g du produit précédemment obtenu en solution dans 50 ml de tétrahydrofurane sont ajoutés goutte à goutte à une suspension de 2,75 g d'hydrure de lithium aluminium chauffée à 60°C.
On maintient la température à 60°C pendant 1 heure. On refroidit le mélange réactionnel, hydrolyse par addition de 3 ml d'eau, 3 ml d'une solution de NaOH 4N et 9 ml d'eau. On sépare le minéral et évapore la phase organique sous vide.
On obtient ainsi 12,4 g de produit attendu.

E) 3-Tétrahydropyranyloxypropyl-3-(3,4-dichlorophényl)-1-(3-méthoxyphényl)-acétyl pipéridine.

3,9 g de BOP sont ajoutés à une solution de 3 g du produit précédemment préparé, 2,4 g de triéthylamine et 1,3 g d'acide 3-méthoxyphénylacétique dans 50 ml de dichlorométhane. On agite le mélange réactionnel pendant 1 heure à température ambiante, évapore à sec, reprend à l'AcOEt, lave à l'eau sèche sur $Na_2SO_4$ et évapore à sec.
On purifie le résidu ainsi obtenu par chromatographie sur gel de silice, éluant : $CH_2Cl_2/CH_3OH$ 100/2 (v/v).
La concentration des fractions pures fournit 3 g de produit attendu.

F) 3-Méthanesulfonyloxypropyl-3-(3,4-dichlorophényl)-1-(3-méthoxyphényl)acétyl pipéridine.

A partir de 3 g de produit précédemment préparé et de 0,68 g de chlorure de mésyle on obtient le composé attendu en procédant comme décrit selon l'étape C de l'exemple 2. Après purification par chromatographie sur gel de silice, éluant : $CH_2Cl_2/CH_3OH$ 100/1,5 (v/v) et concentration des fractions de produit pur, on obtient 2 g du produit attendu.

G) Composé 26

2 g de produit obtenu précédemment et 1,6 g de 4-benzylpipéridine en solution dans 3 ml de diméthylformamide sont chauffés pendant 1 heure à 70°C. On refroidit le mélange réactionnel, verse dans l'eau, extrait à l'éther, lave à l'eau et sèche sur $Na_2SO_4$, filtre et concentre sous vide.
On purifie le résidu ainsi obtenu par chromatographie sur gel de silice, éluant : $CH_2Cl_2/CH_3OH$ 100/3 (v/v). Les fractions pures sont concentrées sous vide, le résidu est repris dans l'acétone et on prépare le chlorhydrate par addition d'éther saturé d'acide chlorhydrique. On filtre le chlorhydrate, lave au pentane et sèche sous vide sur $P_2O_5$.
On obtient ainsi 1,1 g de produit attendu.
F = 108°C

EXEMPLE 27

Chlorhydrate de 3-[3-(4-phényl-4-acétamido-1-pipéridinyl)propyl]-3-(3,4-dichlorophényl)-1-benzoylpipéridine.

(I) : 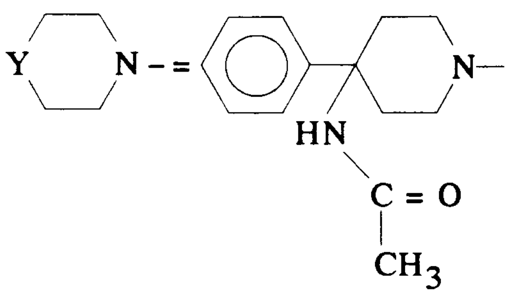 ; m = 3 ; n = 2 ; p = 1 ; Q = 2H ;

Ar' = 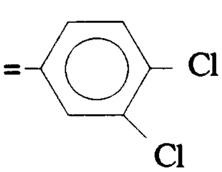 ; $-T-(CH_2)_q-Z$ = 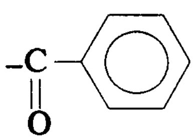

A) 3-Tétrahydropyranyloxypropyl-3-(3,4-dichlorophényl)-1-benzoylpipéridine.

1,13 g de chlorure de benzoyle sont additionnés à 3 g de 3-tétrahydropyranyloxypropyl-3-(3,4-dichlorophényl)pipéridine préparé selon l'exemple 26, étape D en présence de 1,62 g de triéthylamine en solution dans 50 ml de dichlorométhane. On agite le mélange réactionnel pendant 30 minutes à température ambiante, évapore à sec, reprend le résidu à l'éther, lave à l'eau, sèche sur $Na_2SO_4$ et évapore à sec. On purifie le résidu ainsi obtenu par chromatographie sur gel de silice, éluant: $CH_2Cl_2/CH_3OH$ 100/1 (v/v).
On obtient ainsi 3 g d'une huile.

B) 3-Méthanesulfonyloxypropyl-3-(3,4-dichlorophényl)-1-benzoylpipéridine.

A 3 g de produit préparé précédemment en solution dans 50 ml de méthanol on ajoute de l'éther saturé d'acide chlorhydrique jusqu'à pH = 1. On agite le mélange réactionnel pendant 30 minutes à température ambiante et évapore à sec. On reprend le résidu par 50 ml de dichlorométhane et 1,07 g de triéthylamine puis on ajoute 0,72 g de chlorure

de mésyle. On agite à température ambiante pendant 1 heure, évapore à sec, reprend le résidu dans l'acétate d'éthyle, lave à l'eau et sèche sur $Na_2SO_4$, filtre et concentre sous vide. On purifie le résidu par chromatographie sur gel de silice, éluant : $CH_2Cl_2$/AcOEt 100/3 (v/v).

On obtient ainsi 1,6 g du produit attendu.

C) Composé 27

1,5 g de produit préparé précédemment et 1,5 g de 4-phényl-4-acétamidopipéridine en solution dans 5 ml de diméthylformamide sont chauffés à 80°C pendant 4 heures. On refroidit le mélange réactionnel, verse dans l'eau, extrait au dichlorométhane, lave à l'eau et sèche sur $Na_2SO_4$, filtre et concentre sous vide. On purifie le résidu par chromatographie sur gel de silice, éluant : $CH_2Cl_2/CH_3OH$ 100/5 (v/v). On concentre les fractions de produit pur, reprend au dichlorométhane et prépare le chlorhydrate par addition d'éther saturé d'acide chlorhydrique, évapore à sec, reprend le résidu dans l'éthanol et précipite dans de l'éther. On filtre, lave le précipité au pentane et sèche sous vide.

m = 0,60 g

F = 184°C.

EXEMPLE 28

Chlorhydrate de 5-[3-(4-hydroxy-4-phényl-1-pipéridinyl)propyl]-5-(3,4-dichlorophényl)-1-(3-méthoxybenzyl)pipéridone.

(I) : 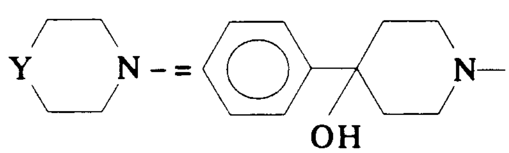 ; m = 3 ; n = 2 ; p = 1 ; Q = O ;

Ar' = 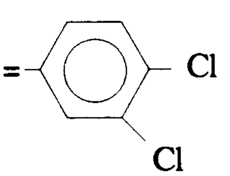 ; $-T-(CH_2)_q-Z$ = 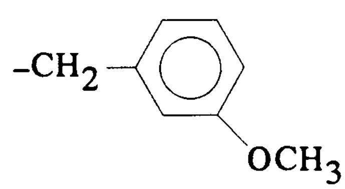

A) 5-Tétrahydropyranyloxypropyl-5-(3,4-dichlorophényl)-1-(3-méthoxybenzyl) pipéridone.

0,66 g de NaH à 60 % sont ajoutés à une solution de 6,4 g de 5-tétrahydropyranyloxypropyl-5-(3,4-dichlorophényl)pipéridone, décrit à l'étape C selon l'exemple 26, dans 60 ml de diméthylformamide. On agite le mélange réactionnel pendant 30 minutes à température ambiante. On ajoute ensuite goutte à goutte 2,5 g de chlorure de 3-méthoxybenzyle et chauffe le mélange réactionnel pendant 1 heure à 80°C. On évapore le diméthylformamide sous vide, extrait le résidu au dichlorométhane, lave à l'eau, sèche sur $Na_2SO_4$ et évapore à sec.

On purifie le résidu ainsi obtenu par chromatographie sur gel de silice, éluant : $CH_2Cl_2$/AcOEt 100/5 (v/v). On concentre les fractions de produit pur pour obtenir 6 g d'une huile.

B) 5-(3-Hydroxypropyl)-5-(3,4-dichlorophényl)-1-(3-méthoxybenzyl)pipéridone.

6 g du produit précédemment préparé en solution dans 50 ml de méthanol saturé d'acide chlorhydrique sont agités à température ambiante pendant une heure.

On évapore à sec pour obtenir 4,3 d'une huile.

C) 5-Méthanesulfonyloxypropyl-5-(3,4-dichlorophényl)-1-(3-méthoxybenzyl)pipéridone.

1,14 g de chlorure de mésyle sont ajoutés à 4,3 g du produit préparé précédemment en présence de 2 g de triéthylamine en solution dans 50 ml de dichlorométhane. On agite le mélange réactionnel pendant 1 heure à température ambiante, évapore à sec, reprend le résidu à l'AcOEt, lave à l'eau, à l'eau saturée de NaCl, sèche sur $Na_2SO_4$ et évapore à sec.

On purifie le résidu ainsi obtenu par chromatographie sur gel de silice, éluant : $CH_2Cl_2/CH_3OH$ 100/2 (v/v). On concentre les fractions de produit pur pour obtenir 4 g d'une huile.

D) Composé 28

4 g du produit préparé précédemment et 3,1 g de 4-hydroxy-4-phénylpipéridine en solution dans 5 ml de diméthylformamide sont chauffés à 80°C pendant 2 heures. On refroidit, verse dans l'eau, extrait à l'AcOEt, lave à l'eau, sèche sur $Na_2SO_4$ et évapore à sec. On reprend l'huile obtenue par de l'éther et prépare le chlorhydrate par addition d'éther saturé d'acide chlorhydrique. On filtre lave à l'éther et sèche sous vide.

m = 4 g

F = 110-117°C.

EXEMPLE 29

Dichlorhydrate de 3-[3-(4-hydroxy-4-phényl-1-pipéridinyl)propyl]-3-(3,4-dichlorophényl)-1-(3-méthoxybenzyl)pipéridine.

(I) : 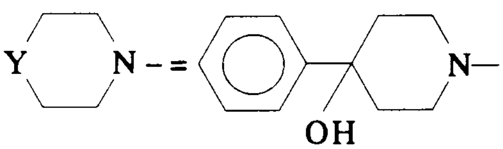 ; m = 3 ; n = 2 ; p = 1 ; Q = 2H ;

Ar' = 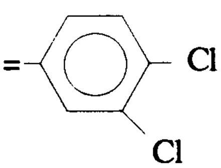 ; $-T-(CH_2)_q-Z$ = $-CH_2$ 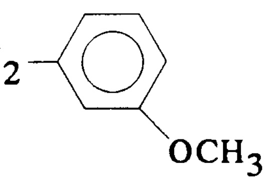

2 g de 5-[3-(4-hydroxy-4-phényl-1-pipéridinyl)propyl]-5-(3,4-dichlorophényl)-1-(3-méthoxybenzyl)pipéridone sont ajoutés à une suspension de 0,60 g d'hydrure de lithium aluminium dans 50 ml de tétrahydrofurane. On chauffe le mélange réactionnel pendant 1 heure à 60°C refroidit, hydrolyse par 5 ml d'eau, filtre le minéral et évapore à sec.

On purifie le résidu ainsi obtenu par chromatographie sur gel de silice, éluant : $CH_2Cl_2/CH_3OH$ 100/5 (v/v), on concentre les fractions de produit pur et prépare le chlorhydrate dans le dichlorométhane par addition d'éther saturé d'acide chlorhydrique.

On sépare le chlorhydrate par filtration, lave à l'éther et sèche sous vide sur $P_2O_5$.

m = 1,5 g

F = 106-175°C.

EXEMPLE 30

Dichlorhydrate de 3-[2-(4-hydroxy-4-phényl-1-pipéridinyl)éthyl]-3-(3,4-dichlorophényl)-1-benzylpyrolidine.

(I) : 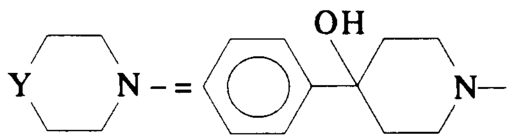 ; m = 2 ; n = 1 ; p = 1 ; Q = 2H ;

Ar' = 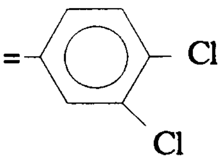 ; $-T-(CH_2)_q-Z$ = $-CH_2-$ 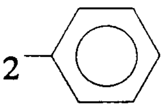

En procédant selon l'exemple 29 et à partir du produit décrit à l'exemple 5, on obtient le produit attendu. F = 170°C.

EXEMPLE 31

Dichlorhydrate de 3-[2-(4-benzyl-1-pipéridinyl)éthyl]-3-(1-naphtyl)-1-benzylpipéridine.

(I) : 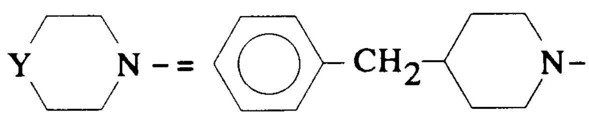 ; m = 2 ; n = 2 ; p = 1 ;

Q = H ; Ar' = 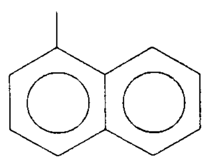 ; $-T-(CH_2)_q-Z$ = $-CH_2-$ 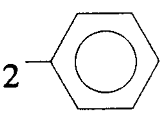

En procédant selon l'exemple 29, à partir du produit décrit à l'exemple 17, on obtient le composé ci-dessus. F = 140°C.

EXEMPLE 32

Chlorhydrate de 3-[2-(4-benzyl-1-pipéridinyl)éthyl]-3-(3,4-dichlorophényl)-1-(3-isopropoxyphényl)acétylpipéridine (-).

(I) : 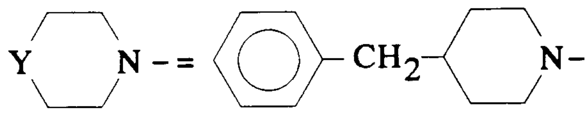 ; m = 2 ; n = 2 ; p = 1 ;

Q = 2H ; Ar' = 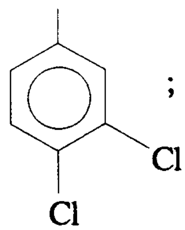 ; $-T-(CH_2)_q-Z$ = 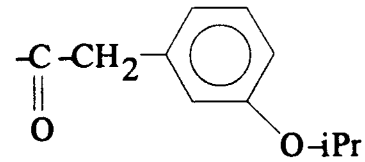

I - PREPARATION DE L'AMINOALCOOL OPTIQUEMENT PUR.

A) 3-(2-hydroxyéthyl)-3-(3,4-dichlorophényl)pipéridine.

A 55 g de 3-tétrahydropyranyloxyéthyl-3-(3,4-dichlorophényl)pipéridine en solution dans 200 ml de méthanol on ajoute de l'éther chlorhydrique jusqu'à pH = 1. On agite une demi-heure à température ambiante, concentre à sec, reprend le résidu à l'eau, basifie avec une solution d'hydroxyde de sodium, extrait au dichlorométhane, lave avec une solution saturée de NaCl, sèche sur $Na_2SO_4$ et évapore à sec. On obtient une huile.

On reprend par 200 ml d'un mélange éther isopropylique/éther 50/50 (v/v). On agite, filtre, lave à l'éther et sèche sous vide sur $P_2O_5$.

m = 45 g

F = 122°C.

B) 3-(2-hydroxyéthyl)-3-(3,4-dichlorophényl)pipéridine (+).

A 43 g de produit obtenu précédemment en solution au reflux dans 250 ml d'éthanol 100°, on ajoute 23,54 g d'acide L (+) tartrique en solution dans 750 ml d'éthanol 100°. On chauffe le mélange réactionnel à reflux pendant une demi-heure, laisse revenir à température ambiante, on filtre les cristaux obtenus, lave à l'éthanol 100° et sèche sous vide à 50°C sur $P_2O_5$.

m = 31 g

On recristallise ensuite dans 540 ml d'éthanol 100°, filtre, lave à l'éther et sèche sous vide sur $P_2O_5$.

m = 25 g

$[\alpha]_D^{20}$ = + 8,5 (c = 1, $H_2O$)

Le tartrate est ensuite repris dans l'eau, on neutralise avec une solution de NaOH, extrait au dichlorométhane, lave à l'eau, sèche sur $Na_2SO_4$ et évapore à sec. On reprend l'huile dans un mélange éther/éther isopropylique, filtre les cristaux, lave à l'éther et sèche sous vide à 50°C.

m = 13,5 g

F = 138°C

$[\alpha]_D^{20}$ =+ 8,2 (c = 1, $CH_3OH$)

C) 3-(2-Hydroxyéthyl)-3-(3,4-dichlorophényl)pipéridine (-).

En procédant comme précédemment à partir de l'acide D (-) tartrique on obtient, l'énantiomère (-).

F = 139°C

$[\alpha]_D^{20}$ = - 8,4° (c = 1, $CH_3OH$)

II - PREPARATION DU COMPOSE 32

A) 3-(2-Hydroxyéthyl)-3-(3,4-dichlorophényl)-1-*tert*-butylcarbamoylpipéridine.

A 13 g de 3-(2-hydroxyéthyl)-3-(3,4-dichlorophényl)pipéridine (+) en solution dans 100 ml de dioxane on additionne 12,4 g de di-*tert*-butyldicarbonate. On agite ensuite pendant 1 heure à 40°C. On évapore à sec, reprend le résidu à l'éther, lave à l'eau puis avec une solution tampon pH = 2 et finalement à l'eau. On sèche sur $Na_2SO_4$, filtre et évapore à sec. On purifie le résidu par chromatographie sur gel de silice, éluant : $CH_2Cl_2/CH_3OH$ 100/2 (v/v). On obtient ainsi après concentration des fractions pures 16,7 g de produit attendu sous forme d'huile.

B) 3-Méthanesulfonyloxyéthyl-3-(3,4-dichlorophényl)-1-*tert*-butylcarbamoylpipéridine.

A 16,5 g du produit précédemment préparé en solution dans 100 ml de dichlorométhane en présence de 4,9 g de triéthylamine, on ajoute goutte à goutte, 5,5 g de chlorure de mésyle. On agite une demi-heure à température ambiante, évapore à sec, reprend le résidu à l'éther, lave à l'eau, sèche sur $Na_2SO_4$ et concentre sous vide. On obtient ainsi 19 g d'une huile.

C) 3-[2-(4-Benzyl-1-pipéridinyl)éthyl]-3-(3,4-dichlorophényl)-1-*tert*-butylcarbamoyl -pipéridine.

18 g de produit précédent et 14 g de 4-benzylpipéridine en solution dans 40 ml de diméthylformamide sont chauffés à 80°C pendant 3 heures. On évapore ensuite le diméthylformamide, reprend le résidu à l'eau, extrait à l'éther, lave à l'eau, sèche sur $Na_2SO_4$ et concentre sous vide. On purifie le résidu par chromatographie sur gel de silice, éluant : $CH_2Cl_2/CH_3OH$ 100/3 (v/v). Les fractions pures sont concentrées sous vide.

m = 15 g.

D) Dichlorhydrate-3-[2-(4-benzyl-1-pipéridinyl)éthyl]-3-(3,4-dichlorophényl)-pipéridine (-).

15 g de produit précédent en solution dans 75 ml de méthanol, 60 ml d'acide chlorhydrique concentré et 15 ml d'eau sont agités à température ambiante pendant 1 heure. On évapore à sec, reprend le résidu par 100 ml de dichlorométhane et précipite sur de l'éther. On filtre le précipité, lave à l'éther et sèche sous vide.

m = 11,5 g

F = 175°C

$[\alpha]_D^{20}$ =- 2,2° (c = 1, $CH_3OH$)

E) Composé 32

10,6 g de BOP sont ajoutés à 11 g du produit précédent, 6,09 g de triéthylamine et 4,65 g d'acide 3-isopropoxyphénylacétique en solution dans 100 ml de dichlorométhane. On agite à température ambiante pendant 1 heure, évapore à sec, reprend le résidu à l'acétate d'éthyle, lave à l'eau, sèche sur $Na_2SO_4$, filtre et concentre sous vide. On purifie le résidu par chromatographie sur gel de silice, éluant : $CH_2Cl_2/CH_3OH$ 100/5 (v/v). Les fractions propres sont concentrées sous vide, on prépare le chlorhydrate dans le $CH_2Cl_2$ par addition d'éther saturé d'acide chlorhydrique, évapore à sec, cristallise dans l'éther isopropylique, filtre, lave à l'éther et sèche sous vide.

m = 11,4 g

F = 105°C

$[\alpha]_D^{20}$ =- 2,9° (c = 1, $CH_3OH$)

EXEMPLE 33

Chlorhydrate de 3-[2-(4-benzyl-1-pipéridinyl)éthyl]-3-(3,4-dichlorophényl)-1-(3-isopropoxyéthyl)acétylpipéridine (+).

(I) :

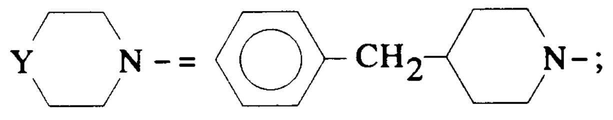

; m = 2 ; n = 2 ; p = 1 ;

Q = 2H ; Ar' =

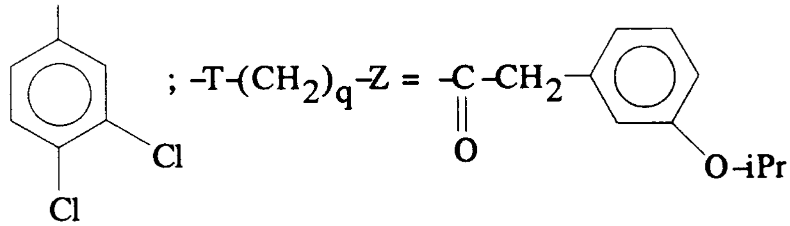

En procédant selon l'exemple 32 et en utilisant comme produit de départ l'énantiomère 3-(2-hydroxyéthyl)-3-(3,4-dichlorophényl)-pipéridine (-), on obtient le composé 33 ci-dessus, énantiomère (+).

F = 105°C

$[\alpha]_D^{20}$ =+ 3,0° (c = 1, $CH_3OH$)

EXEMPLE 34

Chlorhydrate de 3-[2-(4-hydroxy-4-phényl-1-pipéridinyl)éthyl]-3-(3,4-dichloro-phényl)-1-benzoylpipéridine (-).

(I) :

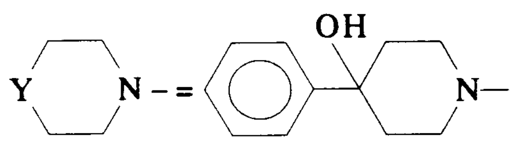

; m = 2 ; n = 2 ; p = 1 ; Q = H ;

Ar' =

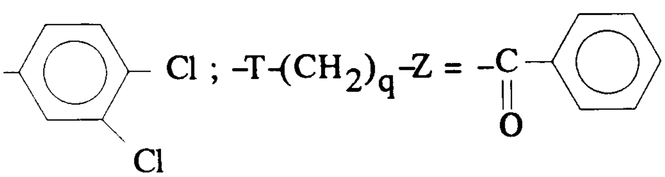

A) 3-[2-(4-hydroxy-4-phényl-1-pipéridinyl)éthyl]-3-(3,4-dichlorophényl)-1-*tert*-butyl-carbamoylpipéridine.

0,9 g de 3-méthanesulfonyloxyéthyl-3-(3,4-dichlorophényl)-1-*tert*-butylcarbamoyl -pipéridine préparés selon l'exemple 32, étape B), et 0,88 g de 4-hydroxy-4-phénylpipéridine en solution dans 3 ml de diméthylformamide sont chauffés à 80°C pendant 2 heures. On évapore à sec, reprend le résidu à l'eau, extrait à l'AcOEt, lave à l'eau saturée de NACl, sèche sur $MgSO_4$ et évapore à sec. On purifie le résidu par chromatographie sur gel de silice, éluant : $CH_2Cl_2/CH_3OH$ 100/2 (v/v). On concentre les fractions propres pour obtenir 0,8 d'une huile.

B) 3-[2-(4-hydroxy-4-phényl-1-pipéridinyl)éthyl]-3-(3,4-dichlorophényl)pipéridine.

0,8 g de produit précédent en solution dans 5 ml de méthanol, 4 ml d'acide chlorhydrique concentré et 1 ml d'eau est agité pendant 1 heure à température ambiante. On évapore ensuite à sec et utilise le résidu tel quel à l'étape suivante.

m = 0,77 g

C) Composé 34

A 0,77 g de produit précédent et 0,3 g de triéthylamine en solution dans 30 ml de dichlorométhane, on ajoute 0,26 g de chlorure de benzoyle. On agite le mélange réactionnel pendant 1 heure à température ambiante, évapore à sec, reprend le résidu dans l'acétate d'éthyle, lave à l'eau, sèche sur $Na_2SO_4$ et évapore à sec. On purifie par chromatographie sur gel de silice, éluant : $CH_2Cl_2/CH_3OH$ 100/3 (v/v). On concentre les fractions propres, reprend au $CH_2Cl_2$ et prépare le chlorhydrate par addition d'éther saturé d'acide chlorhydrique. On évapore à sec, cristallise le résidu dans l'éther, filtre, lave à l'éther et sèche sous vide.

m = 0,2 g
F = 176°C
$[\alpha]_D^{20}$ =- 32,0° (c = 1, $CH_3OH$)

EXEMPLE 35

Chlorhydrate de 3-[2-(4-hydroxy-4-phényl-1-pipéridinyl)éthyl]-3-(3,4-dichloro-phényl)-1-benzoylpipéridine (+).

(I) : 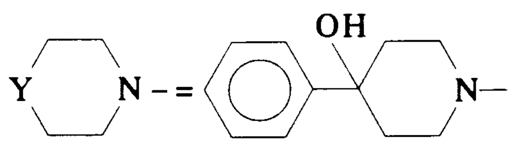 ; m = 2 ; n = 2 ; p = 1 ; Q = 2H ;

Ar' = 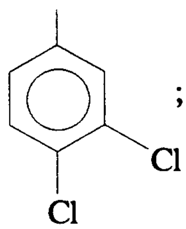 ; $-T-(CH_2)_q-Z$ = 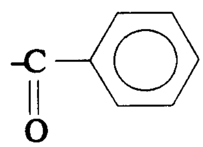

En procédant selon l'exemple 34 à partir du 3-(2-hydroxyéthyl)-3-(3,4-dichloro -phényl)pipéridine (-) on obtient l'énantiomère (+) ci-dessus.

F = 176°C
$[\alpha]_D^{20}$ =+ 32,5° (c = 1, $CH_3OH$)

EXEMPLE 36

Iodure de N(a)-méthyl-3-[2-(4-benzyl-1-pipéridinium)éthyl]3-(3,4-dichloro-phényl)-1-(3-isopropoxyphényl)acétylpipéridine.

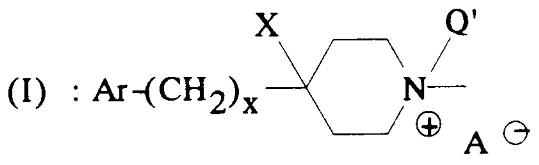 = 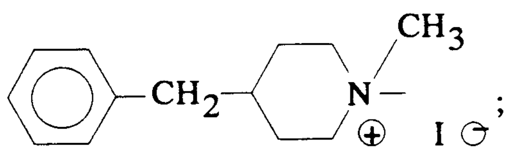 ;

m = 2 ; n = 2 ; p = 1 ; Q = 2H ;

Ar' = 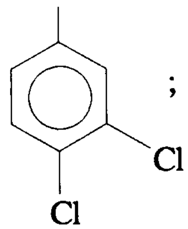 ; $-T-(CH_2)_q-Z$ = 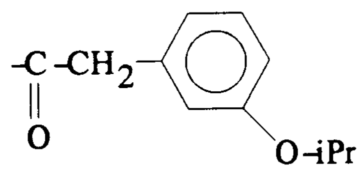

1 g du produit décrit à l'exemple 14 en solution dans 10 ml d'iodure de méthyle est agité à température ambiante pendant 24 heures. On concentre ensuite sous vide. Le résidu est chromatographié sur gel de silice, éluant : $CH_2Cl_2/CH_3OH$ 100/3 (v/v). Le premier produit élué coffespond à celui dont le méthyle en position sur l'azote (b) de la 4-benzylpipéridine est en position axiale.

m = 0,35 g

Spectre de RMN $^{13}C$:

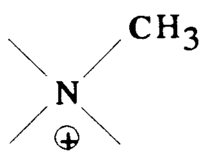 : 43,369 ppm

EXEMPLE 37

Iodure de N(e)-méthyl-3-[2-(4-benzyl-1-pipéridinium)éthyl]3-(3,4-dichlorophényl)-1-(3-isopropoxyphényl)acétylpipéridine.

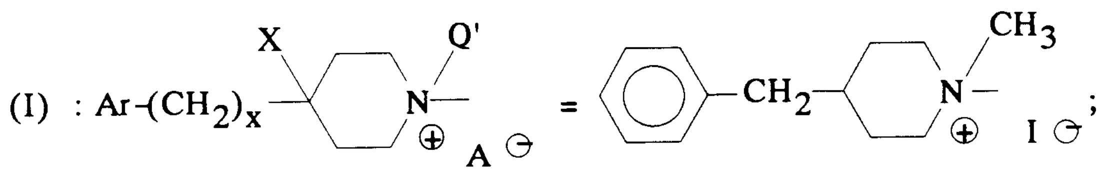

m = 2 ; n = 2 ; p = 1 ; Q = 2H ;

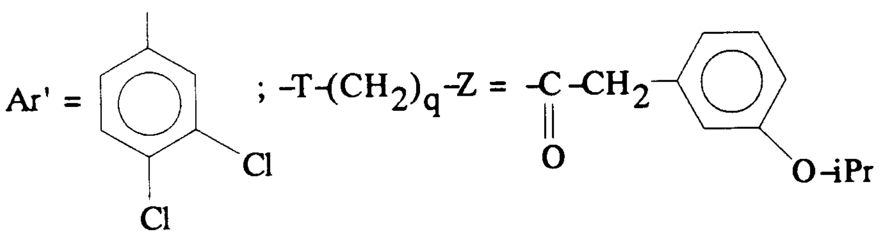

En procédant comme pour l'exemple 36 précédemment décrit et en recueillant la fraction éluée en deuxième lieu, on obtient le produit dont le méthyle sur l'azote (b) de la 4-benzylpipéridine est en position équatoriale.

m = 0,15 g

Spectre de RMN $^{13}C$:

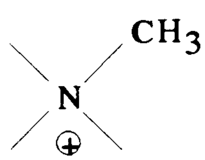 : 50,614 ppm

En procédant selon les exemples 36 et 37 ci-dessus, on prépare les sels d'ammonium quaternaires décrits dans le Tableau V ci-dessous.

## TABLEAU V

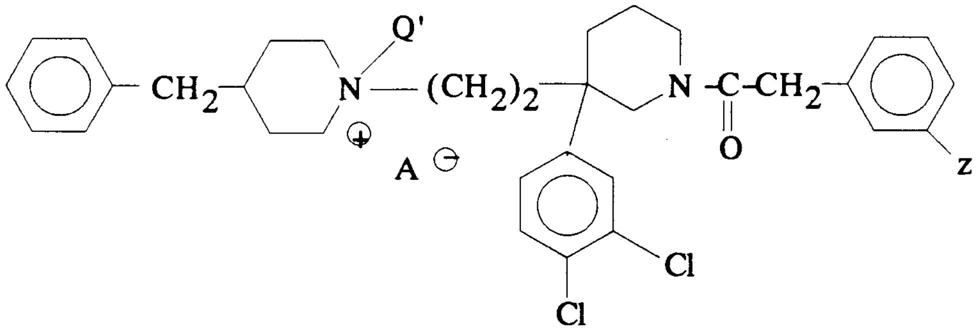

| Exemple n° | Q' (conformation) | A $^{\ominus}$ | z | F ; °C |
|---|---|---|---|---|
| 38 | 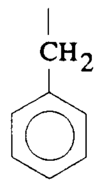 (a) | $Br^-$ | –O–iPr | 124 |
| 39 | 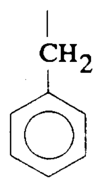 (e) | $Br^-$ | –O–iPr | 144 |
| 40 | $-C_2H_5$ (a) | $I^-$ | –O–iPr | 116 |
| 41 | $-C_2H_5$ (e) | $I^-$ | –O–iPr | 122 |
| 42 | $-CH_3$ (a) | $I^-$ | $-OC_2H_5$ | 120 |
| 43 | $-CH_3$ (e) | $I^-$ | $-OC_2H_5$ | 126 |

EXEMPLE 44

Chlorure de N(a)-méthyl-3-[2-(4-benzyl-1-pipéridinium)éthyl]-3-(3,4-dichlorophényl )-1-(3-isopropoxyphényl)acétylpipéridine (-).

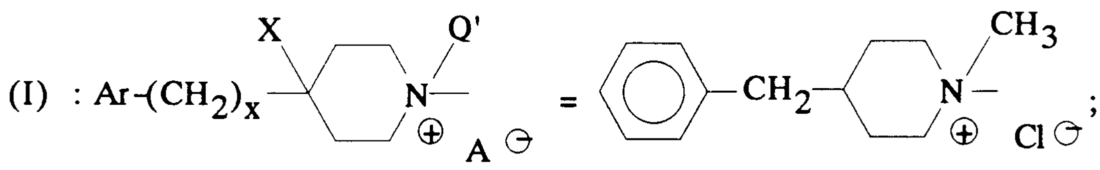

m = 2 ; n = 2 ; p = 1 ; Q = 2H ;

Ar' = 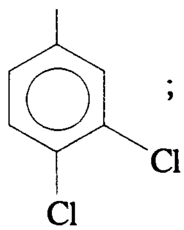 ; $-T-(CH_2)_q-Z$ = 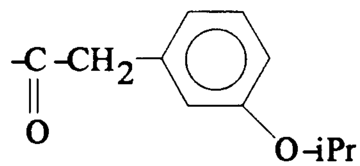

A) Préparation du dérivé iodure.

10 g du produit décrit à l'exemple 32 en solution dans 50 ml d'iodure de méthyle sont agités à température ambiante pendant 2 heures. On évapore à sec et le résidu est chromatographié sur gel de silice, éluant : $CH_2Cl_2/CH_3OH$ 100/3 (v/v). Le conformère qui est élué en premier correspond à celui dont le méthyle est en position axiale sur l'azote (b) de la 4-benzylpipéridine.

B) Préparation du dérivé chlorure.

L'ion iodure est ensuite échangé par l'ion chlorure en éluant le produit sur une résine échangeuse d'ion l'Amberlite IRA68.[R]

On obtient ainsi 5,6 g du chlorure d'ammonium quaternaire.

F = 103°C

$[\alpha]_D^{20}$ =- 12,8° (c = 1, $CH_3OH$)

EXEMPLE 45

Chlorure de N(a)-méthyl-3-[2-(4-benzyl-1-pipéridinium)éthyl]-3-(3,4-dichlorophényl)-1-(3-isopropoxyphényl)acétylpipéridine (+).

(I) : 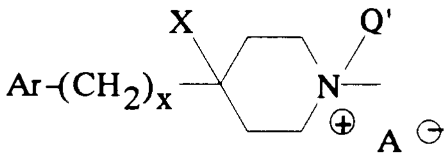 = 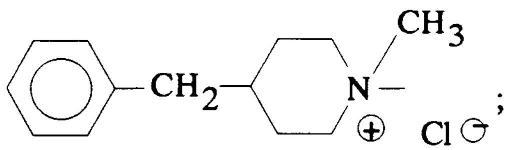 ;

m = 2 ; n = 2 ; p = 1 ; Q = 2H ;

Ar' = 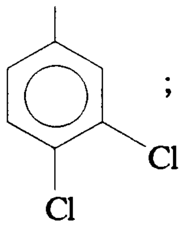 ; $-T-(CH_2)_q-Z$ = 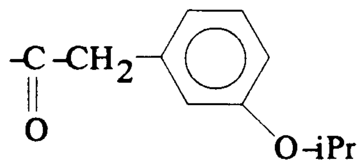

En procédant de manière identique à l'exemple 44, à partir du produit décrit à l'exemple 33, on obtient 8,9 g du sel d'ammonium quaternaire attendu.

F = 104°C

$[\alpha]_D^{20}$ =+ 13,0° (c = 1, $CH_3OH$)

EXEMPLE 46

Iodure de N(e)-Méthyl-3-[2-(4-benzyl-1-pipéridinium)éthyl]-3-(3,4-dichlorophényl)-1-(3-isopropoxyphényl)acétylpipéridine (-).

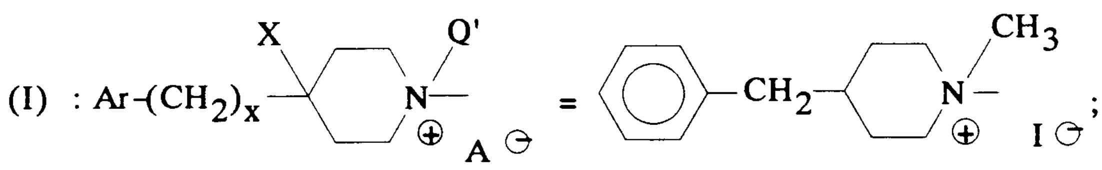

m = 2 ; n = 2 ; p = 1 ; Q = 2H ;

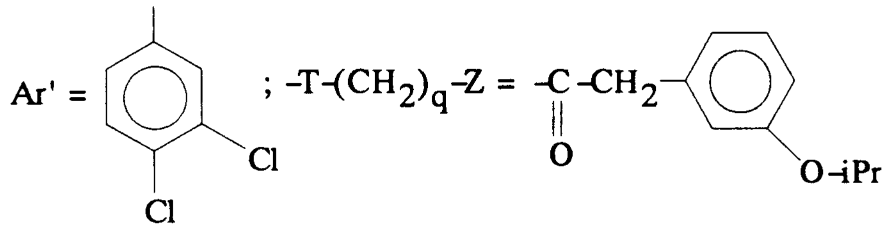

En procédant selon l'exemple 44 A) et en recueillant la fraction éluée en deuxième lieu, on obtient l'énantiomère dont le méthyle sur l'azote (b) de la 4-benzylpipéridine est en position équatoriale. On obtient ainsi 2,6 g du sel d'ammonium quaternaire.

F = 110°C

$[\alpha]_D^{20}$ =- 0,1° (c = 1, $CH_3OH$)

EXEMPLE 47

Iodure de N(e)-Méthyl-3-[2-(4-benzyl-1-pipéridinium)éthyl]-3-(3,4-dichlorophényl)-1-(3-isopropoxyphényl)acétylpipéridine (+).

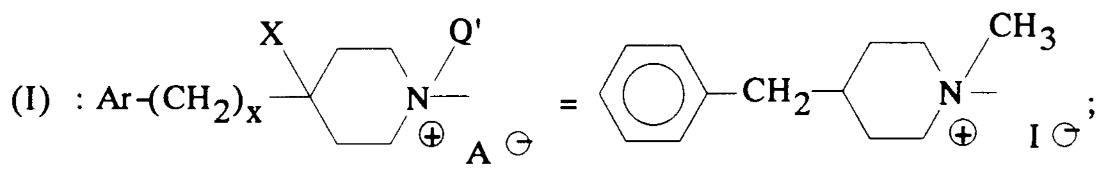

$$m = 2 ; n = 2 ; p = 1 ; Q = 2H ;$$

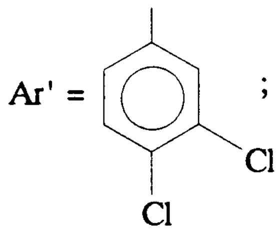

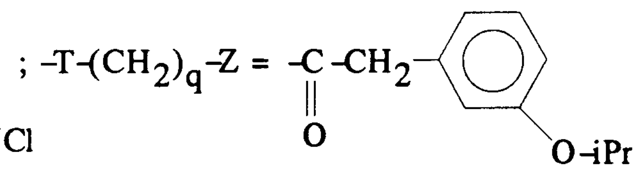

En procédant selon l'exemple 46 à partir du produit décrit à l'exemple 33, on obtient le produit attendu.
F = 110°C
$[\alpha]_D^{20}$ =+ 0,1° (c = 1, $CH_3OH$)

EXEMPLE 48

N-oxyde de 3-[2-(4-benzyl-1-pipéridinium)éthyl]-3-(3,4-dichlorophényl)-1-(3-isopropoxyphényl)acétylpipéridine.

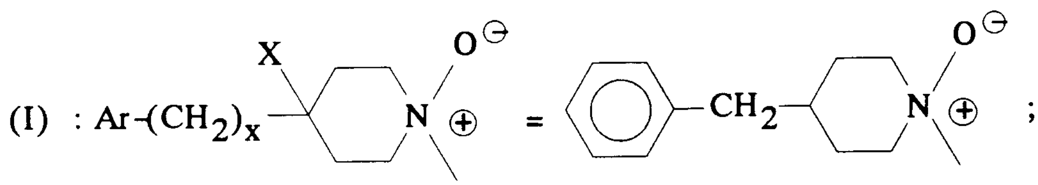

$$m = 2 ; n = 2 ; p = 1 ;$$

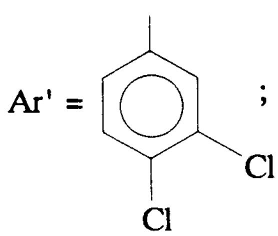

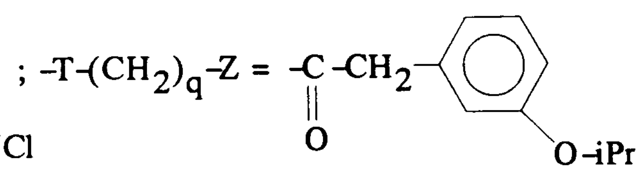

2 g de la base libre du composé de l'exemple 14 sont mis en solution dans 20 ml de tétrahydrofurane. On ajoute 1,1 g d'acide métachloroperbenzoïque et agite la mélange réactionnel pendant 2 heures à température ambiante. On concentre sous vide jusqu'à un volume de 5 ml et dilue le résidu dans 10 ml de dichlorométhane. On lave deux fois la solution avec une solution saturée de $NaHCO_3$, décante, sèche sur $MgSO_4$ et concentre sous vide. On chromatographie le résidu sur gel de silice, éluant : $CH_2Cl_2/CH_3OH$ 100/5 (v/v). On concentre les fractions de produit pur sous vide et cristallise le résidu dans l'éther isopropylique.

m = 1,47 g
F = 135°C.

EXEMPLE 49

Dichlorhydrate de 3-[2-(4-benzyl-1-pipéridinyl)éthyl]-3-(3,4-dichlorophényl)-pipéridine.

Intermédiaire de synthèse de formule (II).

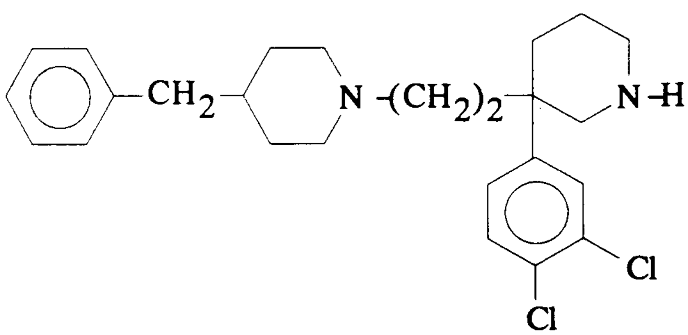

A) 4-(4-benzyl-1-pipéridinyl)-2-(3,4-dichlorophényl)butyronitrile.

A 94 g de 3,4-dichlorophénylacétonitrile en solution dans 500 ml d'éther anhydre, on ajoute par petites portions 23,5 g d'amidure de sodium. On agite ensuite pendant 1 heure à température ambiante puis pendant 3 heures à reflux. On refroidit le mélange à 0°C et on ajoute goutte à goutte 129 g de 2-(4-benzyl-1-pipéridinyl)-1-chloroéthane en solution dans 300 ml d'éther. On laisse revenir le mélange réactionnel à température ambiante puis on chauffe pendant 3 heures à reflux. On refroidit, verse sur 600 ml d'eau, décante la phase organique, lave à l'eau et extrait 2 fois avec 500 ml d'une solution d'HCl 15 %. On agite la phase aqueuse et le produit précipite sous forme de chlorhydrate. On filtre, lave à l'eau et sèche sous vide. On recristallise le résidu dans 600 ml d'isopropanol. On obtient ainsi 95 g.
Le produit est repris dans l'eau et la solution est neutralisé par une solution de NaOH. On extrait à l'éther, lave à l'eau, sèche sur $Na_2SO_4$ et évapore à sec. On obtient 87 g d'une huile.

B) γ-[2-(4-benzyl-1-pipéridinyl)éthyl]γ-cyano-3,4-dichlorobenzylbutanoate d'éthyle.

87 g du produit précédemment décrit, 28 g d'acrylate d'éthyle et 2,5 ml de triton B en solution dans 45 ml de dioxane sont chauffés pendant 24 heures à 80°C. On refroidit, reprend à l'éther, lave à l'eau, sèche sur $Na_2SO_4$ et évapore à sec. On obtient 109,5 g d'une huile.

C) 5-[2-(4-benzyl-1-pipéridinyl)éthyl]-5-(3,4-dichlorophényl)pipéridone.

100 g du produit préparé précédemment en solution dans 1,5 litre d'éthanol sont hydrogénés à 60°C et à pression atmosphérique en présence de Ni de Raney. Lorsque le volume d'hydrogène est consommé, on filtre le catalyseur, évapore à sec, reprend au dichlorométhane, lave à l'eau et sèche sur $Na_2SO_4$. On forme ensuite le chlorhydrate que l'on recristallise dans 220 ml d'isopropanol. On filtre et sèche sous vide. Le produit est repris dans l'eau, on neutralise avec une solution de NaOH, extrait à l'éther et sèche sur $Na_2SO_4$. On obtient 44 g d'une huile.

D) Composé 49

44 g du produit précédent en solution dans 200 ml de tétrahydrofurane sont ajoutés goutte à goutte à une suspension de 9,4 g d'hydrure de lithium aluminium dans 250 ml de tétrahydrofurane chauffé à 60°C. On continue à chauffer à reflux pendant 3 heures. On refroidit dans la glace et ajoute successivement 10 ml d'eau, 10 ml de NaOH 4N et 30 ml d'eau. On filtre le minéral et évapore le filtrat à sec, on reprend au dichlorométhane et prépare le chlorhydrate. On évapore à sec, triture dans du pentane, filtre et sèche sous vide.

m = 35 g

F = 170°C.

EXEMPLE 50

Chlorhydrate de 3-[2-(4-benzyl-1-pipéridinyl)éthyl]-3-(3,4-dichlorophényl)-1-(2-phényl-2-méthoxy)acétylpipéridine.

Diastéréoisomère le moins polaire.

(I) : 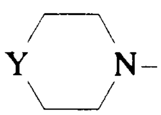 = 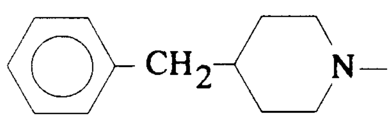 ; m = 2 ; n = 2 ; p = 1 ;

Q = 2H ; Ar' = 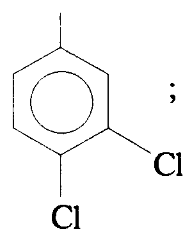 ; $-T-(CH_2)_q-Z$ = 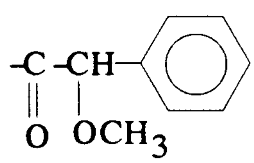

On agite pendant 2 heures 1,5 g de la diamine préparée à l'exemple 49 ; 1,06 g de triéthylamine ; 0,55 g d'acide (±) α-méthoxyphénylacétique et 1,6 g de BOP dans 25 ml de dichlorométhane. On évapore à sec, reprend le résidu à l'acétate d'éthyle, lave à l'eau, sèche sur $Na_2SO_4$ et évapore à sec. On purifie le résidu par chromatographie sur gel de silice, éluant : $CH_2Cl_2/CH_3OH$ 100/0,5 (v/v). Le produit qui est élué en premier est le produit attendu. Les fractions sont concentrées sous vide, on reprend au $CH_2Cl_2$, prépare le chlorhydrate, évapore à sec, triture dans du pentane, filtre et sèche sous vide.

m = 0,50 g

F = 134°C.

EXEMPLE 51

Chlorhydrate de 3-[2-(4-benzyl-1-pipéridinyl)éthyl]-3-(3,4-dichlorophényl)-1-(2-phényl-2-méthoxy)acétylpipéridine.

Diastéréoisomère le plus polaire.

(I) : 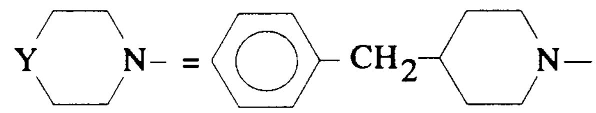 ; m = 2 ; n = 2 ; p = 1 ;

Q = 2H ; Ar' = 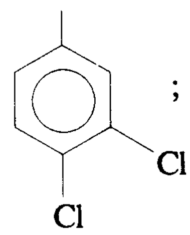 ; $-T-(CH_2)_q-Z$ = 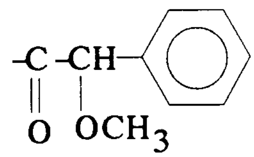

En procédant selon l'exemple 50 et en éluant avec un mélange $CH_2Cl_2/CH_3OH$ 100/2 (v/v), on obtient le diastéréoisomère le plus polaire. On prépare le chlorhydrate dans le dichlorométhane, évapore à sec, reprend dans du pentane.

m = 0,50 g
F = 118°C.

En procédant selon les exemples 50 et 51, on prépare les couples de diastéréoisomères 52, 53 ; 54,55 et 56, 57 décrits dans le tableau VI ci-dessous.

Le réactif acide α-hydroxy-3-isopropoxyphénylacétique utile pour la préparation des composés des exemples 56 et 57 qui est un produit nouveau, peut être préparé comme indiqué ci-dessous.

Acide α-hydroxy-3-isopropoxyphénylacétique.

<u>Etape 1</u>

A une solution de 50 g de 3-hydroxybenzaldéhyde dans 250 ml de DMF, on ajoute 60 g de $K_2CO_3$ Puis 60 ml de 2-iodopropane.

On chauffe le mélange réactionnel à 50°C pendant 18 heures. Le mélange obtenu est versé sur 2,5 l d'eau. On extrait à l'éther, lave avec une solution NaOH diluée, puis à l'eau. On sèche sur $MgSO_4$, évapore le solvant pour obtenir 53,5 g d'un résidu liquide.

<u>Etape 2</u>

53 g de produit obtenu selon l'étape 1 précédente sont ajoutés à une solution de 38 g de bisulfite de sodium dans 120 ml d'eau. On agite pendant 20 heures puis on ajoute à 20°C une solution de 44,2 g de cyanure de potassium dans 90 ml d'eau.

Après 2 heures, on extrait à l'éther, lave à l'eau, sèche sur $MgSO_4$ et évapore le solvant sous vide. Le résidu est chromatographié sur gel de silice, éluant : heptane/acétate d'éthyle 100/30 (v/v). On récupère 57 g de produit sous forme d'huile.

<u>Etape 3</u>

46 g de produit obtenu selon l'étape 2 précédente sont ajoutés à 50 ml d'eau et 50 ml d'HCl concentré. On chauffe à 110°C pendant 1 heure. Après refroidissement, on extrait à l'éther, lave à l'eau. L'acide est extrait avec une solution de NaOH diluée. On acidifie la phase aqueuse, extrait à l'éther, sèche sur $MgSO_4$, évapore les solvants.

L'acide est cristallisé dans un mélange toluène/pentane 1/2 (v/v).
m = 27,5 g

TABLEAU VI

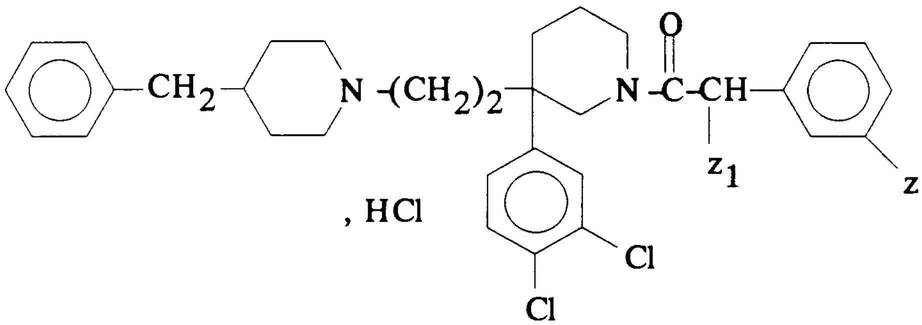

, HCl

| Exemple n° | $z_1$ | z | Propriété physique | F ; °C |
|---|---|---|---|---|
| 52 | $-CH_3$ | H | diastéréoisomère le moins polaire | 112 |
| 53 | $-CH_3$ | H | diastéréoisomère le plus polaire | 120 |
| 54 | $-C_2H_5$ | H | diastéréoisomère le moins polaire | 124 |
| 55 | $-C_2H_5$ | H | diastéréoisomère le plus polaire | 124 |
| 56 | -OH | -O-iPr | diastéréoisomère le moins polaire | 120 |
| 57 | -OH | -O-iPr | diastéréoisomère le plus polaire | 125 |

EXEMPLE 58

Chlorhydrate de 3-[2-(4-benzyl-1-pipéridinyl)éthyl]-3-(3,4-dichlorophényl)-1-[2-(3-chlorophényl)-2-hydroxy]acétylpipéridine (+).

(I) : 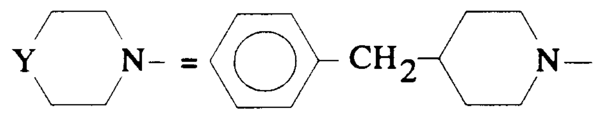 ; m = 2 ; n = 2 ; p = 1 ;

Q = 2H ; Ar' = 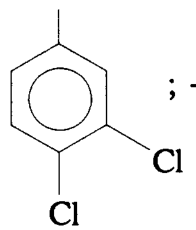 ; $-T-(CH_2)_q-Z$ = 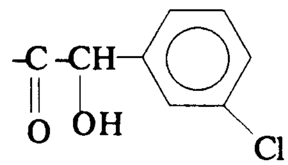

On agite pendant 2 heures, 0,67 g de dichlorhydrate de 3-[2-(4-benzyl-1-pipéridinyl)éthyl]-3-(3,4-dichlorophényl)pipéridine (-) décrit à l'exemple 32 étape D, 0,17 g de triéthylamine, 0,32 g d'acide S (+) $\alpha$-hydroxy-3-chlorophénylacétique et 0,82 g de BOP en solution dans 10 ml de dichlorométhane. On évapore à sec, reprend le résidu dans l'acétate d'éthyle, lave à l'eau, sèche sur $Na_2SO_4$, concentre sous vide. On purifie le résidu par chromatographie sur gel de silice, éluant : $CH_2Cl_2/CH_3OH$ 100/1,5 (v/v). On concentre les fractions propres sous vide, reprend dans le dichlorométhane, prépare le chlorhydrate, évapore à sec et reprend au pentane. On filtre, lave à l'éther et sèche sous vide.

m = 0,40 g
F = 122°C
$[\alpha]_D^{20}$ =+ 68,4° (c = 1, $CH_3OH$)

EXEMPLE 59

Chlorhydrate de 3-[2-(4-benzyl-1-pipéridinyl)éthyl]-3-(3,4-dichlorophényl)-1-[2-(3-chlorophényl)-2-hydroxy]acétylpipéridine (-).

(I) : 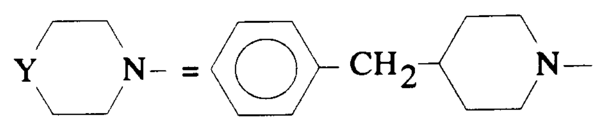 ; m = 2 ; n = 2 ; p = 1 ;

Q = 2H ; Ar' = 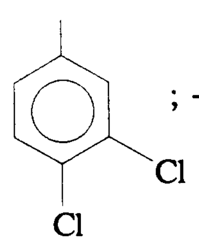 ; $-T-(CH_2)_q-Z$ = 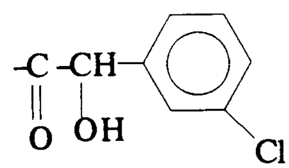

En procédant selon l'exemple 58 à partir de la 3-[2-(4-benzyl-1-picpéridinyl )éthyl]-3-(3,4-dichlorophényl)pipéridine (+) préparée selon l'exemple 32 étape D à partir du 3-(2-hydroxyéthyl)-3-(3,4-dichlorophényl)pipéridine (-) décrite à l'étape C de la préparation de l'aminoalcool optiquement pur et avec l'acide R (-) α-hydroxy-3-chlorophénylacétique, on obtient le produit attendu.

m = 0,50 g
F = 122°C
$[\alpha]_D^{20}$ =- 74° (c = 1, $CH_3OH$)

EXEMPLE 60

Chlorhydrate de 3-[2-(4-benzyl-1-pipéridinyl)éthyl]-3-(3,4-dichlorophényl)-1-[2-(3-chlorophényl)-2-hydroxy]acétylpipéridine (-).

(I) : 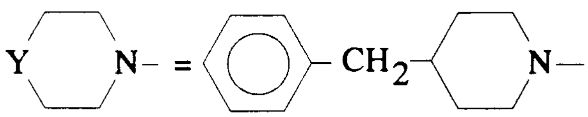 ; m = 2 ; n = 2 ; p = 1 ;

Q = 2H ; Ar' = 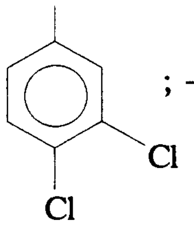 ; $-T-(CH_2)_q-Z$ = 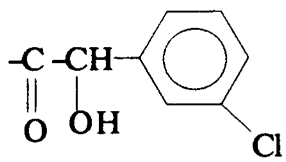

On agite à température ambiante pendant 2 heures 0,67 g de 3-[2-(4-benzyl-1-pipéridinyl)éthyl]-3-(3,4-dichlorophényl)pipéridine (-), 0,32 g d'acide R (-) α-hydroxy-3-chlorophénylacétique, 0,17 g de triéthylamine et 0,82 g de BOP en solution dans 50 ml de dichlorométhane. En procédant ensuite comme pour l'exemple 58, on obtient le produit attendu.

m = 0,40 g
F = 128°C
$[\alpha]_D^{20}$ =- 34° (c = 1, $CH_3OH$)

EXEMPLE 61

Chlorhydrate de 3-[2-(4-benzyl-1-pipéridinyl)éthyl]-3-(3,4-dichlorophényl)-1-[2-(3-chlorophényl)-2-hydroxy]acétylpipéridine (+).

(I) : 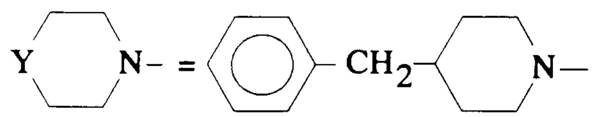 ; m = 2 ; n = 2 ; p = 1 ;

Q = 2H ; Ar' = 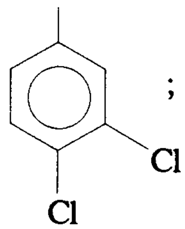 ; $-T-(CH_2)_q-Z$ = 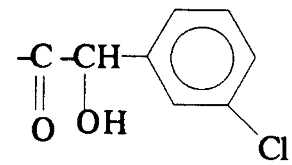

En procédant selon l'exemple 58, à partir de l'acide S (+) α-hydroxy-3-chlorophénylacétique et de la 3-[2-(4-benzyl-1-pipéridinyl)éthyl]-3-(3,4-dichlorophényl )pipéridine (+), préparée selon l'exemple 32 étape D et à partir de la 3-(2-hydroxyéthyl)-3-(3,4-dichlorophényl)pipéridine (-) décrite à l'étape C de la préparation de l'aminoalcool optiquement pur, on obtient le produit attendu.

m = 0,4 g
F = 127°C
$[\alpha]_D^{20}$ =+ 35° (c = 1, $CH_3OH$)

## Revendications

**Revendications pour les Etats contractants suivants : AT, BE, CH/LI, DE, DK, FR, GB, IT, LU, NL, PT, SE**

**1.** Composé de formule :

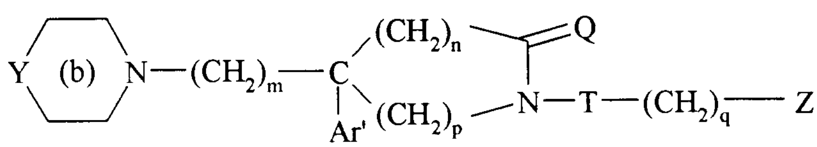

(I)

dans laquelle

- Y représente
  - soit un groupe Cy-N ou $Cy-CH_2-N$ dans lequel
    . Cy représente un phényle non substitué ou substitué une ou plusieurs fois par un des substituants choisis parmi : un atome d'halogène, un hydroxyle, un alcoxy en $C_1$-$C_4$, un alkyle en $C_1$-$C_4$, un trifluorométhyle, lesdits substituants étant identiques ou différents ; un groupe cycloalkyle en $C_3$-$C_7$ ; un groupe pyrimidyle ou un groupe pyridyle ;

- soit un groupe

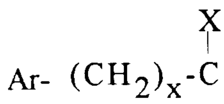

dans lequel

. Ar représente un phényle non substitué ou substitué une ou plusieurs fois par un des substituants choisis parmi : un atome d'halogène, un hydroxyle, un alcoxy en $C_1$-$C_4$, un trifluorométhyle, un alkyle en $C_1$-$C_4$, lesdits substituants étant identiques ou différents ; un groupe pyridyle ; un groupe thiényle;
. x est zéro ou un ;
. X représente un hydrogène, un hydroxyle, un alcoxy en $C_1$-$C_4$ ; un acyloxy en $C_1$-$C_4$ ; un carboxy ; un carbalcoxy en $C_1$-$C_4$ ; un cyano ; un groupe -$N(X_1)_2$ dans lequel les groupes $X_1$ représentent indépendamment l'hydrogène, un alkyle en $C_1$-$C_4$, un hydroxyalkyle en $C_1$-$C_4$, un acyle en $C_1$-$C_4$, ou bien -$(X_1)_2$ constitue avec l'atome d'azote auquel il est lié, un hétérocycle choisi parmi pyrrolidine, pipéridine ou morpholine ; un groupe -S-$X_2$ dans lequel $X_2$ représente l'hydrogène ou un groupe alkyle en $C_1$-$C_4$ ;

ou bien X forme, avec l'atome de carbone auquel il est lié et avec l'atome de carbone voisin dans l'hétérocycle, une double liaison ;

- m est 2 ou 3 ;
- Ar' représente un phényle non substitué ou substitué une ou plusieurs fois par un des substituants choisis parmi : un atome d'halogène, un trifluorométhyle, un alcoxy en $C_1$-$C_4$, un alkyle en $C_1$-$C_4$, lesdits substituants étant identiques ou différents ; un thiényle ; un benzothiényle ; un naphtyle ou un indolyle ;
- n est 0, 1, 2 ou 3 ;
- p est 1 ou 2 et lorsque p est égal à 2, alors n est égal à 1 et Q représente deux atomes d'hydrogène ;
- Q représente l'oxygène ou deux atomes d'hydrogène ;
- T représente un groupe choisi parmi

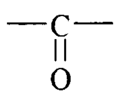 et 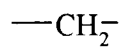

- q est 0,1, 2 ou 3 ;
- Z représente un phényle non substitué ou substitué par un ou plusieurs substituants choisis parmi halogène, CN, OH, $NH_2$, NH-CO-NH,, $NO_2$, $CONH_2$, $CF_3$, alkyle en $C_1$-$C_{10}$, alcényle contenant 2 à 10 atomes de carbone, alcynyle contenant 2 à 10 atomes de carbone, cycloalkyle contenant 3 à 8 atomes de carbone, bicycloalkyle contenant 4 à 11 atomes de carbone, hydroxyalkyle contenant 1 à 5 atomes de carbone, alcoxy contenant 1 à 10 atomes de carbone, alcoxyalkyle contenant 2 à 10 atomes de carbone, alcoxyalcoxyalkyle contenant 3 à 10 atomes de carbone, alcoxyalcoxy contenant 2 à 10 atomes de carbone, alcényloxy contenant 2 à 10 atomes de carbone, alcényloxyalkyle contenant 3 à 10 atomes de carbone, alcynyloxy contenant 2 à 10 atomes de carbone, alcynyloxyalkyle contenant 3 à 10 atomes de carbone, cycloalcoxy contenant 3 à 8 atomes de carbone, alkylthio contenant 1 à 10 atomes de carbone, alkylthioalkyle contenant 2 à 10 atomes de carbone, acylamino contenant 1 à 7 atomes de carbone, acylaminoalkyle contenant 2 à 8 atomes de carbone, acyloxy contenant 1 à 6 atomes de carbone, alcoxycarbonyle contenant 2 à 5 atomes de carbone, cycloalcoxycarbonyle contenant 4 à 8 atomes de carbone, alkylaminocarbonylamino contenant 2 à 4 atomes de carbone, dialkylaminocarbonylamino contenant 3 à 7 atomes de carbone, (1-pyrrolidino)carbonylamino, cycloalkylaminocarbonylamino contenant 4 à 8 atomes de carbone, alkylaminocarbonylaminoalkyle contenant 3 à 9 atomes de carbone, dialkylaminocarbonylaminoalkyle contenant 4 à 11 atomes de carbone, (1-pyrrolidino)carbonylaminoéthyl, (1-pipéridino)carbonylaminoéthyl, alcoxycarbonylaminoalkyle contenant 3 à 12 atomes de carbone, cycloalcoxycarbonylaminoalkyle contenant 5 à 12 atomes de carbone, carbamoylalkyle contenant 2 à 5 atomes de carbone, alkylaminocarbonylalkyle contenant 3 à 9 atomes de carbone, dialkylaminocarbonylalkyle

contenant 4 à 11 atomes de carbone, (1-pyrrolidino)carbonylméthyle, (1-pipéridino)carbonylméthyle, (1-pipéridino)carbonyléthyle, cycloalkylaminocarbonylalkyle contenant de 5 à 12 atomes de carbone, alkylaminocarbonylalcoxy contenant 3 à 10 atomes de carbone, dialkylaminocarbonylalcoxy contenant 4 à 10 atomes de carbone, (pipéridinyl-1)carbonylméthoxy, cycloalkylaminocarbonylalcoxy contenant 5 à 11 atomes de carbone, cycloalkylaminocarbonylaminoalkyle contenant 5 à 12 atomes de carbone;
un groupe naphtyle ou indényle, dont une ou plusieurs liaisons peuvent être hydrogénées, lesdits groupes pouvant être non substitués ou substitués une ou plusieurs fois par un halogène, un groupe alkyle, phényle, cyano, hydroxyalkyle, hydroxy, oxo, alkylcarbonylamino, alcoxycarbonyle, thioalkyle dans lesquels les alkyles sont en $C_1$-$C_4$ ;
un groupe pyridyle ; un thiadiazolyle, indolyle, indazolyle, imidazolyle, benzimidazolyle, quinolyle, benzotriazolyle, benzofurannyle, benzothiényle, benzothiazolyle, benzisothiazolyle, isoquinolyle, benzoxazolyle, benzoxazinyle, berzodioxinyle, isoxazolyle, benzopyranyle, thiazolyle, thiényle, furyle, pyrannyle, chroményle, isobenzofurannyle, pyrrolyle, pyrazolyle, pyrazinyle, pyrimidinyle, pyridazinyle, indolizinyle, phtalazinyle, quinazolinyle, acridinyle, isothiazolyle, isochromannyle, chromannyle, dont une ou plusieurs doubles liaisons peuvent être hydrogénées, lesdits groupes pouvant être non substitués ou substitués une ou plusieurs fois par un groupe alkyle, phényle, cyano, hydroxyalkyle, hydroxy, alkylcarbonylamino, alcoxycarbonyle, thioalkyle dans lesquels les alkyles sont en $C_1$-$C_4$; ou encore lorsque T est -C=O-, -$(CH_2)_q$-Z peut aussi représenter un groupe benzyle substitué sur le -CH- par un hydroxyle, un alcoxy en $C_1$-$C_4$, un alkyle en $C_1$-$C_4$ et éventuellement substitué sur le cycle aromatique par un halogène, un trifluorométhyle, un alkyle en $C_1$-$C_4$, un hydroxyle, un alcoxy en $C_1$-$C_4$ ;
ou un de ses sels éventuels avec des acides minéraux ou organiques, ou lorsque

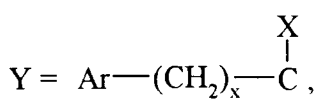

un de ses sels d'ammonium quaternaire avec l'azote (b) de la pipéridine ou un dérivé N-oxyde avec ce même atome d'azote.

2. Composé selon la revendication 1, sous forme optiquement pure, de formule :

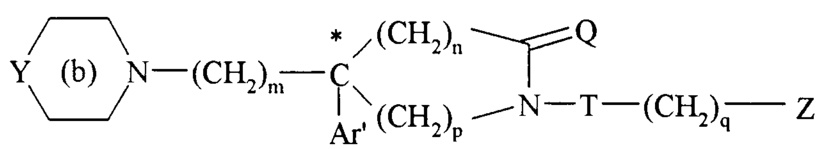

(I*)

dans laquelle

- "*" signifie que l'atome de carbone ainsi marqué à la configuration absolue (+) ou (-) déterminée,
- Y, m, Ar', n, p, Q, T, q et Z sont tels que définis dans la revendication 1, ou un de ses sels avec des acides minéraux ou organiques ou avec l'atome d'azote (b) un de ses sels d'ammonium quaternaires ou un dérivé N-oxyde.

3. Composé selon l'une des revendications 1 ou 2, dans laquelle Z représente un groupe phényle non substitué ou mono- ou di-substitué par un halogène ou un alcoxy; un groupe naphtyle.

4. Composé selon l'une des revendications 1 à 3 de formule (I) ou (I*)

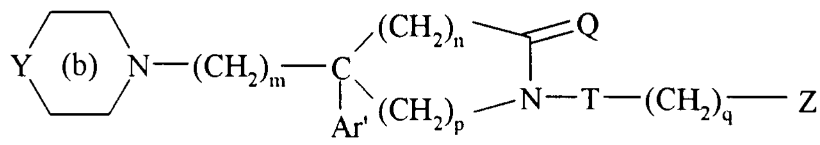

(I)

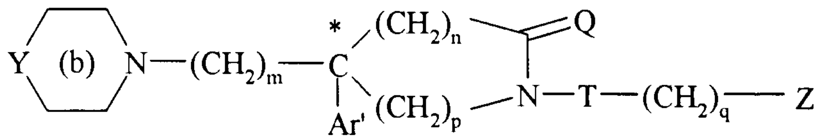

(I*)

dans lesquelles :
Y représente un groupe

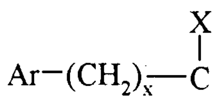

dans lequel Ar, x et X sont tels que définis dans la revendication 1 ;

- m , Ar', n, p, Q, T, q et Z sont tels que définis dans la revendications 1 ; sous forme de sel d'ammonium quaternaire ou de dérivé N-oxyde,

caractérisé en ce que le groupe

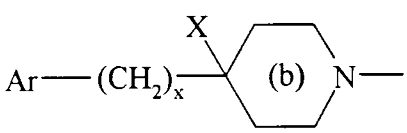

est alors représenté par le groupe

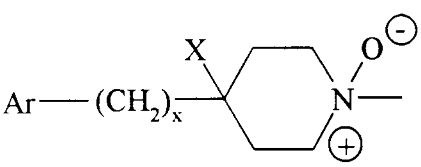

ou

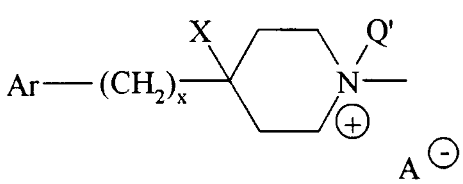

dans lequel :

. Q' représente un groupe alkyle en $C_1$-$C_6$ ou un groupe benzyle et
. $A^-$ représente un anion choisi parmi chlorure, bromure, iodure, acétate, méthanesulfonate ou paratoluènesulfonate.

**5.** Procédé pour la préparation des composés de formule (I) selon la revendication 1 caractérisé en ce que :

a) on traite un composé de formule :

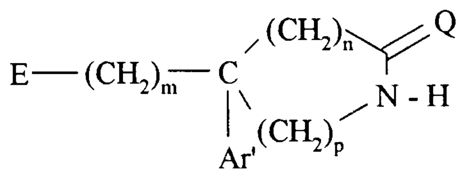

(II)

dans laquelle m, Ar', n, p et Q sont tels que définis dans la revendication 1 et E représente un hydroxyle ou éventuellement un groupe O-protégé ou un groupe :

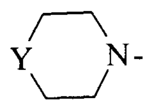

dans lequel Y est tel que défini dans la revendication 1 étant entendu que :
lorsque Y représente le groupe

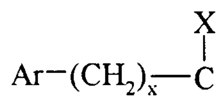

dans lequel X est un hydroxyle, cet hydroxyle peut être protégé,

. soit avec un dérivé fonctionnel d'un acide de formule :

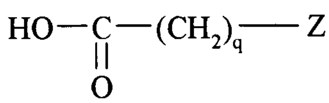

(III)

dans laquelle q et Z sont tels que définis dans la revendication 1, lorsqu'on doit préparer un composé de formule (I) où T est -CO-,

. soit avec un dérivé halogéné de formule :

$$Hal\text{-}(CH_2)_{q+1}\text{-}Z \qquad (IV)$$

dans laquelle q et Z sont tels que définis précédemment et où Hal représente un halogène lorsqu'on doit préparer un composé de formule (I) où T est $-CH_2-$,

pour obtenir le composé de formule :

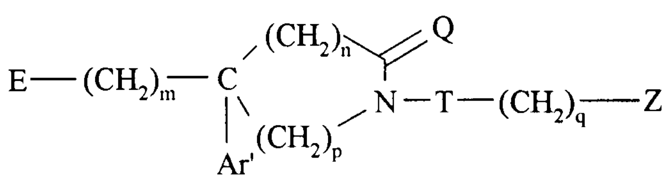

(V)

- b) puis, lorsque E représente un groupe O-protégé, on élimine le groupe O-protecteur par action d'un acide,
- c) on traite l'alcool ainsi obtenu de formule :

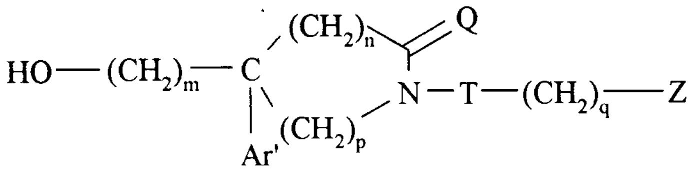

(VI)

avec le chlorure de méthanesulfonyle,

- d) on fait réagir le mésylate ainsi obtenu de formule :

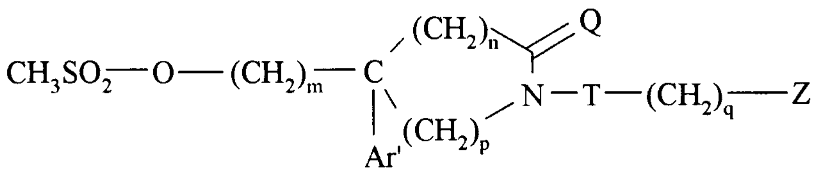

(VII)

avec une amine secondaire de formule :

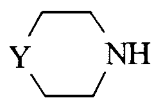

(VIII)

dans laquelle Y est tel que défini précédemment et,

- e) après déprotection éventuelle de l'hydroxyle représenté par X, on transforme éventuellement le produit ainsi obtenu en un de ses sels avec des acides minéraux ou organiques ou en un des ses sels d'ammonium quaternaire avec l'azote (b) de la pipéridine ou en dérivé N-oxyde avec ce même atome d'azote.

**6.** Composé de formule :

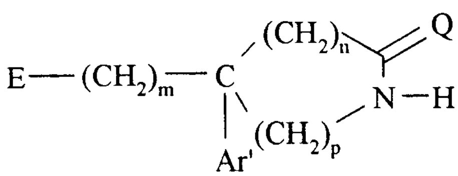

(II)

dans laquelle m, Ar', n, p et Q sont tels que définis dans la revendication 1 et E représente un hydroxyle ou éventuellement un groupe O-protégé ou un groupe :

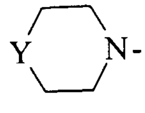

dans lequel Y est tel que défini dans la revendication 1,

. sous réserve que Q représente l'oxygène :

- lorsque E = OH ou ($C_1$-$C_6$)alkylcarbonyloxy, m = 2 ou 3, Ar' représente un phényle substitué par un ($C_1$-$C_4$)alcoxy, n = 3 et p = 1 ;

- lorsque E = $(C_1-C_4)$alkylcarbonyloxy, m = 2, n = 1 et Ar' représente un phényle non substitué ou substitué une ou plusieurs fois par un $(C_1-C_4)$alkyle, un $(C_1-C_4)$alcoxy ou un halogène;

. sous réserve que lorsque E = OH ou un $(C_1-C_4)$alcoxy, m = 2, p = 1, Ar' représente un phényle non substitué ou substitué une ou plusieurs fois par un $(C_1-C_4)$alkyle, un $(C_1-C_4)$alcoxy ou un halogène, n soit différent de 1,
. sous réserve que lorsque E = OH, Q = 2H, m = 2, n = 1, p = 7 Ar' soit différent d'un phényle non substitué.

ou un de ses sels avec des acides minéraux ou organiques.

7. Composé de formule :

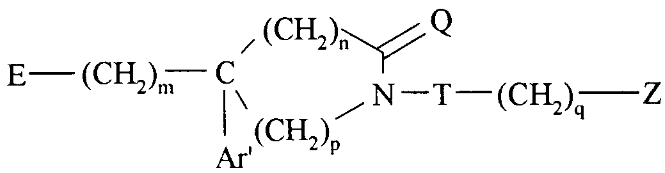

(V)

dans laquelle E représente un hydroxyle, un groupe O-protégé et m, Ar', n, p, Q, T et Z sont tels que définis dans la revendication 1

. sous réserve que lorsque E=OH, Q=2H, m =2, n=1, p=2, T=$-CH_2-$, q=0 et Z = phényle, Ar' soit différent d'un phényle non substitué, et
. sous réserve que lorsque E = OH, Q = 2H, m = 2, n = 1, p = 1, T = $-CH_2-$, q = 0 et Z = phényle, Ar' soit différent d'un phényle non substitué ou substitué une ou plusieurs fois par un $(C_1-C_4)$alkyle, un $(C_1-C_4)$alcoxy ou un halogène, et
. sous réserve que lorsque E = OH ou $(C_1-C_6)$akylcarbonyloxy Q = 2H, m = 2 ou 3, n = 3, p = 1, T = $-CH_2-$, q = 0, 1, 2 ou 3 et Z est un groupe aryle, Ar' soit différent d'un phényle substitué par un $(C_1-C_4)$alcoxy,

ou un de ses sels avec des acides minéraux ou organiques.

8. Composé selon la revendication 7, de formule :

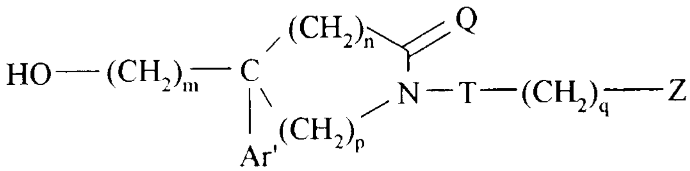

(VI)

dans laquelle m, Ar', Q, n, p, T, q et Z sont tels que définis dans la revendication 1

. sous réserve que lorsque O = 2H, m = 2, n = 1, p = 2, T = $-CH_2-$, q = 0 et Z = phényle, Ar' soit différent d'un phényle non substitué, et
. sous réserve que lorsque Q = 2H, m = 2, n = 1, p = 1, T = $-CH_2-$, q = 0 et Z = phényle, Ar' soit différent d'un phényle non substitué ou substitué une ou plusieurs fois par un $(C_1-C_4)$alkyle, un $(C_1-C_4)$alcoxy ou un halogène, et
. sous réserve que lorsque Q = 2H, m = 2 ou 3, n = 3, p = 1, T = $-CH_2-$, q = 0, 1, 2 ou 3 et Z est un groupe aryle, Ar' soit différent d'un phényle substitué par un $(C_1-C_4)$alcoxy,

ou un de ses sels avec des acides minéraux ou organiques.

9. Composé de formule :

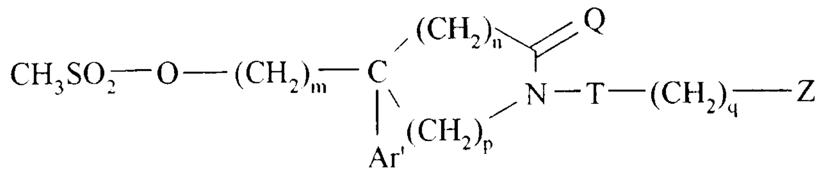

(VII)

dans laquelle m, Ar', n, p, Q, T, q et Z sont tels que définis dans la revendication 1 ou un ses sels avec des acides minéraux ou organiques.

**10.** Composé de formule :

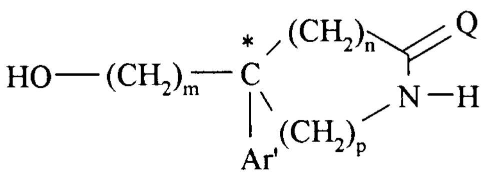

(II*)

dans lequel m, Ar', n et p sont tels que définis dans la revendication 1 et Q représente 2 atomes d'hydrogène, ledit composé étant sous forme optiquement pure, sous réserve que lorsque m = 2 ou 3, n = 3 et p = 1, Ar' soit différent d'un phényle substitué par un alcoxy en $C_1$-$C_4$.

**11.** Composé de formule :

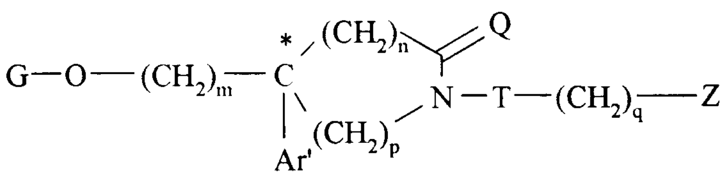

(V*)

dans lequel G représente l'hydrogène, ou un groupe méthanesulfonyle, m, Ar', n, p, q, T et Z étant tels que définis dans la revendication 1 et Q représente 2 atomes d'hydrogène, ledit composé étant sous forme optiquement pure, sous réserve que lorsque G représente l'hydrogène, m = 2 ou 3, n = 3, p = 1 et Z est un groupe aryle, Ar' soit différent d'un phényle substitué par un alcoxy en $C_1$-$C_4$.

**12.** Composition pharmaceutique contenant en tant que principe actif, un composé selon l'une quelconque des revendications 1 à 4, ou un de ses sels pharmaceutiquement acceptables.

**13.** Composition pharmaceutique selon la revendication 12, sous forme d'unité de dosage, dans laquelle le principe actif est mélangé à au moins un excipient pharmaceutique.

**14.** Composition selon la revendication 12 contenant de 2,5 à 1000 mg de principe actif.

**15.** Composés selon l'une quelconque des revendications 1 à 4, dans lesquels Ar' et/ou Z représente(nt) un groupe phényle substitué une ou plusieurs fois par un atome de chlore ou de fluor.

**16.** Procédé selon la revendication 5, caractérisé en ce que dans le composé de formule (II), E représente un groupe tétrahydropyran-2-yloxy.

**17.** Composé selon l'une des revendications 6 ou 7, dans laquelle E représente un groupe tétrahydropyran-2-yloxy.

**18.** Composé de formule (II) selon la revendication 6, choisi parmi :

- la 5-tétrahydropyranyloxypropyl-5-(3,4-dichlorophényl)pipéridone,
- la 3-tétrahydropyranyloxypropyl-3-(3,4-dichlorophényl)pipéridine, et
- la 3-(2-hydroxyéthyl)-3-(3,4-dichlorophényl)pipéridine.

**19.** Composé optiquement pur de formule (II*) selon la revendication 10, choisi parmi :

- la (+) 3-(2-hydroxyéthyl)-3-(3,4-dichlorophényl)pipéridine, et
- la (-) 3-(2-hydroxyéthyl)-3-(3,4-dichlorophényl)pipéridine.

**Revendications pour l'Etat contractant suivant : ES**

**1.** Procédé pour la préparation des composés de formule (I) :

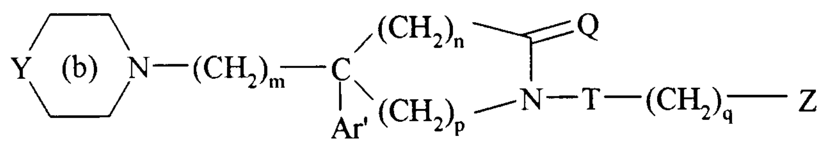

(I)

dans laquelle

- Y représente
  - soit un groupe Cy-N ou Cy-$CH_2$-N dans lequel
    . Cy représente un phényle non substitué ou substitué une ou plusieurs fois par un des substituants choisis parmi : un atome d'halogène, un hydroxyle, un alcoxy en $C_1$-$C_4$, un alkyle en $C_1$-$C_4$, un trifluorométhyle, lesdits substituants étant identiques ou différents ; un groupe cycloalkyle en $C_3$-$C_7$ ; un groupe pyrimidyle ou un groupe pyridyle ;
  - soit un groupe

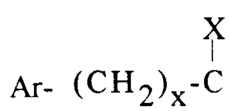

dans lequel

. Ar représente un phényle non substitué ou substitué une ou plusieurs fois par un des substituants choisis parmi : un atome d'halogène, un hydroxyle, un alcoxy en $C_1$-$C_4$, un trifluorométhyle, un alkyle en $C_1$-$C_4$, lesdits substituants étant identiques ou différents ; un groupe pyridyle ; un groupe thiényle ;
. x est zéro ou un ;
. X représente un hydrogène, un hydroxyle, un alcoxy en $C_1$-$C_4$ ; un acyloxy en $C_1$-$C_4$ ; un carboxy ; un carbalcoxy en $C_1$-$C_4$ ; un cyano ; un groupe -$N(X_1)_2$ dans lequel les groupes $X_1$ représentent indépendamment l'hydrogène, un alkyle en $C_1$-$C_4$, un hydroxyalkyle en $C_1$-$C_4$, un acyle en $C_1$-$C_4$, ou bien -$(X_1)_2$ constitue avec l'atome d'azote auquel il est lié, un hétérocycle choisi parmi pyrrolidine, pipéridine ou morpholine ; un groupe -S-$X_2$ dans lequel $X_2$ représente l'hydrogène ou un groupe alkyle en $C_1$-$C_4$ ;

ou bien X forme, avec l'atome de carbone auquel il est lié et avec l'atome de carbone voisin dans l'hétérocycle, une double liaison ;

- m est 2 ou 3 ;
- Ar' représente un phényle non substitué ou substitué une ou plusieurs fois par un des substituants choisis parmi : un atome d'halogène, un trifluorométhyle, un alcoxy en $C_1$-$C_4$, un alkyle en $C_1$-$C_4$, lesdits substituants étant identiques ou différents ; un thiényle ; un benzothiényle ; un naphtyle ou un indolyle ;

- n est 0,1, 2 ou 3 ;
- p est 1 ou 2 et lorsque p est égal à 2, alors n est égal à 1 et Q représente deux atomes d'hydrogène ;
- Q représente l'oxygène ou deux atomes d'hydrogène ;
- T représente un groupe choisi parmi

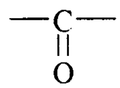 et $-CH_2-$

- q est 0,1, 2 ou 3 ;
- Z représente un phényle non substitué ou substitué par un ou plusieurs substituants choisis parmi halogène, CN, OH, $NH_2$, NH-CO-$NH_2$, $NO_2$, $CONH_2$, $CF_3$, alkyle en $C_1$-$C_{10}$, alcényle contenant 2 à 10 atomes de carbone, alcynyle contenant 2 à 10 atomes de carbone, cycloalkyle contenant 3 à 8 atomes de carbone, bicycloalkyle contenant 4 à 11 atomes de carbone, hydroxyalkyle contenant 1 à 5 atomes de carbone, alcoxy contenant 1 à 10 atomes de carbone, alcoxyalkyle contenant 2 à 10 atomes de carbone, alcoxyalcoxyalkyle contenant 3 à 10 atomes de carbone, alcoxyalcoxy contenant 2 à 10 atomes de carbone, alcényloxy contenant 2 à 10 atomes de carbone, alcényloxyalkyle contenant 3 à 10 atomes de carbone, alcynyloxy contenant 2 à 10 atomes de carbone, alcynyloxyalkyle contenant 3 à 10 atomes de carbone, cycloalcoxy contenant 3 à 8 atomes de carbone, alkylthio contenant 1 à 10 atomes de carbone, alkylthioalkyle contenant 2 à 10 atomes de carbone, acylamino contenant 1 à 7 atomes de carbone, acylaminoalkyle contenant 2 à 8 atomes de carbone, acyloxy contenant 1 à 6 atomes de carbone, alcoxycarbonyle contenant 2 à 5 atomes de carbone, cycloalcoxycarbonyle contenant 4 à 8 atomes de carbone, alkylaminocarbonylamino contenant 2 à 4 atomes de carbone, dialkylaminocarbonylamino contenant 3 à 7 atomes de carbone, (1-pyrrolidino)carbonylamino, cycloalkylaminocarbonylamino contenant 4 à 8 atomes de carbone, alkylaminocarbonylaminoalkyle contenant 3 à 9 atomes de carbone, dialkylaminocarbonylaminoalkyle contenant 4 à 11 atomes de carbone, (1-pyrrolidino)carbonylaminéthyl, (1-pipéridino)carbonaminoéthyl, alcoxycarbonylaminoalkyle contenant 3 à 12 atomes de carbone, cycloalcoxycarbonylaminoalkyle contenant 5 à 12 atomes de carbone, carbamoylalkyle contenant 2 à 5 atomes de carbone, alkylaminocarbonylalkyle contenant 3 à 9 atomes de carbone, dialkylaminocarbonylalkyle contenant 4 à 11 atomes de carbone, (1-pyrrolidino)carbonylméthyle, (1-pipéridino)carbonylméthyle, (1-pipéridino)carbonyléthyle, cycloalkylaminocarbonylalkyle contenant de 5 à 12 atomes de carbone, alkylaminocarbonylalcoxy contenant 3 à 10 atomes de carbone, dialkylaminocarbonylalcoxy contenant 4 à 10 atomes de carbone, (pipéridinyl-1)carbonylméthoxy, cycloalkylaminocarbonylalcoxy contenant 5 à 11 atomes de carbone, cycloalkylaminocarbonylaminoalkyle contenant 5 à 12 atomes de carbone ;
  un groupe naphtyle ou indényle, dont une ou plusieurs liaisons peuvent être hydrogénées, lesdits groupes pouvant être non substitués ou substitués une ou plusieurs fois par un halogène, un groupe alkyle, phényle, cyano, hydroxyalkyle, hydroxy, oxo, alkylcarbonylamino, alcoxycarbonyle, thioalkyle dans lesquels les alkyles sont en $C_1$-$C_4$ ;
  un groupe pyridyle ; un thiadiazolyle, indolyle, indazolyle, imidazolyle, benzimidazolyle, quinolyle, benzotriazolyle, benzofurannyle, benzothiényle, benzothiazolyle, benzisothiazolyle, isoquinolyle, benzoxazolyle, benzoxazinyle, benzodioxinyle, isoxazolyle, benzopyranyle, thiazolyle, thiényle, furyle, pyrannyle, chroményle, isobenzofurannyle, pyrrolyle, pyrazolyle, pyrazinyle, pyrimidinyle, pyridazinyle, indolizinyle, phtalazinyle, quinazolinyle, acridinyle, isothiazolyle, isochromannyle, chromannyle, dont une ou plusieurs doubles liaisons peuvent être hydrogénées, lesdits groupes pouvant être non substitués ou substitués une ou plusieurs fois par un groupe alkyle, phényle, cyano, hydroxyalkyle, hydroxy, alkylcarbonylamino, alcoxycarbonyle, thioalkyle dans lesquels les alkyles sont en $C_1$-$C_4$; ou encore lorsque T est -C=O-, $(CH_2)_q$-Z peut aussi représenter un groupe benzyle substitué sur le -CH- par un hydroxyle, un alcoxy en $C_1$-$C_4$, un alkyle en $C_1$-$C_4$ et éventuellement substitué sur le cycle aromatique par un halogène, un trifluorométhyle, un alkyle en $C_1$-$C_4$, un hydroxyle, un alcoxy en $C_1$-$C_4$ ;
  ou un de ses sels éventuels avec des acides minéraux ou organiques, ou lorsque

Y = 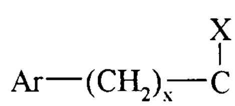 ,

de leurs sels d'ammonium quaternaire avec l'azote (b) de la pipéridine ou un dérivé N-oxyde avec ce même

atome d'azote,

caractérisé en ce que :

a) on traite un composé de formule :

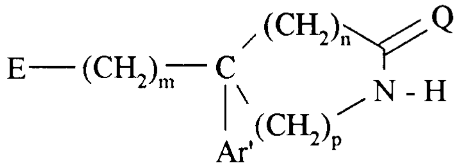

(II)

dans laquelle m, Ar', n, p et Q sont tels que définis précédemment et E représente un hydroxyle ou éventuellement un groupe O-protégé ou un groupe :

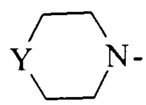

dans lequel Y est tel que défini précédemment étant entendu que : lorsque Y représente le groupe

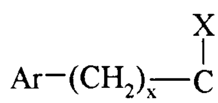

dans lequel X est un hydroxyle, cet hydroxyle peut être protégé,

. soit avec un dérivé fonctionnel

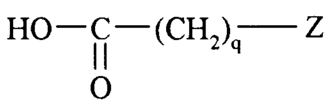

(III)

d'un acide de formule : dans laquelle q et Z sont tels que définis précédemment, lorsqu'on doit préparer un composé de formule (I) où T est -CO-,

. soit avec un dérivé halogéné de formule :

$$Hal\text{-}(CH_2)_{q+1}\text{-}Z \qquad (IV)$$

dans laquelle q et Z sont tels que définis précédemment et où Hal représente un halogène lorsqu'on doit préparer un composé de formule (I) où T est $-CH_2-$,

pour obtenir le composé de formule :

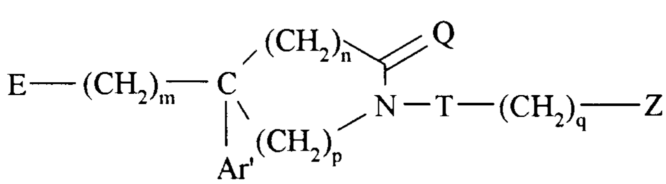

(V)

- b) puis, lorsque E représente un groupe O-protégé, on élimine le groupe O-protecteur par action d'un acide,
- c) on traite l'alcool ainsi obtenu de formule :

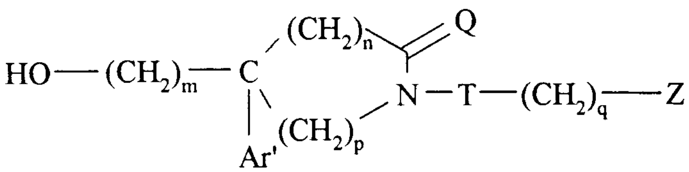

(VI)

avec le chlorure de méthanesulfonyle,
- d) on fait réagir le mésylate ainsi obtenu de formule :

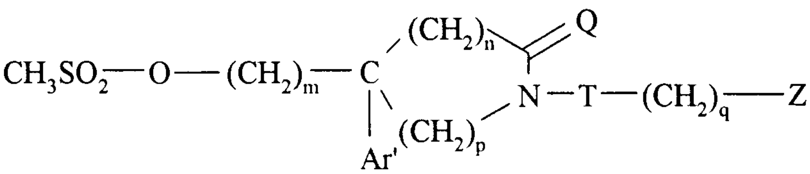

(VII)

avec une amine secondaire de formule :

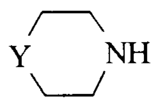

(VIII)

dans laquelle Y est tel que défini précédemment et,
- e) après déprotection éventuelle de l'hydroxyle représenté par X, on transforme éventuellement le produit ainsi obtenu en un de ses sels avec des acides minéraux ou organiques ou en un des ses sels d'ammonium quaternaire avec l'azote (b) de la pipéridine ou en dérivé N-oxyde avec ce même atome d'azote.

2. Procédé pour la préparation de composés optiquement purs de formule :

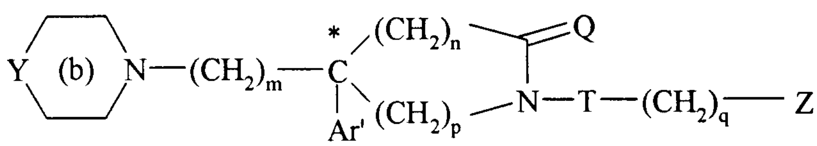

(I*)

dans laquelle

- "*" signifie que l'atome de carbone ainsi marqué à la configuration absolue (+) ou (-) déterminée,
- Y, m, Ar', n, p, Q, T, q et Z sont tels que définis dans la revendication 1, ou d'un de leurs sels avec des acides minéraux ou organiques ou avec l'atome d'azote (b) un de leurs sels d'ammonium quaternaires ou un dérivé N-oxyde,

caractérisé en ce que lesdits composés sont isolés par résolution, des mélanges racémiques des composés de formule (I) obtenus selon le procédé de la revendication 1, par cristallisation ou par chromatographie, et éventuellement transformés en l'un de leurs sels.

3. Procédé pour la préparation de composés optiquement purs de formule :

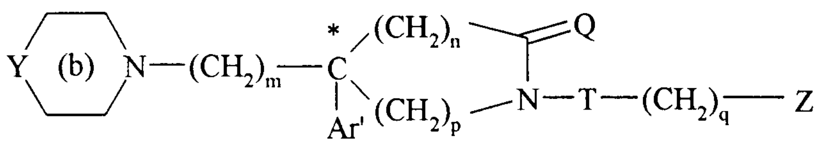

(I*)

dans laquelle

- "*" signifie que l'atome de carbone ainsi marqué à la configuration absolue (+) ou (-) déterminée,
- Y, m, Ar', n, p, T, q et Z sont tels que définis dans la revendication 1 et Q représente l'hydrogène,

caractérisé en ce que :

- a) on traite un composé de formule :

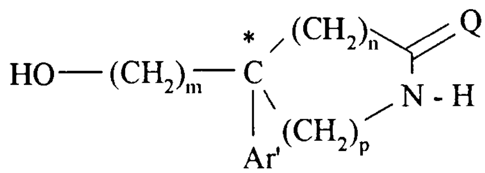

(II*, Q = H)

dans laquelle m, Ar', n, p et Q sont tels que définis ci-dessus,

. soit avec un dérivé fonctionnel d'un acide de formule :

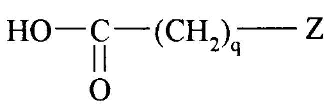

(III)

dans laquelle q et Z sont tels que définis précédemment, lorsqu'on doit préparer un composé de formule (I*) où T est -CO-,

. soit avec un dérivé halogéné de formule :

$$Hal\text{-}(CH_2)_{q+1}\text{-}Z \quad (IV)$$

dans laquelle q et Z sont tels que définis précédemment et où Hal représente un halogène et préférentiellement un atome de brome ou de chlore, lorsqu'on doit préparer un composé de formule (I*) où T est $-CH_2-$, pour obtenir le composé de formule :

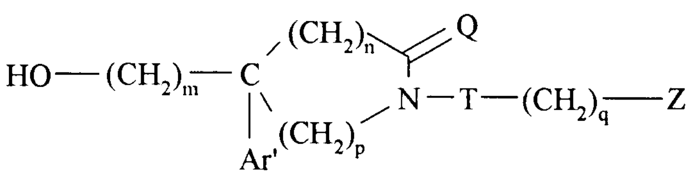

(VI)

- b) on traite l'alcool de formule (VI*) avec le chlorure de méthanesulfonyle,
- e) on fait réagir le mésylate ainsi obtenu de formule :

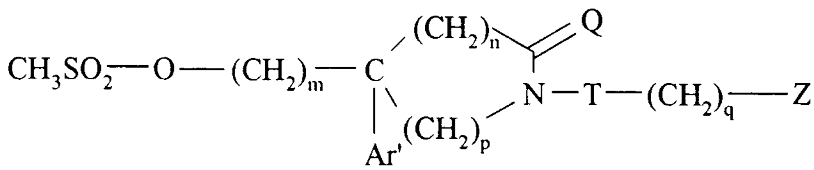

(VII)

avec une amine secondaire de formule :

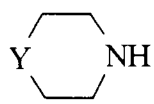

(VIII)

dans laquelle Y est tel que défini précédemment et,

- d) on transforme éventuellement le produit ainsi obtenu en un de ses sels avec des acides minéraux ou organiques ou en un des ses sels d'ammonium quaternaire avec l'azote (b) de la pipéridine ou en dérivé N-oxyde avec ce même atome d'azote.

**4.** Procédé selon l'une quelconque des revendications 1 à 3, caractérisé en ce que, pour transformer les composés (I) ou (I*) en un de leurs sels d'ammonium quaternaire, on fait réagir les bases libres des composés de formules (I) ou (I*) dans lesquelles

Y = 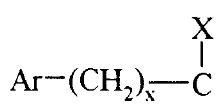 ,

pour lesquelles les fonctions aminées autres, éventuellement présentes sont N-protégées par un groupe N-protecteur habituel, avec un excès d'agent alkylant de formule :

A—Q'

dans laquelle A est un groupement partant et est tel que défini ci-après, et Q' est tel que défini pour (I) ou (I*), et on chauffe le mélange réactionnel dans un solvant par exemple choisi parmi le dichlorométhane, le chloroforme, l'acétone ou l'acétonitrile à une température comprise entre la température ambiante et le reflux pendant une à plusieurs heures pour obtenir après traitement selon les méthodes habituelles et après déprotection éventuelle, un mélange des diastéréoisomères axiaux et équatoriaux des sels d'ammonium quaternaires des composés de formules (I) ou (I*) dans lesquelles : le groupe

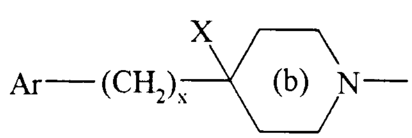

est alors représenté par
le groupe

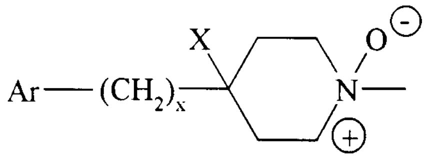 ou 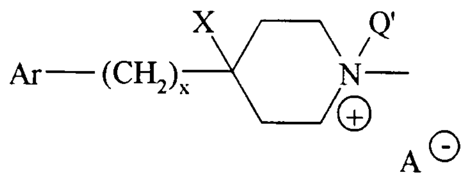

dans lequel :

- . Q' représente un groupe alkyle en $C_1$-$C_6$ ou un groupe benzyle et
- . A⁻ représente un anion choisi parmi chlorure, bromure, iodure, acétate, méthanesulfonate ou paratoluènesulfonate.

**5.** Procédé selon l'une quelconque des revendications 1 à 3, caractérisé en ce que, pour transformer les composés de formule (I) ou (I*) en dérivés N-oxydes, on fait réagir les composés de formules (I) ou (I*) dans lesquelles

Y = 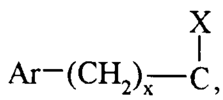,

avec un dérivé peroxydé, par exemple l'acide métachloroperbenzoïque ou l'eau oxygénée, selon les méthodes habituelles, pour obtenir des dérivés N-oxydes des composés (I) ou (I*) dans lesquels le groupe : le groupe

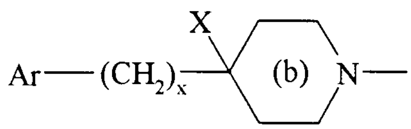

est alors représenté par
le groupe

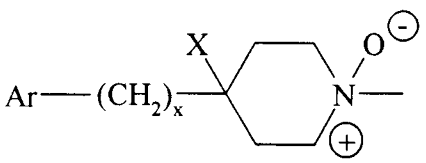 ou 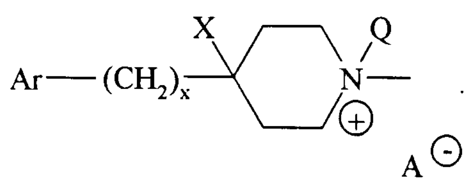 .

**6.** Procédé pour la préparation de composés de formule (V) :

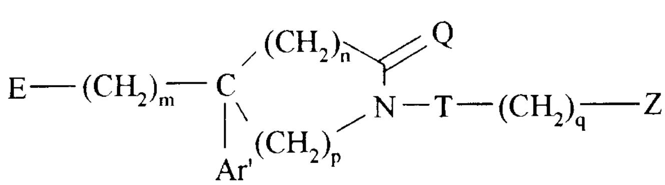

(V)

dans laquelle E représente un hydroxyle, un groupe O-protégé et m, Ar', n, p, Q, T et Z sont tels que définis dans la revendication 1,

. sous réserve que lorsque E = OH, Q = 2H, m = 2, n = 1, p = 2, T = $-CH_2-$, q = 0 et Z = phényle, Ar' soit différent d'un phényle non substitué, et

. sous réserve que lorsque E = OH, Q = 2H, m = 2, n = 1, p = 1, T = $-CH_2-$, q =0 et Z = phényle, Ar' soit différent d'un phényle non substitué ou substitué une ou plusieurs fois par un $(C_1-C_4)$alkyle, un $(C_1-C_4)$alcoxy ou un halogène, et

. sous réserve que lorsque E = OH ou $(C_1-C_6)$alkycarbonyloxy, Q = 2H, m = 2 ou 3, n = 3, p = 1, T = $-CH_2-$, q = 0, 1, 2 ou 3 et Z est un groupe aryle, Ar' soit différent d'un phényle substitué par un $(C_1-C_4)$alcoxy,

ou un de ses sels avec des acides minéraux ou organiques,
caractérisé en ce que :

- a) on traite un composé de formule :

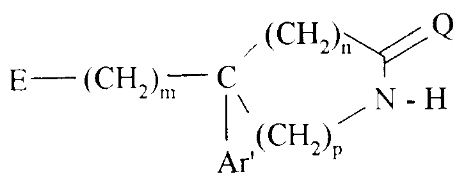

(II)

dans laquelle m, Ar', n, p et Q sont tels que définis précédemment et E représente un hydroxyle ou éventuellement un groupe O-protégé ou un groupe

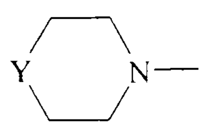

dans lequel Y est tel que défini précédemment étant entendu que :
lorsque Y représente le groupe

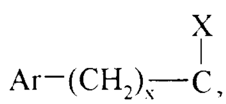

dans lequel X est un hydroxyle, cet hydroxyle peut être protégé,

. soit avec un dérivé fonctionnel d'un acide de formule :

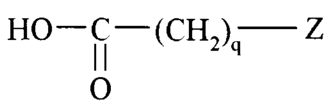

(III)

dans laquelle q et Z sont tels que définis précédemment, lorsqu'on doit préparer un composé de formule (V) où T est -CO-,

. soit avec un dérivé halogéné de formule :

$$Hal-(CH_2)_{q+1}-Z \qquad (IV)$$

dans laquelle q et Z sont tels que définis précédemment et où Hal représente un halogène lorsqu'on doit préparer un composé de formule (I) où T est $-CH_2-$,

pour obtenir le composé de formule (V) et,

- b) après déprotection éventuelle de l'hydroxyle représenté par X, on transforme éventuellement le produit ainsi

obtenu en un de ses sels avec des acides minéraux ou organiques.

**7.** Procédé pour la préparation de composés de formule (VI) :

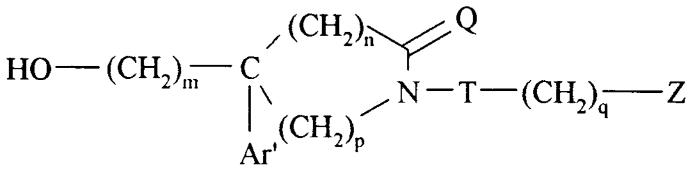

(VI)

dans laquelle m, Ar', Q, n, p, T, q et Z sont tels que définis dans la revendication 1,

. sous réserve que lorsque Q = 2H, m = 2, n = 1, p = 2, T = $-CH_2-$, q = 0 et Z = phényle, Ar' soit différent d'un phényle non substitué, et
. sous réserve que lorsque Q = 2H, m = 2, n = 1, p = 1, T = $-CH_2-$, q = 0 et Z = phényle, Ar' soit différent d'un phényle non substitué ou substitué une ou plusieurs fois par un $(C_1-C_4)$alkyle, un $(C_1-C_4)$alcoxy ou un halogène, et
. sous réserve que lorsque Q = 2H, m = 2 ou 3, n = 3, p = 1, T = $-CH_2-$, q = 0, 1, 2 ou 3 et Z est un groupe aryle, Ar' soit différent d'un phényle substitué par un $(C_1-C_4)$alcoxy.

ou un de ses sels avec des acides minéraux ou organiques,
caractérisé en ce que :

- a) on traite un composé de formule :

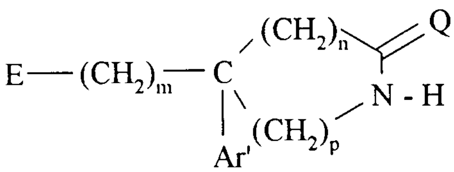

(II)

dans laquelle m, Ar', n, p et Q sont tels que définis précédemment et E représente un hydroxyle ou éventuellement un groupe O-protégé ou un groupe

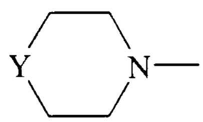

dans lequel Y est tel que défini précédemment étant entendu que :
lorsque Y représente le groupe

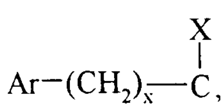

dans lequel X est un hydroxyle, cet hydroxyle peut être protégé,

. soit avec un dérivé fonctionnel d'un acide de formule :

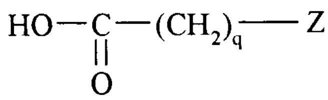 (III)

dans laquelle q et Z sont tels que définis précédemment, lorsqu'on doit préparer un composé de formule (VI) où T est -CO-,

. soit avec un dérivé halogéné de formule :

$$Hal\text{-}(CH_2)_{q+1}\text{-}Z \qquad (IV)$$

dans laquelle q et Z sont tels que définis précédemment et où Hal représente un halogène lorsqu'on doit préparer un composé de formule (VI) où T est $-CH_2-$,

pour obtenir le composé de formule :

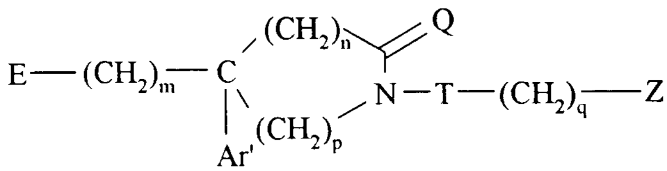

(V)

- b) puis, lorsque E représente un groupe O-protégé, on élimine le groupe O-protecteur par action d'un acide, et
- c) après déprotection éventuelle de l'hydroxyle représenté par X, on transforme éventuellement le produit ainsi obtenu en un de ses sels avec des acides minéraux ou organiques.

**8.** Procédé pour la préparation de composés de formule (VII) :

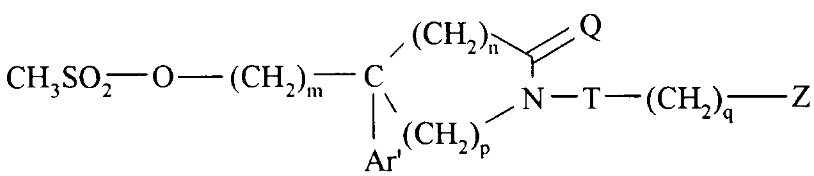

(VII)

dans laquelle m, Ar', n, p, Q, T, q et Z sont tels que définis dans la revendication 1 ou un ses sels avec des acides minéraux ou organiques,
caractérisé en ce que :

- a) on traite un composé de formule :

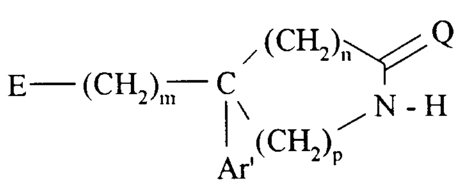

(II)

dans laquelle m, Ar', n, p et Q sont tels que définis précédemment et E représente un hydroxyle ou éventuellement un groupe O-protégé ou un groupe

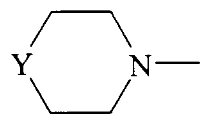

dans lequel Y est tel que défini précédemment étant entendu que :
lorsque Y représente le groupe

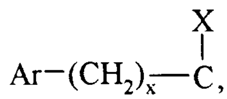

dans lequel X est un hydroxyle, cet hydroxyle peut être protégé,

. soit avec un dérivé fonctionnel d'un acide de formule :

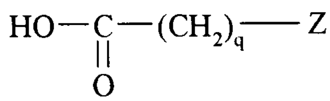 (III)

dans laquelle q et Z sont tels que définis précédemment, lorsqu'on doit préparer un composé de formule (VII) où T est -CO-,

. soit avec un dérivé halogéné de formule :

$$Hal\text{-}(CH_2)_{q+1}\text{-}Z \qquad (IV)$$

dans laquelle q et Z sont tels que définis précédemment et où Hal représente un halogène lorsqu'on doit préparer un composé de formule (VII) où T est $-CH_2-$, pour obtenir le composé de formule :

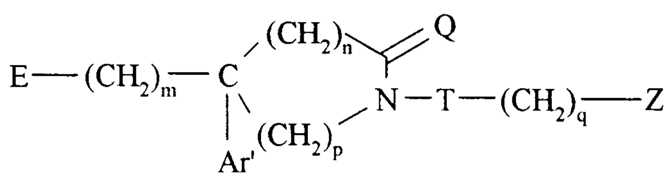

(V)

- b) puis, lorsque E représente un groupe O-protégé, on élimine le groupe O-protecteur par action d'un acide, et
- c) on traite l'alcool ainsi obtenu de formule :

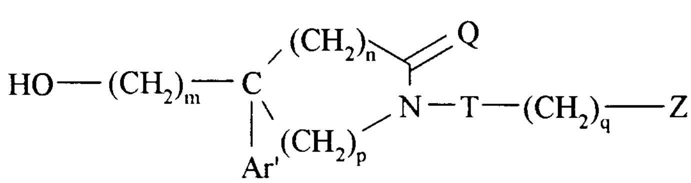

(VI)

avec le chlorure de méthane sulfonyle, et

- d) après déprotection éventuelle de l'hydroxyle représenté par X, on transforme éventuellement le produit ainsi obtenu en un de ses sels avec des acides minéraux ou organiques.

**9.** Procédé pour la préparation de composés de formule (II*) :

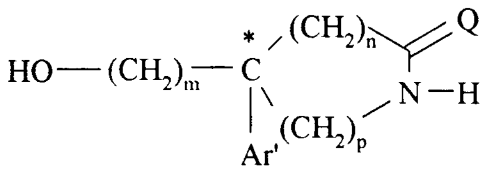

(II*)

dans laquelle

- "*" signifie que l'atome de carbone ainsi marqué à la configuration absolue (+) ou (-) déterminée,
- m, Ar', n et p sont tels que définis dans la revendication 1,
- Q représente l'hydrogène ou d'un de leurs sels avec des acides minéraux ou organiques,

caractérisé en ce que lesdits composés sont isolés par résolution des mélanges racémiques des composés de formule (II), par cristallisation ou par chromatographie, et éventuellement transformés en l'un de leurs sels.

**10.** Procédé pour la préparation de composés de formule :

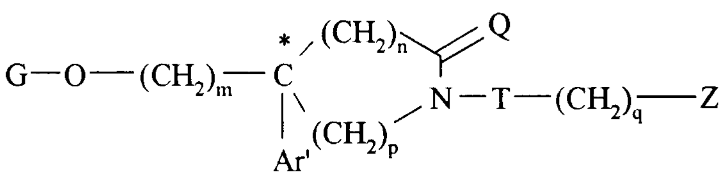

(V*, Q = H)

dans laquelle

- "*" signifie que l'atome de carbone ainsi marqué à la configuration absolue (+) ou (-) déterminée,
- m, Ar', n et p sont tels que définis dans la revendication 1,
- Q représente l'hydrogène,
- G représente l'hydrogène, ou un groupe méthanesulfonyle,

caractérisé en ce que :

a) on traite un composé de formule :

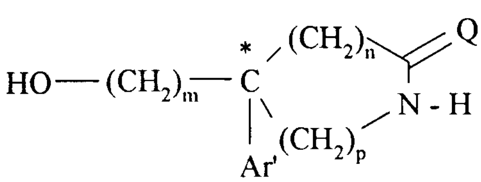

(II*, Q = H)

dans laquelle m, Ar', n, p et Q sont tels que définis ci-dessus,

. soit avec un dérivé fonctionnel d'un acide de formule :

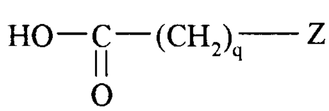

(III)

dans laquelle q et Z sont tels que définis précédemment, lorsqu'on doit préparer un composé de formule

(V*) où T est -CO-,

. soit avec un dérivé halogéné de formule :

$$Hal\text{-}(CH_2)_{q+1}\text{-}Z \qquad (IV)$$

dans laquelle q et Z sont tels que définis précédemment et où Hal représente un halogène lorsqu'on doit préparer un composé de formule (V*) où T est $-CH_2-$,

pour obtenir le composé de formule (V*) dans laquelle G représente l'hydrogène que l'on traite éventuellement avec le chlorure de méthanesulfonyle pour obtenir le composé de formule (V*) dans laquelle G est un groupe méthanesulfonyle, et

- b) on transforme le produit ainsi obtenu en un de ses sels avec des acides minéraux ou organiques.

**11.** Procédé selon l'une quelconque des revendications 1, 6, 7 ou 8, caractérisé en ce que dans le composé de formule (II), E représente un groupe tétrahydropyran-2-yloxy.

**12.** Procédé selon la revendication 9, pour la préparation d'un composé de formule (II*) dans laquelle :

- m = 2
- Ar' = 3,4-dichlorophényl
- n = 2
- p = 1

sous forme optiquement pure (+) ou (-),
caractérisé en ce que lesdits composés sont isolés par résolution des mélanges racémiques des composés de formule (II), par cristallisation ou par chromatographie, et éventuellement transformés en l'un de leurs sels.

**13.** Procédé pour la préparation d'une composition pharmaceutique, caractérisé en ce que l'on mélange, en tant que principe actif, un composé de formule (I) ou (I*) préparé par le procédé selon l'une quelconque des revendications 1 à 5, avec un véhicule pharmaceutiquement acceptable.

**Revendications pour l'Etat contractant suivant : GR**

**1.** Composé de formule :

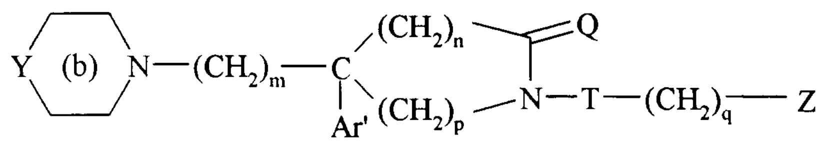

(I)

dans laquelle

- Y représente

  - soit un groupe Cy-N ou $Cy-CH_2-N$ dans lequel

    . Cy représente un phényle non substitué ou substitué une ou plusieurs fois par un des substituants choisis parmi : un atome d'halogène, un hydroxyle, un alcoxy en $C_1$-$C_4$, un alkyle en $C_1$-$C_4$, un trifluorométhyle, lesdits substituants étant identiques ou différents ; un groupe cycloalkyle en $C_3$-$C_7$ ; un groupe pyrimidyle ou un groupe pyridyle ;

  - soit un groupe

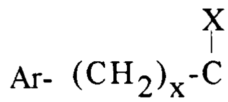

dans lequel

. Ar représente un phényle non substitué ou substitué une ou plusieurs fois par un des substituants choisis parmi : un atome d'halogène, un hydroxyle, un alcoxy en $C_1$-$C_4$, un trifluorométhyle, un alkyle en $C_1$-$C_4$, lesdits substituants étant identiques ou différents ; un groupe pyridyle ; un groupe thiényle ;

. x est zéro ou un ;

. X représente un hydrogène, un hydroxyle, un alcoxy en $C_1$-$C_4$ ; un acyloxy en $C_1$-$C_4$ ; un carboxy ; un carbalcoxy en $C_1$-$C_4$ ; un cyano; un groupe $-N(X_1)_2$ dans lequel les groupes $X_1$ représentent indépendamment l'hydrogène, un alkyle en $C_1$-$C_4$, un hydroxyalkyle en $C_1$-$C_4$, un acyle en $C_1$-$C_4$, ou bien $-(X_1)_2$ constitue avec l'atome d'azote auquel il est lié, un hétérocycle choisi parmi pyrrolidine, pipéridine ou morpholine; un groupe $-S-X_2$ dans lequel $X_2$ représente l'hydrogène ou un groupe alkyle en $C_1$-$C_4$ ;

ou bien X forme, avec l'atome de carbone auquel il est lié et avec l'atome de carbone voisin dans l'hétérocycle, une double liaison ;

- m est 2 ou 3 ;
- Ar' représente un phényle non substitué ou substitué une ou plusieurs fois par un des substituants choisis parmi : un atome d'halogène, un trifluorométhyle, un alcoxy en $C_1$-$C_4$, un alkyle en $C_1$-$C_4$, lesdits substituants étant identiques ou différents ; un thiényle ; un benzothiényle ; un naphtyle ou un indolyle ;
- n est 0,1, 2 ou 3 ;
- p est 1 ou 2 et lorsque p est égal à 2, alors n est égal à 1 et Q représente deux atomes d'hydrogène ;
- Q représente l'oxygène ou deux atomes d'hydrogène ;
- T représente un groupe choisi parmi

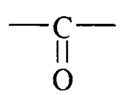 et $—CH_2—$

- q est 0, 1, 2 ou 3 ;
- Z représente un phényle non substitué ou substitué par un ou plusieurs substituants choisis parmi halogène, CN, OH, $NH_2$, NH-CO-$NH_2$, $NO_2$, $CONH_2$, $CF_3$, alkyle en $C_1$-$C_{10}$, alcényle contenant 2 à 10 atomes de carbone, alcynyle contenant 2 à 10 atomes de carbone, cycloalkyle contenant 3 à 8 atomes de carbone, bicycloalkyle contenant 4 à 11 atomes de carbone, hydroxyalkyle contenant 1 à 5 atomes de carbone, alcoxy contenant 1 à 10 atomes de carbone, alcoxyalkyle contenant 2 à 10 atomes de carbone, alcoxyalcoxyalkyle contenant 3 à 10 atomes de carbone, alcoxyalcoxy contenant 2 à 10 atomes de carbone, alcényloxy contenant 2 à 10 atomes de carbone, alcényloxyalkyle contenant 3 à 10 atomes de carbone, alcynyloxy contenant 2 à 10 atomes de carbone, alcynyloxyalkyle contenant 3 à 10 atomes de carbone, cycloalcoxy contenant 3 à 8 atomes de carbone, alkylthio contenant 1 à 10 atomes de carbone, alkylthioalkyle contenant 2 à 10 atomes de carbone, acylamino contenant 1 à 7 atomes de carbone, acylaminoalkyle contenant 2 à 8 atomes de carbone, acyloxy contenant 1 à 6 atomes de carbone, alcoxycarbonyle contenant 2 à 5 atomes de carbone, cycloalcoxycarbonyle contenant 4 à 8 atomes de carbone, alkylaminocarbonylamino contenant 2 à 4 atomes de carbone, dialkylaminocarbonylamino contenant 3 à 7 atomes de carbone, (1-pyrrolidino)carbonylamino, cycloalkylaminocarbonylamino contenant 4 à 8 atomes de carbone, alkylaminocarbonylaminoalkyle contenant 3 à 9 atomes de carbone, dialkylaminocarbonylaminoalkyle contenant 4 à 11 atomes de carbone, (1-pyrrolidino)carbonylaminéthyl, (1-pipéridino)carbonaminoéthyl, alcoxycarbonylaminoalkyle contenant 3 à 12 atomes de carbone, cycloalcoxycarbonylaminoalkyle contenant 5 à 12 atomes de carbone, carbamoylalkyle contenant 2 à 5 atomes de carbone, alkylaminocarbonylalkyle contenant 3 à 9 atomes de carbone, dialkylaminocarbonylalkyle contenant 4 à 11 atomes de carbone, (1-pyrrolidino)carbonylméthyle, (1-pipéridino)carbonylméthyle, (1-pipéridino)carbonyléthyle, cycloalkylaminocarbonylalkyle contenant de 5 à 12 atomes de carbone, alkylaminocarbonylalcoxy contenant 3 à 10 atomes de carbone, dialkylaminocarbonylalcoxy contenant 4 à 10 atomes de carbone, (pipéridinyl-1)carbonylméthoxy, cycloalkylaminocarbonylalcoxy contenant 5 à 11 atomes de carbone,

cycloalkylaminocarbonylaminoalkyle contenant 5 à 12 atomes de carbone ;
un groupe naphtyle ou indényle, dont une ou plusieurs liaisons peuvent être hydrogénées, lesdits groupes pouvant être non substitués ou substitués une ou plusieurs fois par un halogène, un groupe alkyle, phényle, cyano, hydroxyalkyle, hydroxy, oxo, alkylcarbonylamino, alcoxycarbonyle, thioalkyle dans lesquels les alkyles sont en $C_1$-$C_4$ ;
un groupe pyridyle ; un thiadiazolyle, indolyle, indazolyle, imidazolyle, benzimidazoly'le, quinolyle, benzotriazolyle, benzofurannyle, benzothiényle, benzothiazolyle, benzisothiazolyle, isoquinolyle, benzoxazolyle, benzoxazinyle, benzodioxinyle, isoxazolyle, benzopyranyle, thiazolyle, thiényle, furyle, pyrannyle, chroményle, isobenzofurannyle, pyrrolyle, pyrazolyle, pyrazinyle, pyrimidinyle, pyridazinyle, indolizinyle, phtalazinyle, quinazolinyle, acridinyle, isothiazolyle, isochromannyle, chromannyle, dont une ou plusieurs doubles liaisons peuvent être hydrogénées, lesdits groupes pouvant être non substitués ou substitués une ou plusieurs fois par un groupe alkyle, phényle, cyano, hydroxyalkyle, hydroxy, alkylcarbonylamino, alcoxycarbonyle, thioalkyle dans lesquels les alky'les sont en $C_1$-$C_4$; ou encore lorsque T est -C=O-, $-(CH_2)_q$-Z peut aussi représenter un groupe benzyle substitué sur le -CH- par un hydroxyle, un alcoxy en $C_1$-$C_4$, un alkyle en $C_1$-$C_4$ et éventuellement substitué sur le cycle aromatique par un halogène, un trifluorométhyle, un alkyle en $C_1$-$C_4$, un hydroxyle, un alcoxy en $C_1$-$C_4$ ;
ou un de ses sels éventuels avec des acides minéraux ou organiques, ou lorsque

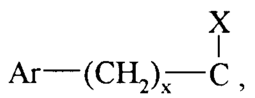

un de ses sels d'ammonium quaternaire avec l'azote (b) de la pipéridine ou un dérivé N-oxyde avec ce même atome d'azote.

**2.** Composé selon la revendication 1, sous forme optiquement pure, de formule ;

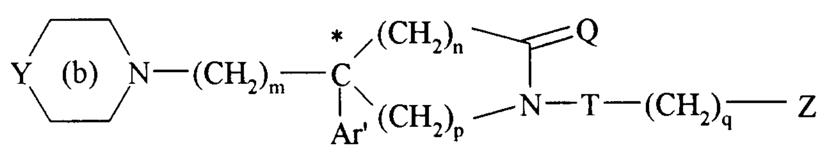

(I*)

dans laquelle

- "*" signifie que l'atome de carbone ainsi marqué à la configuration absolue (+) ou (-) déterminée,
- Y, m, Ar', n, p, Q, T, q et Z sont tels que définis dans la revendication 1, ou un de ses sels avec des acides minéraux ou organiques ou avec l'atome d'azote (b) un de ses sels d'ammonium quaternaires ou un dérivé N-oxyde.

**3.** Composé selon l'une des revendications 1 ou 2, dans laquelle Z représente un groupe phényle non substitué ou mono- ou di-substitué par un halogène ou un alcoxy ; un groupe naphtyle.

**4.** Composé selon l'une des revendications 1 à 3 de formule (I) ou (I*)

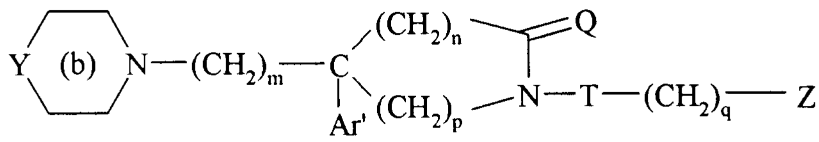

(I)

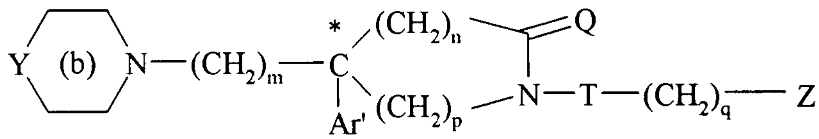

(I*)

dans lesquelles :
Y représente un groupe

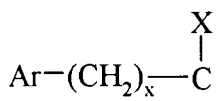

dans lequel Ar, x et X sont tels que définis dans la revendication 1 ;

- m , Ar', n, p, Q, T, q et Z sont tels que définis dans la revendications 1; sous forme de sel d'ammonium quaternaire ou de dérivé N-oxyde,
  caractérisé en ce que

le groupe

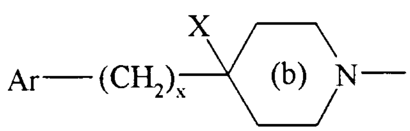

est alors représenté par
le groupe

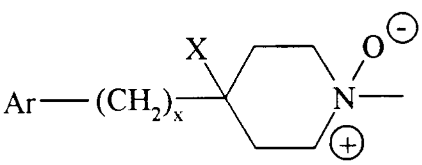

ou

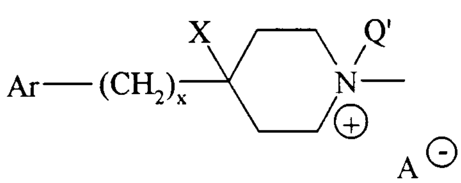

dans lequel :

. Q' représente un groupe alkyle en $C_1$-$C_6$ ou un groupe benzyle et
. $A^-$ représente un anion choisi parmi chlorure, bromure, iodure, acétate, méthanesulfonate ou paratoluènesulfonate.

**5.** Procédé pour la préparation des composés de formule (I) selon la revendication 1 caractérisé en ce que :

a) on traite un composé de formule :

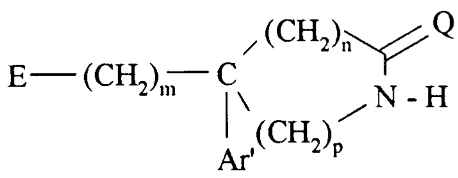

(II)

dans laquelle m, Ar', n, p et Q sont tels que définis dans la revendication 1 et E représente un hydroxyle ou éventuellement un groupe O-protégé ou un groupe :

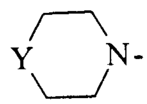

dans lequel Y est tel que défini dans la revendication 1 étant entendu que :
lorsque Y représente le groupe

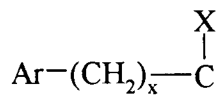

dans lequel X est un hydroxyle, cet hydroxyle peut être protégé,

. soit avec un dérivé fonctionnel d'un acide de formule :

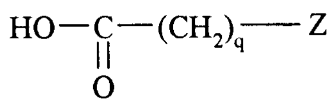

(III)

dans laquelle q et Z sont tels que définis dans la revendication 1, lorsqu'on doit préparer un composé de formule (I) où T est -CO-,

. soit avec un dérivé halogéné de formule :

$$Hal\text{-}(CH_2)_{q+1}\text{-}Z \qquad (IV)$$

dans laquelle q et Z sont tels que définis précédemment et où Hal représente un halogène lorsqu'on doit préparer un composé de formule (I) où T est $-CH_2-$,

pour obtenir le composé de formule :

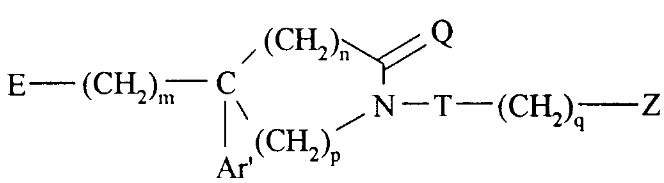

(V)

- b) puis, lorsque E représente un groupe O-protégé, on élimine le groupe O-protecteur par action d'un acide,
- c) on traite l'alcool ainsi obtenu de formule :

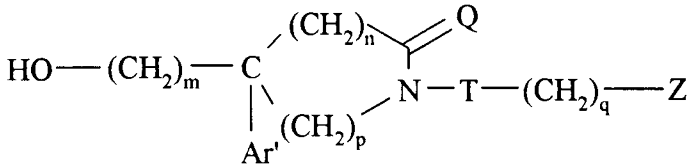

(VI)

avec le chlorure de méthanesulfonyle,

- d) on fait réagir le mésylate ainsi obtenu de formule :

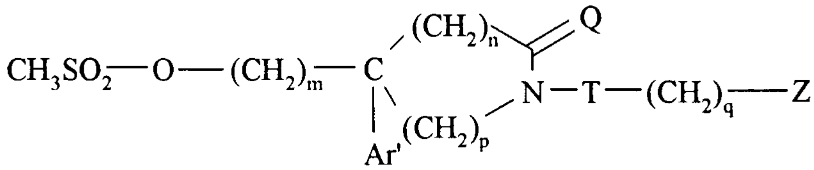

(VII)

avec une amine secondaire de formule :

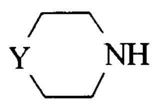

(VIII)

dans laquelle Y est tel que défini précédemment et,

- e) après déprotection éventuelle de l'hydroxyle représenté par X, on transforme éventuellement le produit ainsi obtenu en un de ses sels avec des acides minéraux ou organiques ou en un des ses sels d'ammonium quaternaire avec l'azote (b) de la pipéridine ou en dérivé N-oxyde avec ce même atome d'azote.

**6.** Composé de formule :

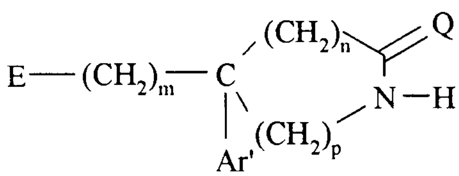

(II)

dans laquelle m, Ar', n, p et Q sont tels que définis dans la revendication 1 et E représente un hydroxyle ou éventuellement un groupe O-protégé ou un groupe :

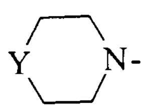

dans lequel Y est tel que défini dans la revendication 1,

. sous réserve que Q représente l'oxygène :

- lorsque E = OH ou ($C_1$-$C_6$)alkylcarbonyloxy, m = 2 ou 3, Ar' représente un phényle substitué par un ($C_1$-$C_4$)alcoxy, n = 3 et p = 1 ;

- lorsque E = $(C_1-C_4)$alkylcarbonyloxy, m = 2, n = 1 et Ar' représente un phényle non substitué ou substitué une ou plusieurs fois par un $(C_1-C_4)$alkyle, un $(C_1-C_4)$alcoxy ou un halogène.

. sous réserve que lorsque E = OH ou un $(C_1-C_4)$alcoxy, m = 2, p = 1, Ar' représente un phényle non substitué ou substitué une ou plusieurs fois par un $(C_1-C_4)$alkyle, un $(C_1-C_4)$alcoxy ou un halogène, n soit différent de 1.
. sous réserve que lorsque E = OH, Q = 2H, m = 2, n = 1, p = 2, Ar' soit différent d'un phényle non substitué. ou un de ses sels avec des acides minéraux ou organiques.

**7.** Composé de formule :

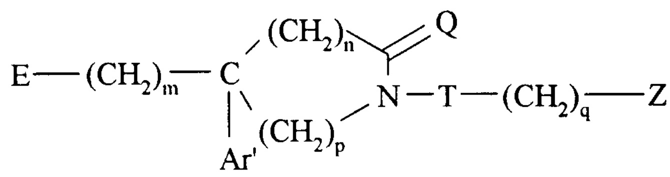

(V)

dans laquelle E représente un hydroxyle un groupe O-protégé et m, Ar', n, p, Q, T et Z sont tels que définis dans la revendication 1

. sous réserve que lorsque E = OH, Q = 2H, m = 2, n = 1, p = 2, T = $-CH_2-$, q = 0 et Z = phényle, Ar' soit différent d'un phényle non substitué, et
. sous réserve que lorsque E = OH, Q = 2H, m = 2, n = 1, p = 1, T = $-CH_2-$, q = 0 et Z = phényle, Ar' soit différent d'un phényle non substitué ou substitué une ou plusieurs fois par un $(C_1-C_4)$alkyle, un $(C_1-C_4)$alcoxy ou un halogène, et
. sous réserve que lorsque E = OH ou $(C_1-C_6)$alkylcarbonyloxy, Q = 2H, m = 2 ou 3, n = 3, p = 1, T = $-CH_2-$, q = 0, 1, 2 ou 3 et Z est un groupe aryle, Ar' soit différent d'un phényle substitué par un $(C_1-C_4)$alcoxy,

ou un de ses sels avec des acides minéraux ou organiques.

**8.** Composé selon la revendication 7, de formule :

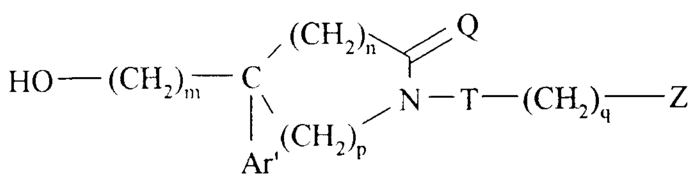

(VI)

dans laquelle m, Ar', Q, n, p, T, q et Z sont tels que définis dans la revendication 1

. sous réserve que lorsque Q = 2H, m = 2, n = 1, p = 2, T = $-CH_2-$, q = 0 et Z = phényle, Ar' soit différent d'un phényle non substitué, et
. sous réserve que lorsque Q = 2H, m = 2, n = 1, p = 1, T = $-CH_2-$, q = 0 et Z = phényle, Ar' soit différent d'un phényle non substitué ou substitué une ou plusieurs fois par un $(C_1-C_4)$alkyle, un $(C_1-C_4)$alcoxy ou un halogène, et
. sous réserve que lorsque Q = 2H m = 2 ou 3, n = 3, p = 1, T = $-CH_2-$, q = 0, 1, 2 ou 3 et Z est un groupe aryle. Ar' soit différent d'un phényle substitué par un $(C_1-C_4)$alcoxy,

ou un de ses sels avec des acides minéraux ou organiques.

**9.** Composé de formule :

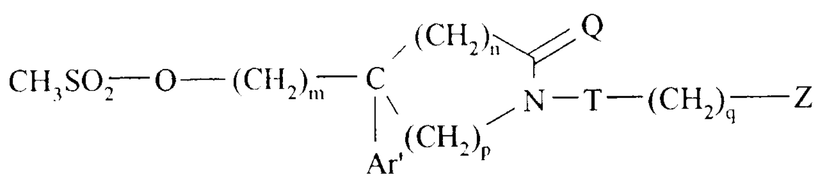

(VII)

dans laquelle m, Ar', n, p, Q, T, q et Z sont tels que définis dans la revendication 1 ou un ses sels avec des acides minéraux ou organiques.

**10.** Composé de formule :

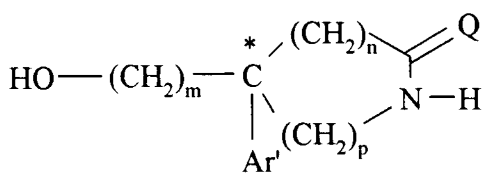

(II*)

dans lequel m, Ar', n et p sont tels que définis dans la revendication 1 et Q représente 2 atomes d'hydrogène, ledit composé étant sous forme optiquement pure, sous réserve que lorsque m = 2 ou 3, n = 3 et p = 1, Ar' soit différent d'un phényle substitué par un alcoxy en $C_1$-$C_4$.

**11.** Composé de formule :

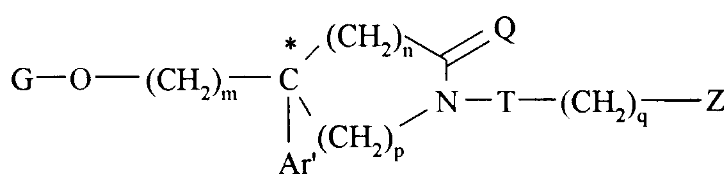

(V*)

dans lequel G représente l'hydrogène, ou un groupe méthanesulfonyle, m, Ar', n, p, q, T et Z étant tels que définis dans la revendication 1 et a représente 2 atomes d'hydrogène, ledit composé étant sous forme optiquement pure, sous réserve que lorsque & représente l'hydrogène, m = 2 ou 3, n = 3, p = 1 et Z est un groupe aryle, Ar' soit différent d'un phényle substitué par un alcoxy en $C_1$-$C_4$.

**12.** Procédé pour la préparation d'une composition pharmaceutique caractérisé en ce que l'on mélange en tant que principe actif, un composé selon l'une quelconque des revendications 1 à 4 avec un véhicule pharmaceutiquement acceptable.

**13.** Composés selon l'une quelconque des revendications 1 à 4, dans lesquels Ar' et/ou Z représente(nt) un groupe phény'le substitué une ou plusieurs fois par un atome de chlore ou de fluor.

**14.** Procédé selon la revendication 5, caractérisé en ce que dans le composé de formule (II), E représente un groupe tétrahydropyran-2-yloxy.

**15.** Composé selon l'une des revendications 6 ou 7, dans laquelle E représente un groupe tétrahydropyran-2-yloxy.

**16.** Composé de formule (II) selon la revendication 6, choisi parmi :

- la 5-tétrahydropyranyloxypropyl-5-(3,4-dichlorophényl)pipéridone,
- la 3-tétrahydropyranyloxypropyl-3-(3,4-dichlorophényl)pipéridine, et

- la 3-(2-hydroxéthyl)-3-(3,4-dichlorophényl)pipéridine.

**17.** Composé optiquement pur de formule (II*) selon la revendication 10, choisi parmi :

- la (+) 3-(2-hydroxyéthyl)-3-(3,4-dichlorophényl)pipéridine, et
- la (-) 3-(2-hydroxyéthyl)-3-(3,4-dichlorophényl)pipéridine.

## Claims

**Claims for the following Contracting States : AT, BE, CH/LI, DE, DK, FR, GB, IT, LU, NL, PT, SE**

**1.** A compound of the formula

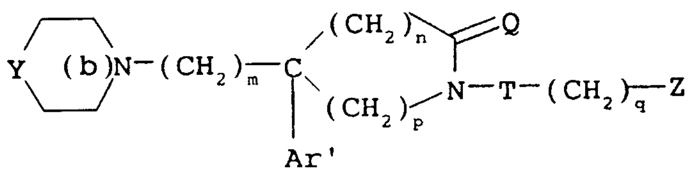

(I)

in which:

- Y is
  - either a group Cy-N or Cy- $CH_2$ -N, in which:
    . Cy is a phenyl which is unsubstituted or monosubstituted or polysubstituted by a substituent selected from: a halogen atom, a hydroxyl, a $C_1$-$C_4$ alkoxy, a $C_1$-$C_4$ alkyl, a trifluoromethyl, said substituents being identical or different; a $C_3$-$C_7$ cycloalkyl group; a pyrimidyl group or a pyridyl group;
- or a group

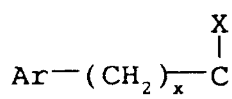

in which

. Ar is a phenyl which is unsubstituted or monosubstituted or polysubstituted by a substituent selected from: a halogen atom, a hydroxyl, a $C_1$-$C_4$ alkoxy, a trifluoromethyl, a $C_1$-$C_4$ alkyl, said substituents being identical or different; a pyridyl group; a thienyl group;
. x is zero or one;
. X is a hydrogen, a hydroxyl, a $C_1$-$C_4$ alkoxy; a $C_1$-$C_4$ acyloxy; a carboxyl; a $C_1$-$C_4$ carbalkoxy; a cyano; a -$N(X_1)_2$ group, in which the groups $X_1$ independently are hydrogen, a $C_1$-$C_4$ alkyl, a $C_1$-$C_4$ hydroxyalkyl, a $C_1$-$C_4$ acyl, or else -$(X_1)_2$ forms, with the nitrogen atom to which it is bonded, a heterocycle selected from pyrrolidine, piperidine or morpholine; a -S-$X_2$ group, in which $X_2$ is hydrogen or a $C_1$-$C_4$ alkyl group;

or else X forms a double bond with the carbon atom to which it is bonded and with the adjacent carbon atom in the heterocycle;

- m is 2 or 3;
- Ar' is a phenyl which is unsubstituted or monosubstituted or polysubstituted by a substituent selected from: a halogen atom, a trifluoromethyl, a $C_1$-$C_4$ alkoxy, a $C_1$-$C_4$ alkyl, said substituents being identical or different; a thienyl; a benzothienyl; a naphthyl or an indolyl;
- n is 0, 1, 2 or 3;
- p is 1 or 2, and when p is equal to 2, n is then equal to 1 and Q is two hydrogen atoms;
- O is oxygen or two hydrogen atoms;

- T is a group selected from

$$-\underset{\underset{O}{\|}}{C}- \quad \text{and} \quad -CH_2-$$

- q is 0, 1, 2 or 3;
- Z is a phenyl which is unsubstituted or monosubstituted or polysubstituted by a substituent selected from halogen, CN, OH, $NH_2$, NH-CO-$NH_2$, $NO_2$, $CONH_2$, $CF_3$, $C_1$-$C_{10}$ alkyl, alkenyl containing 2 to 10 carbon atoms, alkynyl containing 2 to 10 carbon atoms, cycloalkyl containing 3 to 8 carbon atoms, bicycloalkyl containing 4 to 11 carbon atoms, hydroxyalkyl containing 1 to 5 carbon atoms, alkoxy containing 1 to 10 carbon atoms, alkoxyalkyl containing 2 to 10 carbon atoms, alkoxyalkoxyalkyl containing 3 to 10 carbon atoms, alkoxyalkoxy containing 2 to 10 carbon atoms, alkenyloxy containing 2 to 10 carbon atoms, alkenyloxyalkyl containing 3 to 10 carbon atoms, alkynyloxy containing 2 to 10 carbon atoms, alkynyloxyalkyl containing 3 to 10 carbon atoms, cycloalkoxy containing 3 to 8 carbon atoms, alkylthio containing 1 to 10 carbon atoms, alkylthioalkyl containing 2 to 10 carbon atoms, acylamino containing 1 to 7 carbon atoms, acylaminoalkyl containing 2 to 8 carbon atoms, acyloxy containing 1 to 6 carbon atoms, alkoxycarbonyl containing 2 to 5 carbon atoms, cycloalkoxycarbonyl containing 4 to 8 carbon atoms, alkylaminocarbonylamino containing 2 to 4 carbon atoms, dialkylaminocarbonylamino containing 3 to 7 carbon atoms, (pyrrolidin-1-yl)carbonylamino, cycloalkylaminocarbonylamino containing 4 to 8 carbon atoms, alkylaminocarbonylaminoalkyl containing 3 to 9 carbon atoms, dialkylaminocarbonylaminoalkyl containing 4 to 11 carbon atoms, (pyrrolidin-1-yl)carbonylaminoethyl, (piperidin-1-yl)carbonylaminoethyl, alkoxycarbonylaminoalkyl containing 3 to 12 carbon atoms, cycloalkoxycarbonylaminoalkyl containing 5 to 12 carbon atoms, carbamoylalkyl containing 2 to 5 carbon atoms, alkylaminocarbonylalkyl containing 3 to 9 carbon atoms, dialkylaminocarbonylalkyl containing 4 to 11 carbon atoms, (pyrrolidin-1-yl)carbonylmethyl, (piperidin-1-yl)carbonylmethyl, (piperidin-1-yl)-carbonyethyl, cycloalkylaminocarbonylalkyl containing 5 to 12 carbon atoms, alkylaminocarbonylalkoxy containing 3 to 10 carbon atoms, dialkylaminocarbonylalkoxy containing 4 to 10 carbon atoms, (piperidin-1-yl)carbonylmethoxy, cycloalkylaminocarbonylalkoxy containing 5 to 11 carbon atoms, cycloalkylaminocarbonylaminoalkyl containing 5 to 12 carbon atoms;
  a naphthyl or indenyl group in which one or more bonds can be hydrogenated, it being possible for said groups to be unsubstituted or monosubstituted or polysubstituted by a halogen, an alkyl, phenyl, cyano, hydroxyalkyl, hydroxyl, oxo, alkylcarbonylamino, alkoxycarbonyl, thioalkyl group, in which the alkyls are $C_1$-$C_4$ alkyls;
  a pyridyl group; a thiadiazolyl, indolyl, indazolyl, imidazolyl, benzimidazolyl, quinolyl, benzotriazolyl, benzofuranyl, benzothienyl, benzothiazolyl, benzisothiazolyl, isoquinolyl, benzoxazolyl, benzoxazinyl, benzodioxinyl, isoxazolyl, benzopyranyl, thiazolyl, thienyl, furyl, pyranyl, chromenyl, isobenzofuranyl, pyrrolyl, pyrazolyl, pyrazinyl, pyrimidinyl, pyridazinyl, indolizinyl, phthalazinyl, quinazolinyl, acridinyl, isothiazolyl, isochromanyl, chromanyl, in which one or more double bonds can be hydrogenated, it being possible for said groups to be unsubstituted or monosubstituted or polysubstituted by an alkyl, phenyl, cyano, hydroxyalkyl, hydroxyl, alkylcarbonylamino, alkoxycarbonyl, thioalkyl group, in which the alkyls are $C_1$-$C_4$ alkyls; or else when T is - C=O-, -$(CH_2)_q$-Z can also be a benzyl group substituted on the -CH- by a hydroxyl, a $C_1$-$C_4$ alkoxy, a $C_1$-$C_4$ alkyl and unsubstituted or substituted on the aromatic ring by a halogen, a trifluoromethyl, a $C_1$-$C_4$ alkyl, a hydroxyl, a $C_1$-$C_4$ alkoxy;
  or, if appropriate, one of its salts with mineral or organic acids, or, when

$$Y = Ar-(CH_2)_x-\overset{\overset{X}{|}}{C}$$

one of its quaternary ammonium salts formed with nitrogen (b) of the piperidine, or an N-oxide derivative formed with this same nitrogen atom.

**2.** An optically pure compound according to claim 1, of the formula

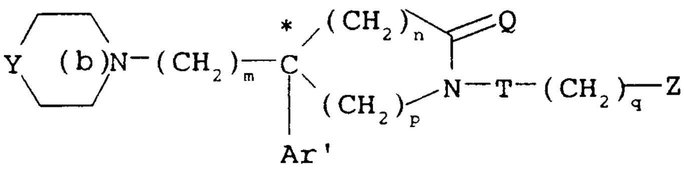

(I*)

in which:

- "*" means that the carbon atom marked with this symbol has a determined (+) or (-) absolute configuration,
- Y, m, Ar', n, p, Q, T, q and Z are as defined in claim 1, or one of its salts with mineral or organic acids, or, with nitrogen atom (b), one of its quaternary ammonium salts or an N-oxide derivative.

**3.** A compound according to claim 1 or claim 2, in which Z is a phenyl group which is unsubstituted or monosubstituted or disubstituted by a halogen or an alkoxy; a naphthyl group.

**4.** A compound according to one of claims 1 to 3 , of formula (I) or (I*)

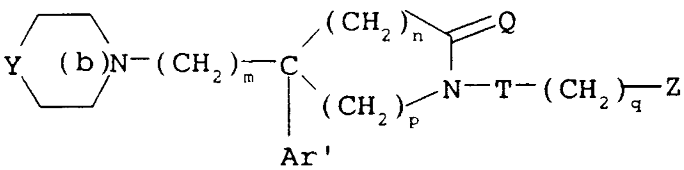

(I)

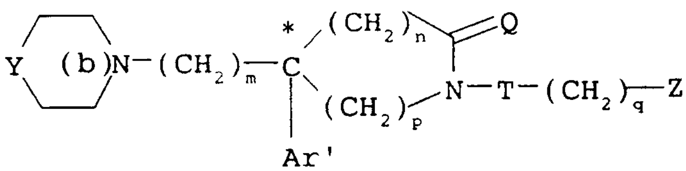

(I*)

in which:
Y is a group

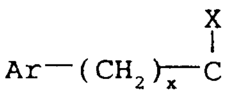

in which Ar, x and X are as defined in claim 1;

- m, Ar', n, p, Q, T, q and Z are as defined in claim 1; in the form of a quaternary ammonium salt or an N-oxide derivative,
characterised in that the group

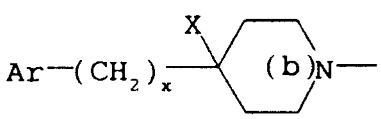

is then represented by the group

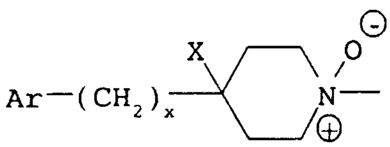

or the group

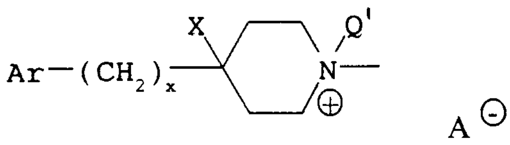

in which:

- Q' is a $C_1$-$C_6$ alkyl group or a benzyl group, and
- $A^-$ is an anion selected from chloride, bromide, iodide, acetate, methanesulfonate or paratoluenesulfonate.

**5.** A method of preparing the compounds of formula (I) according to claim 1, characterised in that:

- a) a compound of the formula

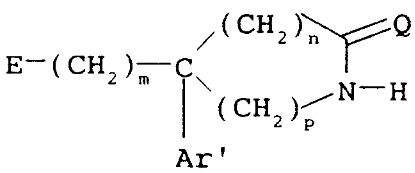

(II)

is treated in which m, Ar', n, p and Q are as defined in claim 1 and E is a hydroxyl or, if appropriate, an O-protected group or a group

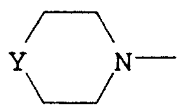

in which Y is as defined in claim 1, it being understood that:
when Y is the group

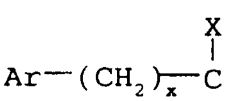

in which X is a hydroxyl, this hydroxyl can be protected,

- either with a functional derivative of an acid of the formula

$$HO-\underset{\underset{O}{\|}}{C}-(CH_2)_q-Z \qquad (III)$$

in which q and Z are as defined in claim 1, when a compound of formula (I) in which T is -CO- is to be prepared,

. or with a halogenated derivative of the formula

$$Hal—(CH_2)_{q+1}—Z \quad (IV)$$

in which q and Z are as defined above and where Hal is a halogen when a compound of formula (I) in which T is $-CH_2-$ is to be prepared,

to give the compound of the formula

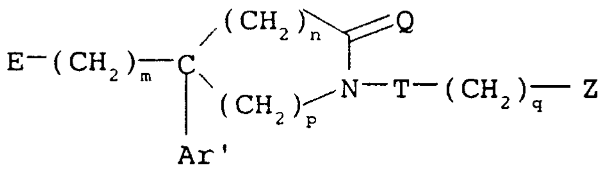

(V)

- b) then, when E is an O-protected group, the O-protecting group is removed by reaction with an acid,
- c) the resulting alcohol of the formula

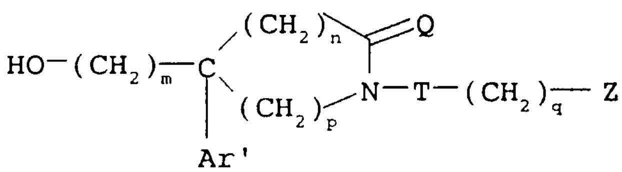

(VI)

is treated with methanesulfonyl chloride,

- d) the resulting mesylate of the formula

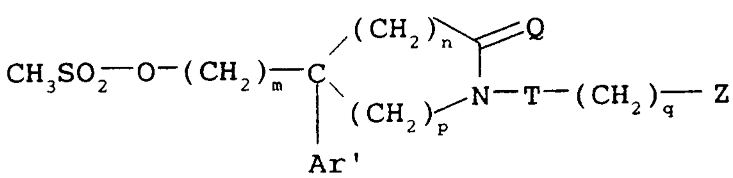

(VII)

is reacted with a secondary amine of the formula

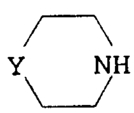

(VIII)

in which Y is as defined above, and

- e) after deprotection of the hydroxyl represented by X, if appropriate, the resulting product, if desired, is converted to one of its salts with mineral or organic acids, or to one of its quaternary ammonium salts formed with nitrogen (b) of the piperidine, or to an N-oxide derivative formed with this same nitrogen atom.

**6.** A compound of the formula

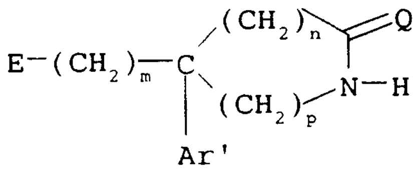

(II)

in which m, Ar', n, p and Q are as defined in claim 1 and E is a hydroxyl or, if appropriate, an O-protected group or a group

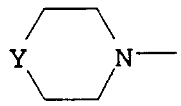

in which Y is as defined in claim 1,

. provided Q is oxygen:

- when E = OH or $(C_1-C_6)$alkylcarbonyloxy, m = 2 or 3, Ar' is a phenyl which is substituted by a $(C_1-C_4)$alkoxy, n = 3 and p = 1;
- when E = $(C_1-C_4)$alkylcarbonyloxy, m = 2, n = 1 and Ar' is a phenyl which is unsubstituted or monosubstituted or polysubstituted by a $(C_1-C_4)$alkyl, a $(C_1-C_4)$alkoxy or a halogen;

. provided that when E = OH or a $(C_1-C_4)$alkoxy, m = 2, p= 1, Ar' is a phenyl which is unsubstituted or monosubstituted or polysubstituted by a $(C_1-C_4)$alkyl, a $(C_1-C_4)$alkoxy or a halogen, n is different from 1,

. provided that when E = OH, Q = 2H, m = 2, n= 1, p = 2, Ar' is different from an unsubstituted phenyl. or one of its salts with mineral or organic acids.

7. A compound of the formula

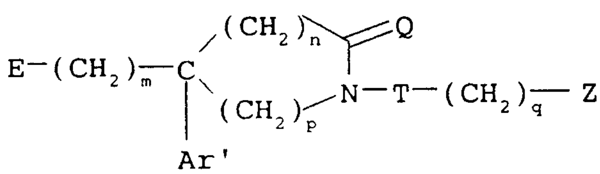

(V)

in which E is a hydroxyl, an O-protected group and m, Ar', n, p, Q, T and Z are as defined in claim 1

. provided that when E = OH, Q = 2H, m = 2, n=1, p = 2, T = $-CH_2-$, q = 0 and Z = phenyl, Ar' is different from an unsubstituted phenyl, and

. provided that when E = OH, Q = 2H, m = 2, n = 1, p = 1, T = $-CH_2-$, q = 0 and Z = phenyl, Ar' is different from a phenyl which is unsubstituted or monosubstituted or polysubstituted by a $(C_1-C_4)$alkyl, a $(C_1-C_4)$alkoxy or a halogen, and

. provided that when E = OH or $(C_1-C_6)$alyklcarbonyloxy, Q = 2H, m = 2 or 3, n = 3, p = 1, T = - $CH_2$-, q = 0, 1, 2 or 3 and Z is an aryl group, Ar' is different from a phenyl which is substituted by a $(C_1-C_4)$alkoxy,

or one of its salts with mineral or organic acids.

8. A compound according to claim 7, of the formula

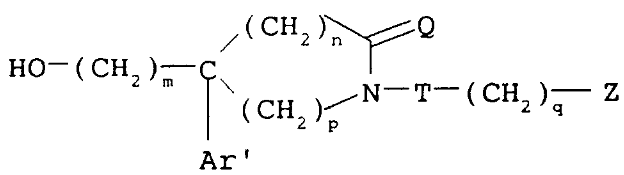

(VI)

in which m, Ar', Q, n, p, T, q and Z are as defined in claim 1,

- provided that when Q = 2H, m = 2, n = 1, p = 2, T = - $CH_2$-, q = 0 and Z = phenyl, Ar' is different from an unsubstituted phenyl, and
- provided that when Q = 2H, m = 2, n = 1, p = 1, T = -$CH_2$-, q = 0 and Z = phenyl, Ar' is different from a phenyl which is unsubstituted or monosubstituted or polysubstituted by a ($C_1$-$C_4$)alkyl, a ($C_1$-$C_4$)alkoxy or a halogen, and
- provided that when Q = 2H, m = 2 or 3, n = 3, p = 1, T = -$CH_2$-, q = 0, 1, 2 or 3 and Z is an aryl group, Ar' is different from a phenyl which is substituted by a ($C_1$-$C_4$)alkoxy,

or one of its salts with mineral or organic acids.

9. A compound of the formula

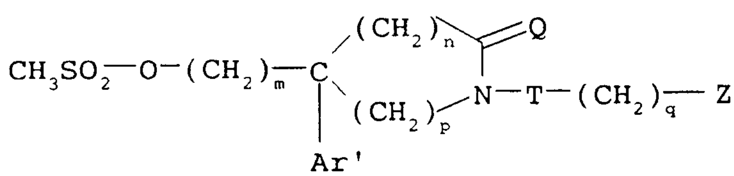

(VII)

in which m, Ar', n, p, Q, T, q and Z are as defined in claim 1, or one of its salts with mineral or organic acids.

10. A compound of the formula

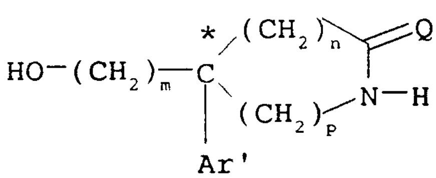

(II*)

in which m, Ar', n and p are as defined in claim 1 and Q represents 2 hydrogen atoms, said compound being in optically pure form, provided that when m = 2 or 3, n = 3 and p= 1, Ar' is different from a phenyl substituted by a $C_1$-$C_4$ alkoxy.

11. A compound of the formula

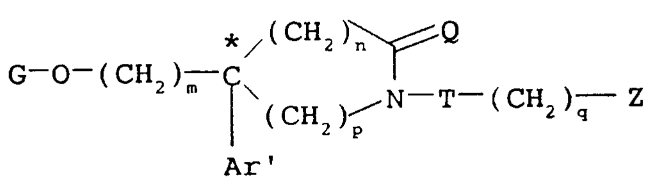

(V*)

in which G is hydrogen or a methanesulfonyl group, m, Ar', n, p, q, T and Z being as defined in claim 1 and Q represents 2 hydrogen atoms, said compound being in optically pure form, provided that when G is hydrogen, m = 2

or 3, n = 3, p = 1 and Z is an aryl group, Ar' is different from a phenyl substituted by a $C_1$-$C_4$ alkoxy.

**12.** A pharmaceutical composition containing, as the active principle, a compound according to any one of claims 1 to 4, or one of its pharmaceutically acceptable salts.

**13.** A pharmaceutical composition according to claim 12, in the form of a dosage unit, in which the active principle is mixed with at least one pharmaceutical excipient.

**14.** A composition according to claim 12 containing from 2.5 to 1000 mg of active principle.

**15.** Compounds according to any one of claims 1 to 4, in which A' and/or Z is/are a phenyl group which is monosubstituted or polysubstituted by a chlorine or fluorine atom.

**16.** A method according to claim 5, characterised in that in the compound of formula (III), E is a tetrahydropyran-2-yloxy group.

**17.** A compound according to claim 6 or 7, in which E is a tetrahydropyran-2-yloxy group.

**18.** A compound of formula (II) according to claim 6, selected from:

5-Tetrahydropyranyloxypropyl-5-(3,4-dichlorophenyl)piperidinone,
3-Tetrahydropyranyloxypropyl-3-(3,4-dichlorophenyl)piperidine, and
3-(2-Hydroxyethyl)-3-(3,4-dichlorophenyl)piperidine.

**19.** An optically pure compound of formula (II*) according to claim 10, selected from:

- 3-(2-Hydroxyethyl)-3-(3,4-dichlorophenyl)piperidine (+), and
- 3-(2-Hydroxyethyl)-3-(3,4-dichlorophenyl)piperidine (-).

**Claims for the following Contracting State : ES**

**1.** A method of preparing compounds of formula (I)

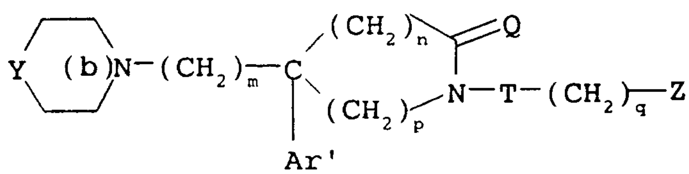

(I)

in which:

- Y is
  - either a group Cy-N or Cy-$CH_2$-N, in which:
    . Cy is a phenyl which is unsubstituted or monosubstituted or polysubstituted by a substituent selected from: a halogen atom, a hydroxyl, a $C_1$-$C_4$ alkoxy, a $C_1$-$C_4$ alkyl, a trifluoromethyl, said substituents being identical or different; a $C_3$-$C_7$ cycloalkyl group; a pyrimidyl group or a pyridyl group;
  - or a group

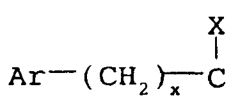

in which

. Ar is a phenyl which is unsubstituted or monosubstituted or polysubstituted by a substituent selected from: a halogen atom, a hydroxyl, a $C_1$-$C_4$ alkoxy, a trifluoromethyl, a $C_1$-$C_4$ alkyl, said substituents being identical or different; a pyridyl group; a thienyl group;
. x is zero or one;
. X is a hydrogen, a hydroxyl, a $C_1$-$C_4$ alkoxy; a $C_1$-$C_4$ acyloxy; a carboxyl; a $C_1$-$C_4$ carbalkoxy; a cyano; a $-N(X_1)_2$ group, in which the groups $X_1$ independently are hydrogen, a $C_1$-$C_4$ alkyl, a $C_1$-$C_4$ hydroxyalkyl, a $C_1$-$C_4$ acyl, or else $-(X_1)_2$ forms, with the nitrogen atom to which it is bonded, a heterocycle selected from pyrrolidine, piperidine or morpholine; a $-S-X_2$ group, in which $X_2$ is hydrogen or a $C_1$-$C_4$ alkyl group;

or else X forms a double bond with the carbon atom to which it is bonded and with the adjacent carbon atom in the heterocycle;
- m is 2 or 3;
- Ar' is a phenyl which is unsubstituted or monosubstituted or polysubstituted by a substituent selected from: a halogen atom, a trifluoromethyl, a $C_1$-$C_4$ alkoxy, a $C_1$-$C_4$ alkyl, said substituents being identical or different; a thienyl; a benzothienyl; a naphthyl or an indolyl;
- n is 0, 1, 2 or 3;
- p is 1 or 2, and when p is equal to 2, n is then equal to 1 and Q is two hydrogen atoms;
- Q is oxygen or two hydrogen atoms;
- T is a group selected from

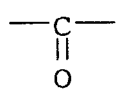 and $-CH_2-$

- q is 0, 1, 2 or 3;
- Z is a phenyl which is unsubstituted or monosubstituted or polysubstituted by a substituent selected from halogen, CN, OH, $NH_2$, $NH-CO-NH_2$, $NO_2$, $CONH_2$, $CF_3$, $C_1$-$C_{10}$ alkyl, alkenyl containing 2 to 10 carbon atoms, alkynyl containing 2 to 10 carbon atoms, cycloalkyl containing 3 to 8 carbon atoms, bicycloalkyl containing 4 to 11 carbon atoms, hydroxyalkyl containing 1 to 5 carbon atoms, alkoxy containing 1 to 10 carbon atoms, alkoxyalkyl containing 2 to 10 carbon atoms, alkoxyalkoxyalkyl containing 3 to 10 carbon atoms, alkoxyalkoxy containing 2 to 10 carbon atoms, alkenyloxy containing 2 to 10 carbon atoms, alkenyloxyalkyl containing 3 to 10 carbon atoms, alkynyloxy containing 2 to 10 carbon atoms, alkynyloxyalkyl containing 3 to 10 carbon atoms, cycloalkoxy containing 3 to 8 carbon atoms, alkylthio containing 1 to 10 carbon atoms, alkylthioalkyl containing 2 to 10 carbon atoms, acylamino containing 1 to 7 carbon atoms, acylaminoalkyl containing 2 to 8 carbon atoms, acyloxy containing 1 to 6 carbon atoms, alkoxycarbonyl containing 2 to 5 carbon atoms, cycloalkoxycarbonyl containing 4 to 8 carbon atoms, alkylaminocarbonylamino containing 2 to 4 carbon atoms, dialkylaminocarbonylamino containing 3 to 7 carbon atoms, (pyrrolidin-1-yl)carbonylamino, cycloalkylaminocarbonylamino containing 4 to 8 carbon atoms, alkylaminocarbonylaminoalkyl containing 3 to 9 carbon atoms, dialkylaminocarbonylaminoalkyl containing 4 to 11 carbon atoms, (pyrrolidin-1-yl)carbonylaminoethyl, (piperidin-1-yl)carbonylaminoethyl, alkoxycarbonylaminoalkyl containing 3 to 12 carbon atoms, cycloalkoxycarbonylaminoalkyl containing 5 to 12 carbon atoms, carbamoylalkyl containing 2 to 5 carbon atoms, alkylaminocarbonylalkyl containing 3 to 9 carbon atoms, dialkylaminocarbonylalkyl containing 4 to 11 carbon atoms, (pyrrolidin-1-yl)carbonylmethyl, (piperidin-1-yl)carbonylmethyl, (piperidin-1-yl)-carbonyethyl, cycloalkylaminocarbonyalkyl containing 5 to 12 carbon atoms, alkylaminocarbonylalkoxy containing 3 to 10 carbon atoms, dialkylaminocarbonylalkoxy containing 4 to 10 carbon atoms, (piperidin-1-yl)carbonylmethoxy, cycloalkylaminocarbonylalkoxy containing 5 to 11 carbon atoms, cycloalkylaminocarbonylaminoalkyl containing 5 to 12 carbon atoms;
a naphthyl or indenyl group in which one or more bonds can be hydrogenated, it being possible for said groups to be unsubstituted or monosubstituted or polysubstituted by a halogen, an alkyl, phenyl, cyano, hydroxyalkyl, hydroxyl, oxo, alkylcarbonylamino, alkoxycarbonyl, thioalkyl group, in which the alkyls are $C_1$-$C_4$ alkyls;
a pyridyl group; a thiadiazolyl, indolyl, indazolyl, imidazolyl, benzimidazolyl, quinolyl, benzotriazolyl, benzofuranyl, benzothienyl, benzothiazolyl, benzisothiazolyl, isoquinolyl, benzoxazolyl, benzoxazinyl, benzodioxinyl, isoxazolyl, benzopyranyl, thiazolyl, thienyl, furyl, pyranyl, chromenyl, isobenzofuranyl, pyrrolyl, pyrazolyl, pyrazinyl, pyrimidinyl, pyridazinyl, indolizinyl, phthalazinyl, quinazolinyl, acridinyl, isothiazolyl, isochromanyl,

chromanyl, in which one or more double bonds can be hydrogenated, it being possible for said groups to be unsubstituted or monosubstituted or polysubstituted by an alkyl, phenyl, cyano, hydroxyalkyl, hydroxyl, alkylcarbonylamino, alkoxycarbonyl, thioalkyl group, in which the alkyls are $C_1$-$C_4$ alkyls; or else when T is - C=O-, $-(CH_2)_q$-Z can also be a benzyl group substituted on the -CH- by a hydroxyl, a $C_1$-$C_4$ alkoxy, a $C_1$-$C_4$ alkyl and unsubstituted or substituted on the aromatic ring by a halogen, a trifluoromethyl, a $C_1$-$C_4$ alkyl, a hydroxyl, a $C_1$-$C_4$ alkoxy;
or, if appropriate, one of its salts with mineral or organic acids, or, when

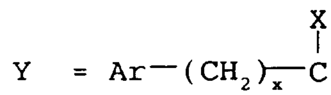

one of its quaternary ammonium salts formed with nitrogen (b) of the piperidine, or an N-oxide derivative formed with this same nitrogen atom,

characterised in that

a) a compound of the formula

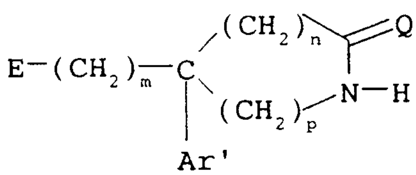

(II)

is treated in which m, Ar', n, p and Q are as defined above and E is a hydroxyl or, if appropriate, an O-protected group or a group

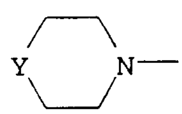

in which Y is as defined above, it being understood that:
when Y is the group

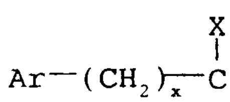

in which X is a hydroxyl, this hydroxyl can be protected,

. either with a functional derivative of an acid of the formula

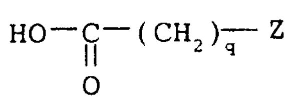

(III)

in which q and Z are as defined above, when a compound of formula (I) in which T is -CO- is to be prepared,
. or with a halogenated derivative of the formula

$$Hal—(CH_2)_{q+1}—Z \qquad (IV)$$

in which q and Z are as defined above and where Hal is a halogen when a compound of formula (I) in which T is $-CH_2-$ is to be prepared,

to give the compound of the formula

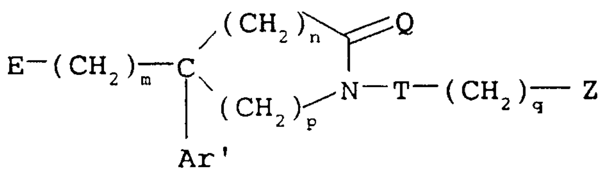

(V)

- b) then, when E is an O-protected group, the O-protecting group is removed by reaction with an acid,
- c) the resulting alcohol of the formula

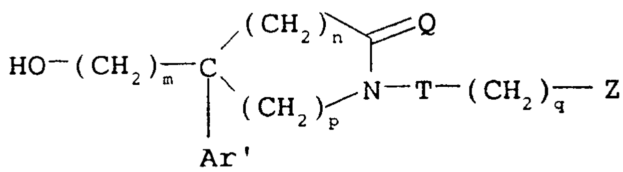

(VI)

is treated with methanesulfonyl chloride,
- d) the resulting mesylate of the formula

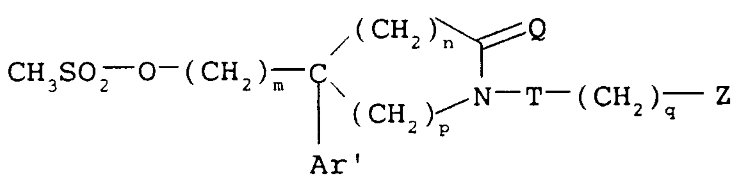

(VII)

is reacted with a secondary amine of the formula

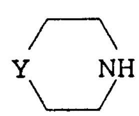

(VIII)

in which Y is as defined above, and
- e) after deprotection of the hydroxyl represented by X, if appropriate, the resulting product, if desired, is converted to one of its salts with mineral or organic acids, or to one of its quaternary ammonium salts formed with nitrogen (b) of the piperidine, or to an N-oxide derivative formed with this same nitrogen atom.

**2.** A method of preparing optically pure compounds of the formula

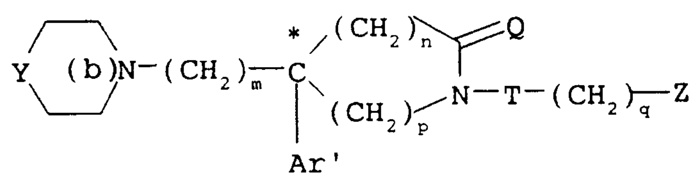

(I*)

in which:

- "*" means that the carbon atom marked with this symbol has a determined (+) or (-) absolute configuration,
- Y, m, Ar', n, p, Q, T, q and Z are as defined in claim 1, or one of their salts with mineral or organic acids, or, with nitrogen atom (b), one of their quaternary ammonium salts or an N-oxide derivative, characterised in that said compounds are isolated by resolution of the racemic mixtures of the componds of formula (I) obtained according to the method of claim 1, by crystallization or chromatography, and if appropriate, converted into one of their salts.

**3.** A method of preparing optically pure compounds of the formula

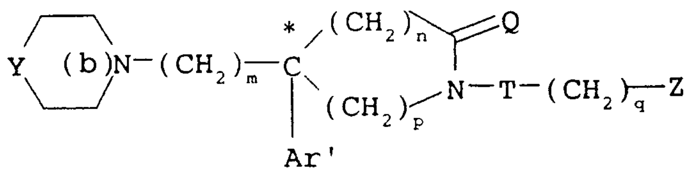

(I*)

in which:

- "*" means that the carbon atom marked with this symbol has a determined (+) or (-) absolute configuration,
- Y, m, Ar', n, p, T, q and Z are as defined in claim 1 and Q is hydrogen, characterised in that:
- a) a compound of the formula

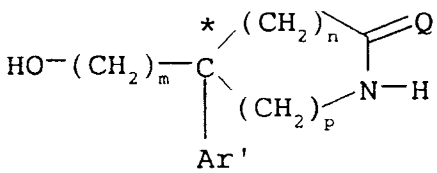

(II*, Q = H)

in which m, Ar', n, p and Q are as defined above, is treated

. either with a functional derivative of an acid of the formula

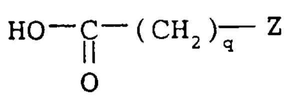

(III)

in which q and Z are as defined above, when a compound of formula (I*) in which T is -CO- is to be prepared,

. or with a halogenated derivative of the formula

$$Hal—(CH_2)_{q+1}—Z \qquad (IV)$$

in which q and Z are as defined above and where Hal is a halogen and preferably a bromine or chlorine atome, when a compound of formula (I*) in which T is $-CH_2-$ is to be prepared,

to give the compound of the formula

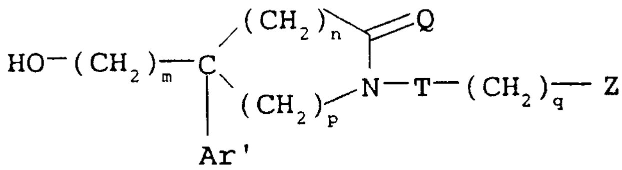

(VI)

- b) the alcohol of formula (VI*) is treated with the methanesulfonyl chloride,
- c) the resulting mesylate of the formula

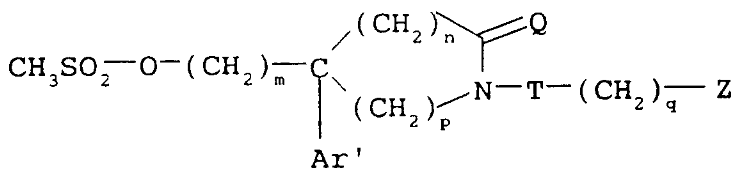

(VII)

is reacted with a secondary amine of the formula

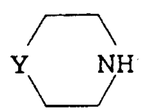

(VIII)

in which Y is as defined above, and

- d) the resulting product, if desired, is converted to one of its salts with mineral or organic acids, or to one of its quaternary ammonium salts formed with nitrogen (b) of the piperidine, or to an N-oxide derivative formed with this same nitrogen atom.

**4.** A method according to any one of claims 1 to 3, characterised in that, in order to convert the compounds (I) or (I*) into one of their quaternary ammonium salts, the free bases of the compounds of formulae (I) or (I*) are reacted in which

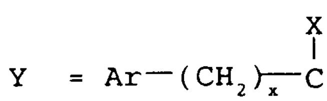

in which any other amine groups present are N-protected by a customary N-protecting group, with an excess of an alkylating agent of the formula

A-Q'

in which A is a leaving group and is as defined hereinafter, and Q' is as defined for (I) or (I*), and the reaction mixture is heated in a solvent selected for example from methylene chloride, chloroform, acetone or acetonitrile, at a temperature between room temperature and the reflux point, for one to several hours, to give a mixture of the axial and equatorial diastereoisomers of the quaternary ammonium salts of the compounds of formulae (I) or (I*), after treatment by the customary methods and after deprotection if appropriate, in which : the group

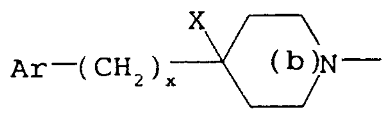

is then represented by the group

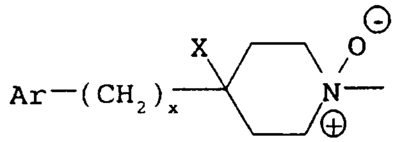

or the group

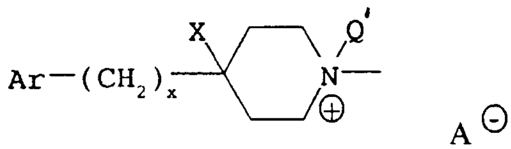

in which:

. Q' is a $C_1$-$C_6$ alkyl group or a benzyl group, and
. $A^-$ is an anion selected from chloride, bromide, iodide, acetate, methanesulfonate or paratoluenesulfonate.

**5.** A method according to any one of claims 1 to 3, characterised in that, in order to convert the compounds of formulae (I) or (I*) into N-oxide derivatives, the compounds of formulae (I) or (I*) in which

Y =

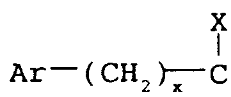

are reacted with a peroxide derivative, for example metachloroperbenzoic acid or hydrogen peroxide, by the customary methods, to give N-oxide derivatives of the compounds (I) or (I*) in which the group : the group

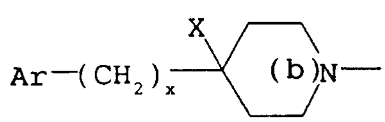

is then represented by the group

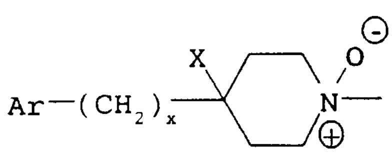

or the group

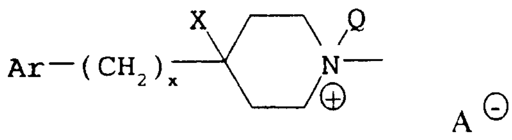

6. A method of preparing compounds of formula (V)

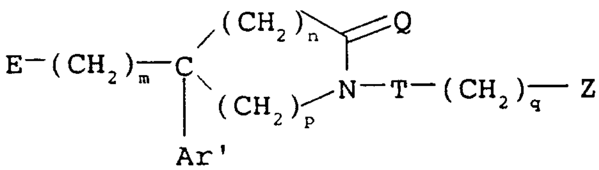

(V)

in which E is a hydroxyl, an O-protected group and m, Ar', n, p, Q, T and Z are as defined in claim 1,

- provided that when E = OH, Q = 2H, m = 2, n = 1, p = 2, T = $-CH_2-$, q = 0 and Z = phenyl, Ar' is different from an unsubstituted phenyl, and
- provided that when E = OH, Q = 2H, m = 2, n = 1, p = 1, T = -CH2-, q = 0 and Z = phenyl, Ar' is different from a phenyl which is unsubstituted or monosubstituted or polysubstituted by a $(C_1-C_4)$alkyl, a $(C_1-C_4)$alkoxy or a halogen, and
- provided that when E = OH or $(C_1-C_6)$alyklcarbonyloxy, Q = 2H, m = 2 or 3, n = 3, p = 1, T = $-CH_2-$, q = 0, 1, 2 or 3 and Z is an aryl group, Ar' is different from a phenyl which is substituted by a $(C_1-C_4)$alkoxy,

or one of its salts with mineral or organic acids.
characterised in that:

- a) a compound of the formula

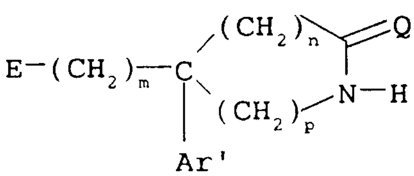

(II)

in which m, Ar', n, p and Q are as defined above and E is a hydroxyl or, if appropriate, an O-protected group or a group

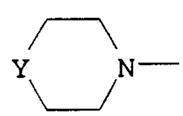

in which Y is as defined above, it being understood that:
when Y is the group

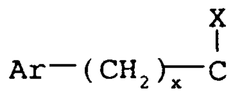

in which X is a hydroxyl, this hydroxyl can be protected, is treated

. either with a functional derivative of an acid of the formula

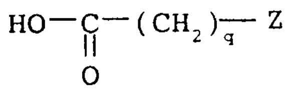 (III)

in which q and Z are as defined above, when a compound of formula (V) in which T is -CO- is to be prepared,

. or with a halogenated derivative of the formula

$$Hal—(CH_2)_{q+1}—Z \qquad (IV)$$

in which q and Z are as defined above and where Hal is a halogen when a compound of formula (I) in which T is $-CH_2-$ is to be prepared,

to give the compound of formula (V) and

- b) after deprotection of the hydroxyl represented by X, if appropriate, the resulting product, if desired, is converted to one of its salts with mineral or organic acids.

**7.** A method of preparing compounds of formula (VI)

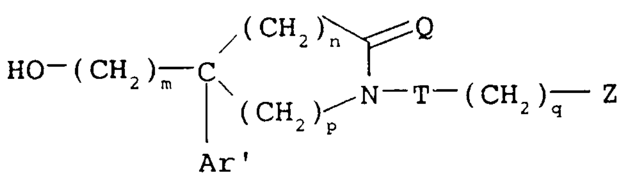 (VI)

in which m, Ar', Q, n, p, T, q and Z are as defined in claim 1,

. provided that when Q = 2H, m = 2, n = 1, p = 2, T = $-CH_2-$, q = 0 and Z = phenyl, Ar' is different from an unsubstituted phenyl, and

. provided that when Q = 2H, m = 2, n = 1, p = 1, T = $-CH_2-$, q = 0 and Z = phenyl, Ar' is different from a phenyl which is unsubstituted or monosubstituted or polysubstituted by a $(C_1-C_4)$alkyl, a $(C_1-C_4)$alkoxy or a halogen, and

. provided that when Q = 2H, m = 2 or 3, n = 3, p = 1, T = $-CH_2-$, q = 0, 1, 2 or 3 and Z is an aryl group, Ar' is different from a phenyl which is substituted by a $(C_1-C_4)$alkoxy,

or one of its salts with mineral or organic acids,
characterised in that

- a) a compound of the formula

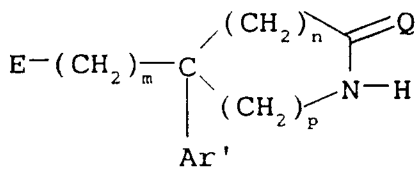

(II)

is treated in which m, Ar', n, p and Q are as defined above and E is a hydroxyl or, if appropriate, an O-protected group or a group

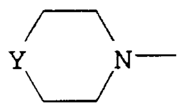

in which Y is as defined above, it being understood that: when Y is the group

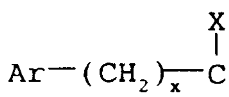

in which X is a hydroxyl, this hydroxyl can be protected,

- either with a functional derivative of an acid of the formula

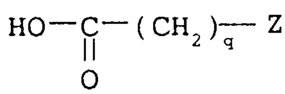

(III)

  in which q and Z are as defined above, when a compound of formula (VI) in which T is -CO- is to be prepared,
- or with a halogenated derivative of the formula

$$Hal—(CH_2)_{q+1}—Z \qquad (IV)$$

  in which q and Z are as defined above and where Hal is a halogen when a compound of formula (VI) in which T is $-CH_2-$ is to be prepared,

to give the compound of the formula

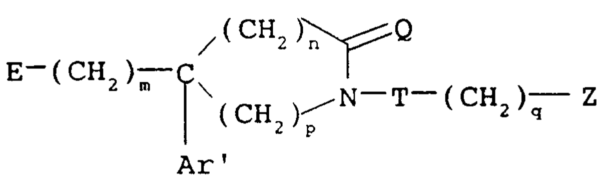

(V)

- b) then, when E is an O-protected group, the O-protecting group is removed by reaction with an acid, and
- c) after deprotection of the hydroxyl represented by X, if appropriate, the resulting product, if desired, is converted to one of its salts with mineral or organic acids.

**8.** A method of preparing compounds of formula (VII)

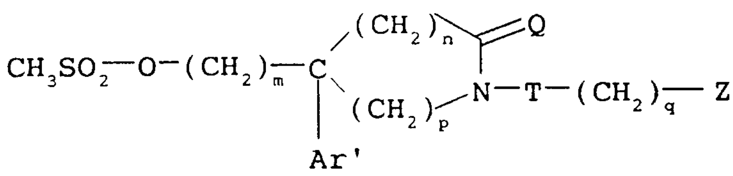

(VII)

in which m, Ar', n, p, Q, T, q and Z are as defined in claim 1, or one of its salts with mineral or organic acids, characterised in that

- a) a compound of the formula

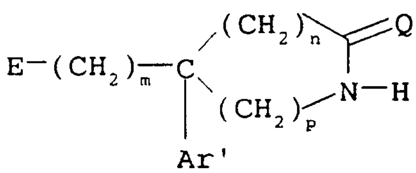

(II)

is treated in which m, Ar', n, p and Q are as defined above and E is a hydroxyl or, if appropriate, an O-protected group or a group

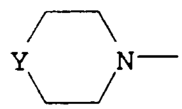

in which Y is as defined above, it being understood that:
when Y is the group

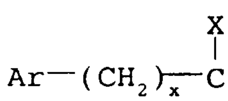

in which X is a hydroxyl, this hydroxyl can be protected,

. either with a functional derivative of an acid of the formula

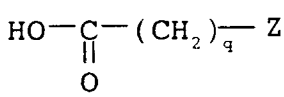

(III)

in which q and Z are as defined above, when a compound of formula (VII) in which T is -CO- is to be prepared,

. or with a halogenated derivative of the formula

$$Hal—(CH_2)_{q+1}—Z \qquad (IV)$$

in which q and Z are as defined above and where Hal is a halogen when a compound of formula (VII) in which T is $-CH_2-$ is to be prepared,

to give the compound of the formula

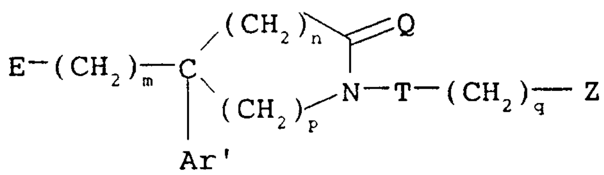

(V)

- b) then, when E is an O-protected group, the O-protecting group is removed by reaction with an acid, and
- c) the resulting alcohol of the formula

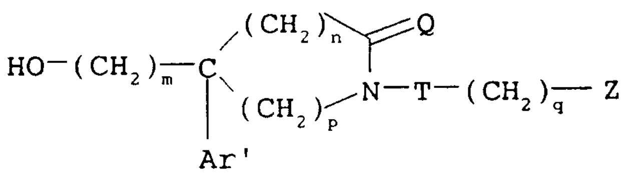

(VI)

is treated with methanesulfonyl chloride,

- d) after deprotection of the hydroxyl represented by X, if appropriate, the resulting product, if desired, is converted to one of its salts with mineral or organic acids.

**9.** A method of preparing compounds of formula (II*)

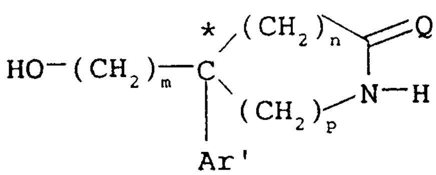

(II*)

in which

- "*" means that the carbon atom marked with this symbol has a determined (+) or (-) absolute configuration,
- m, Ar', n and p are as defined in claim 1
- Q is hydrogen or one of their salts with mineral or organic acids, characterised in that said compounds are isolated by resolution of the racemic mixtures of the compounds of formula (II), by crystallization or chromatography, and if appropriate, converted into one of their salts.

**10.** A method of preparing compounds of the formula

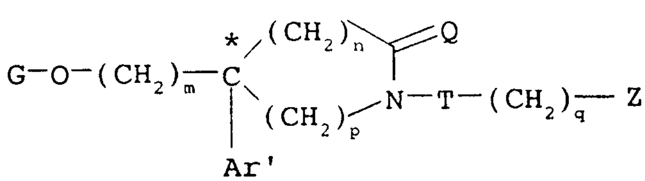

(V*, Q = H)

in which

- "*" means that the carbon atom marked with this symbol has a determined (+) or (-) absolute configuration,
- m, Ar', n and p are as defined in claim 1,
- Q is hydrogen,

- G is hydrogen or a methanesulfonyl group,

characterised in that

a) a compound of the formula

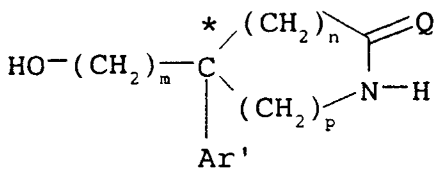

(II*, Q = H)

in which m, Ar', n, p and Q are as defined above, is treated

. either with a functional derivative of an acid of the formula

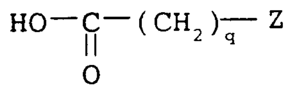

(III)

in which q and Z are as defined above, when a compound of formula (V*) in which T is -CO- is to be prepared,

. or with a halogenated derivative of the formula

$$Hal—(CH_2)_{q+1}—Z \quad (IV)$$

in which q and Z are as defined above and where Hal is a halogen when a compound of formula (V*) in which T is $-CH_2-$ is to be prepared,

to give the compound of formula (V*) in which G is the hydrogen that is treated, if desired, with the methanesulfonyl chloride to give the compound of formula (V*) in which G is a methanesulfonyl group, and

- b) the resulting product is converted to one of its salts with mineral or organic acids.

**11.** A method according to any one of claims 1, 6, 7 or 8, characterised in that in the compound of formula (II), E is a tetrahydropyran-2-yloxy group.

**12.** A method according to claim 9, for the preparation of a compound of formula (II*) in which

- m = 2
- Ar' = 3,4-dichlorophenyl
- n = 2
- p = 1

in the optically pure form (+) or (-), characterised in that said compounds are isolated by resolution of the racemic mixtures of the componds of formula (II), by crystallization or chromatography, and if appropriate, converted into one of their salts.

**13.** A method of preparing a pharmaceutical composition, characterised in that a compound of formula (I) or (I*) prepared by the method according to any one of claims 1 to 5 is mixed, as the active principle, with a pharmaceutically acceptable vehicle.

**Claims for the following Contracting State : GR**

**1.** A compound of the formula

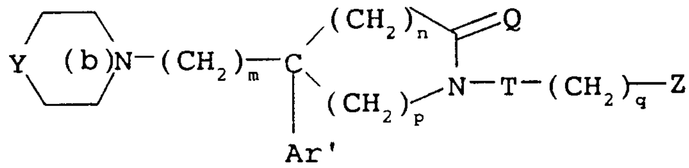

(I)

in which:

- Y is
  - either a group Cy-N or Cy- $CH_2$ -N, in which:
    . Cy is a phenyl which is unsubstituted or monosubstituted or polysubstituted by a substituent selected from: a halogen atom, a hydroxyl, a $C_1$-$C_4$ alkoxy, a $C_1$-$C_4$ alkyl, a trifluoromethyl, said substituents being identical or different; a $C_3$-$C_7$ cycloalkyl group; a pyrimidyl group or a pyridyl group;
  - or a group

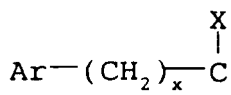

  in which

    . Ar is a phenyl which is unsubstituted or monosubstituted or polysubstituted by a substituent selected from: a halogen atom, a hydroxyl, a $C_1$-$C_4$ alkoxy, a trifluoromethyl, a $C_1$-$C_4$ alkyl, said substituents being identical or different; a pyridyl group; a thienyl group;
    . x is zero or one;
    . X is a hydrogen, a hydroxyl, a $C_1$-$C_4$ alkoxy; a $C_1$-$C_4$ acyloxy; a carboxyl; a $C_1$-$C_4$ carbalkoxy; a cyano; a -$N(X_1)_2$ group, in which the groups $X_1$ independently are hydrogen, a $C_1$-$C_4$ alkyl, a $C_1$-$C_4$ hydroxyalkyl, a $C_1$-$C_4$ acyl, or else -$(X_1)_2$ forms, with the nitrogen atom to which it is bonded, a heterocycle selected from pyrrolidine, piperidine or morpholine; a -S-$X_2$ group, in which $X_2$ is hydrogen or a $C_1$-$C_4$ alkyl group;

  or else X forms a double bond with the carbon atom to which it is bonded and with the adjacent carbon atom in the heterocycle;
- m is 2 or 3;
- Ar' is a phenyl which is unsubstituted or monosubstituted or polysubstituted by a substituent selected from: a halogen atom, a trifluoromethyl, a $C_1$-$C_4$ alkoxy, a $C_1$-$C_4$ alkyl, said substituents being identical or different; a thienyl; a benzothienyl; a naphthyl or an indolyl;
- n is 0, 1, 2 or 3;
- p is 1 or 2, and when p is equal to 2, n is then equal to 1 and Q is two hydrogen atoms;
- Q is oxygen or two hydrogen atoms;
- T is a group selected from

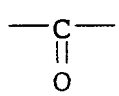 and $-CH_2-$

- q is 0, 1, 2 or 3;
- Z is a phenyl which is unsubstituted or monosubstituted or polysubstituted by a substituent selected from halogen, CN, OH, $NH_2$, NH-CO-$NH_2$, $NO_2$, $CONH_2$, $CF_3$, $C_1$-$C_{10}$ alkyl, alkenyl containing 2 to 10 carbon atoms,

alkynyl containing 2 to 10 carbon atoms, cycloalkyl containing 3 to 8 carbon atoms, bicycloalkyl containing 4 to 11 carbon atoms, hydroxyalkyl containing 1 to 5 carbon atoms, alkoxy containing 1 to 10 carbon atoms, alkoxyalkyl containing 2 to 10 carbon atoms, alkoxyalkoxyalkyl containing 3 to 10 carbon atoms, alkoxyalkoxy containing 2 to 10 carbon atoms, alkenyloxy containing 2 to 10 carbon atoms, alkenyloxyalkyl containing 3 to 10 carbon atoms, alkynyloxy containing 2 to 10 carbon atoms, alkynyloxyalkyl containing 3 to 10 carbon atoms, cycloalkoxy containing 3 to 8 carbon atoms, alkylthio containing 1 to 10 carbon atoms, alkylthioalkyl containing 2 to 10 carbon atoms, acylamino containing 1 to 7 carbon atoms, acylaminoalkyl containing 2 to 8 carbon atoms, acyloxy containing 1 to 6 carbon atoms, alkoxycarbonyl containing 2 to 5 carbon atoms, cycloalkoxycarbonyl containing 4 to 8 carbon atoms, alkylaminocarbonylamino containing 2 to 4 carbon atoms, dialkylaminocarbonylamino containing 3 to 7 carbon atoms, (pyrrolidin-1-yl)carbonylamino, cycloalkylaminocarbonylamino containing 4 to 8 carbon atoms, aikylaminocarbonylaminoalkyl containing 3 to 9 carbon atoms, dialkylaminocarbonylaminoalkyl containing 4 to 11 carbon atoms, (pyrrolidin-1-yl)carbonylaminoethyl, (piperidin-1-yl)carbonylaminoethyl, alkoxycarbonylaminoalkyl containing 3 to 12 carbon atoms, cycloalkoxycarbonylaminoalkyl containing 5 to 12 carbon atoms, carbamoylalkyl containing 2 to 5 carbon atoms, alkylaminocarbonylalkyl containing 3 to 9 carbon atoms, dialkylaminocarbonylalkyl containing 4 to 11 carbon atoms, (pyrrolidin-1-yl)carbonylmethyl, (piperidin-1-yl)carbonylmethyl, (piperidin-1-yl)-carbonyethyl, cycloalkylaminocarbonylalkyl containing 5 to 12 carbon atoms, alkylaminocarbonylalkoxy containing 3 to 10 carbon atoms, dialkylaminocarbonylalkoxy containing 4 to 10 carbon atoms, (piperidin-1-yl)carbonylmethoxy, cycloalkylaminocarbonylalkoxy containing 5 to 11 carbon atoms, cycloalkylaminocarbonylaminoalkyl containing 5 to 12 carbon atoms;
a naphthyl or indenyl group in which one or more bonds can be hydrogenated, it being possible for said groups to be unsubstituted or monosubstituted or polysubstituted by a halogen, an alkyl, phenyl, cyano, hydroxyalkyl, hydroxyl, oxo, alkylcarbonylamino, alkoxycarbonyl, thioalkyl group, in which the alkyls are $C_1$-$C_4$ alkyls;
a pyridyl group; a thiadiazolyl, indolyl, indazolyl, imidazolyl, benzimidazolyl, quinolyl, benzotriazolyl, benzofuranyl, benzothienyl, benzothiazolyl, benzisothiazolyl, isoquinolyl, benzoxazolyl, benzoxazinyl, benzodioxinyl, isoxazolyl, benzopyranyl, thiazolyl, thienyl, furyl, pyranyl, chromenyl, isobenzofuranyl, pyrrolyl, pyrazolyl, pyrazinyl, pyrimidinyl, pyridazinyl, indolizinyl, phthalazinyl, quinazolinyl, acridinyl, isothiazolyl, isochromanyl, chromanyl, in which one or more double bonds can be hydrogenated, it being possible for said groups to be unsubstituted or monosubstituted or polysubstituted by an alkyl, phenyl, cyano, hydroxyalkyl, hydroxyl, alkylcarbonylamino, alkoxycarbonyl, thioalkyl group, in which the alkyls are $C_1$-$C_4$ alkyls; or else when T is - C=O-, $-(CH_2)_q$-Z can also be a benzyl group substituted on the -CH- by a hydroxyl, a $C_1$-$C_4$ alkoxy, a $C_1$-$C_4$ alkyl and unsubstituted or substituted on the aromatic ring by a halogen, a trifluoromethyl, a $C_1$-$C_4$ alkyl, a hydroxyl, a $C_1$-$C_4$ alkoxy;
or, if appropriate, one of its salts with mineral or organic acids, or, when

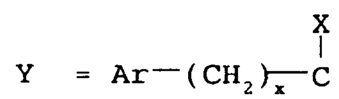

one of its quaternary ammonium salts formed with nitrogen (b) of the piperidine, or an N-oxide derivative formed with this same nitrogen atom.

2. An optically pure compound according to claim 1, of the formula

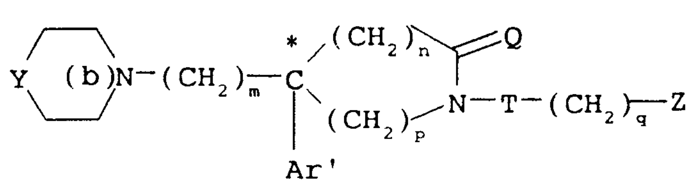

(I*)

in which:

- "*" means that the carbon atom marked with this symbol has a determined (+) or (-) absolute configuration,
- Y, m, Ar', n, p, Q, T, q and Z are as defined in claim 1, or one of its salts with mineral or organic acids, or, with nitrogen atom (b), one of its quaternary ammonium salts or an N-oxide derivative.

3. A compound according to claim 1 or claim 2, in which Z is a phenyl group which is unsubstituted or monosubstituted or disubstituted by a halogen or an alkoxy; a naphthyl group.

4. A compound according to one of claims 1 to 3 , of formula (I) or (I*)

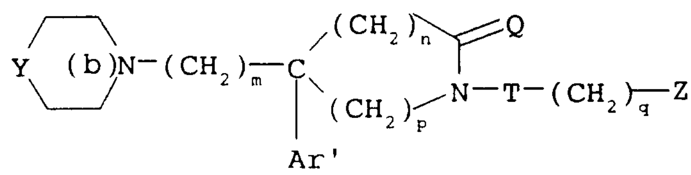

(I)

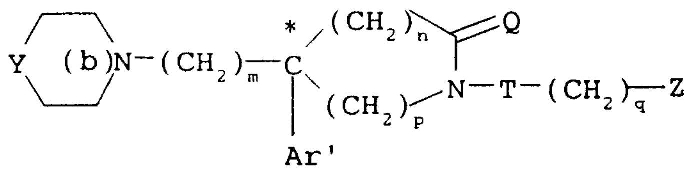

(I*)

in which:
Y is a group

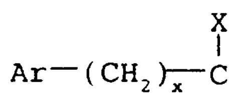

in which Ar, x and X are as defined in claim 1;

- m, Ar', n, p, Q, T, q and Z are as defined in claim 1;

in the form of a quaternary ammonium salt or an N-oxide derivative, characterised in that the group

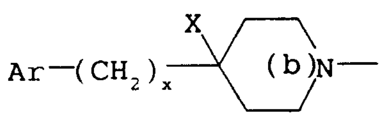

is then represented by the group

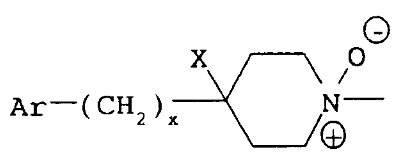

or the group

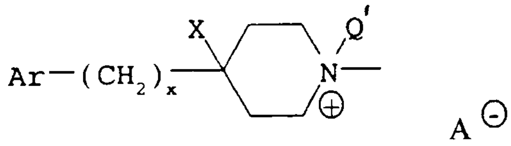

in which:

. Q' is a $C_1$-$C_6$ alkyl group or a benzyl group, and
. $A^-$ is an anion selected from chloride, bromide, iodide, acetate, methanesulfonate or paratoluenesulfonate.

**5.** A method of preparing the compounds of formula (I) according to claim 1, characterised in that:

- a) a compound of the formula

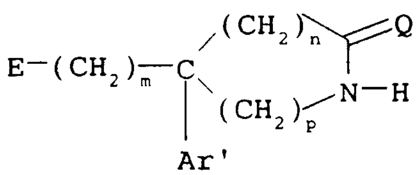

(II)

is treated in which m, Ar', n, p and Q are as defined in claim 1 and E is a hydroxyl or, if appropriate, an O-protected group or a group

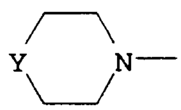

in which Y is as defined in claim 1, it being understood that:
when Y is the group

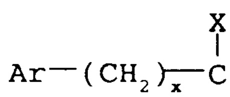

in which X is a hydroxyl, this hydroxyl can be protected,

. either with a functional derivative of an acid of the formula

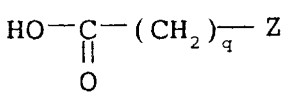

(III)

in which q and Z are as defined in claim 1, when a compound of formula (I) in which T is -CO- is to be prepared,
. or with a halogenated derivative of the formula

$$Hal—(CH_2)_{q+1}—Z \qquad (IV)$$

in which q and Z are as defined above and where Hal is a halogen when a compound of formula (I) in which T is $-CH_2-$ is to be prepared,

to give the compound of the formula

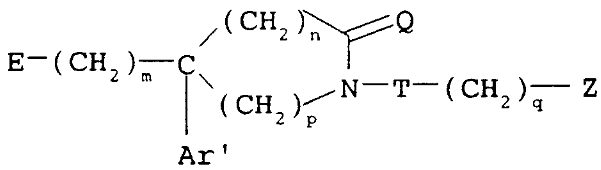

(V)

- b) then, when E is an O-protected group, the O-protecting group is removed by reaction with an acid,
- c) the resulting alcohol of the formula

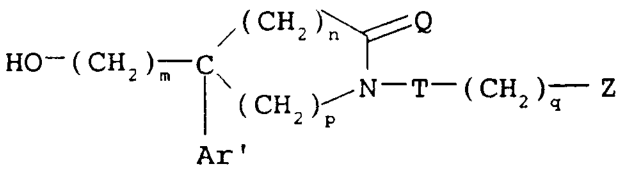

(VI)

is treated with methanesulfonyl chloride,

- d) the resulting mesylate of the formula

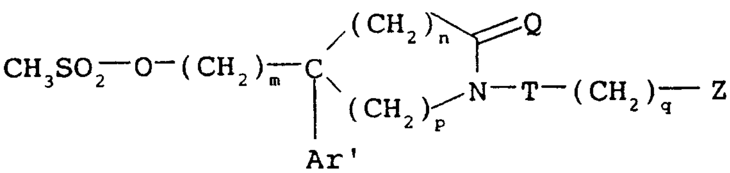

(VII)

is reacted with a secondary amine of the formula

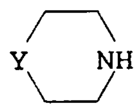

(VIII)

in which Y is as defined above, and

- e) after deprotection of the hydroxyl represented by X, if appropriate, the resulting product, if desired, is converted to one of its salts with mineral or organic acids, or to one of its quaternary ammonium salts formed with nitrogen (b) of the piperidine, or to an N-oxide derivative formed with this same nitrogen atom.

**6.** A compound of the formula

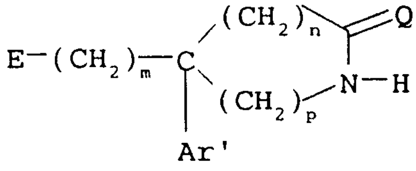

(II)

in which m, Ar', n, p and Q are as defined in claim 1 and E is a hydroxyl or, if appropriate, an O-protected group or a group

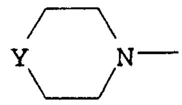

in which Y is as defined in claim 1,

. provided Q is oxygen:

- when E = OH or ($C_1$-$C_6$)alkylcarbonyloxy, m = 2 or 3, Ar' is a phenyl which is substituted by a ($C_1$-$C_4$)alkoxy, n = 3 and p = 1;
- when E = ($C_1$-$C_4$)alkylcarbonyloxy, m = 2, n = 1 and Ar' is a phenyl which is unsubstituted or monosubstituted or polysubstituted by a ($C_1$-$C_4$)alkyl, a ($C_1$-$C_4$)alkoxy or a halogen.

. provided that when E = OH or a ($C_1$-$C_4$)alkoxy, m = 2, p = 1, Ar' is a phenyl which is unsubstituted or monosubstituted or polysubstituted by a ($C_1$-$C_4$)alkyl, a ($C_1$-$C_4$)alkoxy or a halogen, n is different from 1.
. provided that when E = OH, Q = 2H, m = 2, n = 1, p = 2, Ar' is different from an unsubstituted phenyl.

or one of its salts with mineral or organic acids.

**7.** A compound of the formula

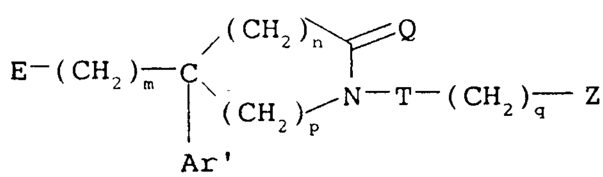

(V)

in which E is a hydroxyl, an O-protected group and m, Ar', n, p, Q, T and Z are as defined in claim 1

. provided that when E = OH, Q = 2H, m = 2, n = 1, p = 2, T = -$CH_2$-, q = 0 and Z = phenyl, Ar' is different from an unsubstituted phenyl, and
. provided that when E = OH, Q = 2H, m = 2, n = 1, p = 1, T = -$CH_2$-, q = 0 and Z = phenyl, Ar' is different from a phenyl which is unsubstituted or monosubstituted or polysubstituted by a ($C_1$-$C_4$)alkyl, a ($C_1$-$C_4$)alkoxy or a halogen, and
. provided that when E = OH or ($C_1$-$C_6$)alyklcarbonyloxy, Q = 2H, m = 2 or 3, n = 3, p = 1, T = -$CH_2$-, q = 0, 1, 2 or 3 and Z is an aryl group, Ar' is different from a phenyl which is substituted by a ($C_1$-$C_4$)alkoxy,

or one of its salts with mineral or organic acids.

**8.** A compound according to claim 7, of the formula

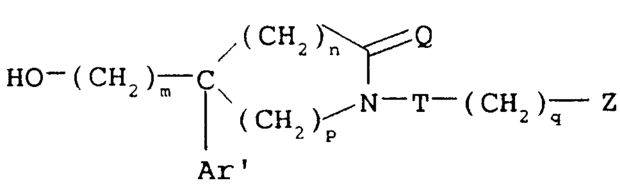

(VI)

in which m, Ar', Q, n, p, T, q and Z are as defined in claim 1,

. provided that when Q = 2H, m = 2, n = 1, p = 2, T = $CH_2$-, q = 0 and Z = phenyl, Ar' is different from an unsubstituted phenyl, and

. provided that when Q = 2H, m = 2, n = 1, p = 1, T = $-CH_2-$, q = 0 and Z = phenyl, Ar' is different from a phenyl which is unsubstituted or monosubstituted or polysubstituted by a $(C_1-C_4)$alkyl, a $(C_1-C_4)$alkoxy or a halogen, and

. provided that when Q = 2H, m = 2 or 3, n = 3, p = 1, T = $-CH_2-$, q = 0, 1, 2 or 3 and Z is an aryl group, Ar' is different from a phenyl which is substituted by a $(C_1-C_4)$alkoxy,

or one of its salts with mineral or organic acids.

**9.** A compound of the formula

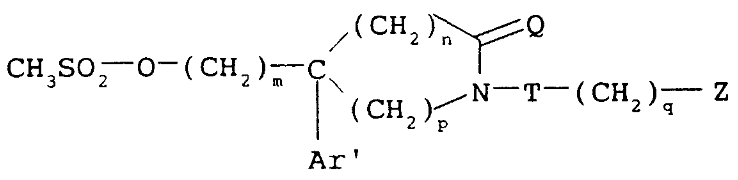

(VII)

in which m, Ar', n, p, Q, T, q and Z are as defined in claim 1, or one of its salts with mineral or organic acids.

**10.** A compound of the formula

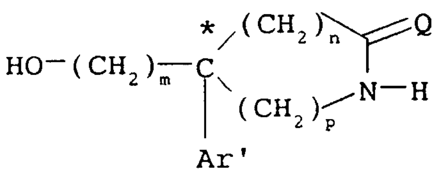

(II*)

in which m, Ar', n and p are as defined in claim 1 and Q represents 2 hydrogen atoms, said compound being in optically pure form, provided that when m = 2 or 3, n = 3 and p = 1, Ar' is different from a phenyl substituted by a $C_1$-$C_4$ alkoxy.

**11.** A compound of the formula

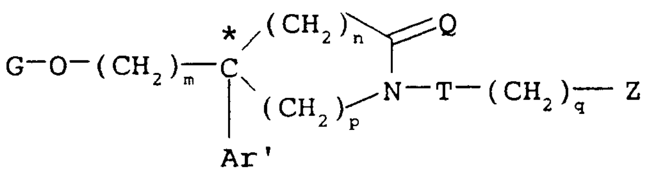

(V*)

in which G is hydrogen or a methanesulfonyl group, m, Ar', n, p, q, T and Z being as defined in claim 1 and Q represents 2 hydrogen atoms, said compound being in optically pure form, provided that when G is hydrogen, m = 2 or 3, n = 3, p = 1 and Z is an aryl group, Ar' is different from a phenyl substituted by a $C_1$-$C_4$ alkoxy.

**12.** A method of preparing a pharmaceutical composition characterised in that a compound according to any one of claims 1 to 4 is mixed, as the active principle, with a pharmaceutically acceptable vehicle.

**13.** Compounds according to any one of claims 1 to 4, in which A' and/or Z is/are a phenyl group which is monosubstituted or polysubstituted by a chlorine or fluorine atom.

**14.** A method according to claim 5, characterised in that in the compound of formula (III), E is a tetrahydropyran-2-yloxy group.

**15.** A compound according to claim 6 or 7, in which E is a tetrahydropyran-2-yloxy group.

**16.** A compound of formula (II) according to claim 6, selected from:

5-Tetrahydropyranyloxypropyl-5-(3,4-dichlorophenyl)piperidinone,
3-Tetrahydropyranyloxypropyl-3-(3,4-dichlorophenyl)piperidine, and
3-(2-Hydroxyethyl)-3-(3,4-dichlorophenyl)piperidine.

**17.** An optically pure compound of formula (II*) according to claim 10, selected from:

- 3-(2-Hydroxyethyl)-3-(3,4-dichlorophenyl)piperidine (+), and
- 3-(2-Hydroxyethyl)-3-(3,4-dichlorophenyl)piperidine (-).

**Patentansprüche**

**Patentansprüche für folgende Vertraggsstaaten: AT, BE, CH/LI, DE, DK, FR, GB, IT, LU, NL, PT, SE**

**1.** Verbindung der Formel:

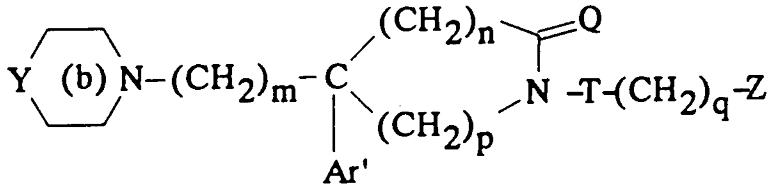

(I),

worin

- Y bedeutet:

- entweder eine Gruppe Cy-N oder Cy-$CH_2$-N, wobei

• Cy darstellt: ein Phenyl, unsubstituiert oder substituiert einfach oder mehrfach durch einen Substituenten, ausgewählt aus: einem Halogenatom, einem Hydroxyl, einem $C_1$-$C_4$-Alkoxy, einem $C_1$-$C_4$-Alkyl, einem Trifluormethyl, welche Substituenten gleich oder verschieden sind; eine $C_3$-$C_7$-Cycloalkyl-Gruppe; eine Pyrimidyl-Gruppe oder eine Pyridyl-Gruppe;

- oder eine Gruppe

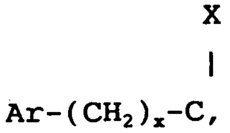

wobei

• Ar bedeutet: ein Phenyl, unsubstituiert oder substituiert einfach oder mehrfach durch einen Substituenten, ausgewählt aus: einem Halogenatom, einem Hydroxyl, einem $C_1$-$C_4$-Alkoxy, einem Trifluormethyl, einem $C_1$-$C_4$-Alkyl, welche Substituenten gleich oder verschieden sind; eine Pyridyl-Gruppe; eine Thienyl-Gruppe;
• x Null oder 1 ist;
• X darstellt: einen Wasserstoff, ein Hydroxyl, ein $C_1$-$C_4$-Alkoxy; ein $C_1$-$C_4$-Acyloxy; ein Carboxy; ein $C_1$-$C_4$-Carbalkoxy; ein Cyano; eine Gruppe -$N(X_1)_2$, wobei die Gruppen $X_1$ unabhängig bedeuten: Wasserstoff, ein $C_1$-$C_4$-Alkyl, ein $C_1$-$C_4$-Hydroxyalkyl, ein $C_1$-$C_4$-Acyl, oder auch -$(X_1)_2$ mit dem Stickstoffatom, an das es gebunden ist, einen Heterocyclus bildet, ausgewählt aus Pyrrolidin, Piperidin

oder Morpholin; eine Gruppe -S-$X_2$, wobei $X_2$ Wasserstoff oder eine $C_1$-$C_4$-Alkyl-Gruppe ist;

oder auch X mit dem Kohlenstoffatom, an das es gebunden ist, und mit dem benachbarten Kohlenstoffatom im Heterocyclus eine Doppelbindung bildet;
- m 2 oder 3 ist;
- Ar' bedeutet: ein Phenyl, unsubstituiert oder substituiert einfach oder mehrfach durch einen Substituenten, ausgewählt aus: einem Halogenatom, einem Trifluormethyl, einem $C_1$-$C_4$-Alkoxy, einem $C_1$-$C_4$-Alkyl, welche Substituenten gleich oder verschieden sind; ein Thienyl; ein Benzothienyl; ein Naphthyl oder ein Indolyl;
- n Null, 1, 2 oder 3 ist;
- p 1 oder 2 ist, und wenn p 2 ist, n 1 ist, und Q zwei Wasserstoffatome darstellt;
- Q Sauerstoff oder zwei Wasserstoffatome bedeutet;
- T eine Gruppe ist, ausgewählt aus

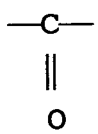 und $-CH_2-$ ;

- q Null, 1, 2 oder 3 ist;
- Z darstellt: ein Phenyl, unsubstituiert oder substituiert durch einen oder mehrere Substituenten, ausgewählt aus: Halogen, CN, OH, $NH_2$, NH-CO-$NH_2$, $NO_2$, $CONH_2$, $CF_3$, $C_1$-$C_{10}$-Alkyl, Alkenyl mit 2 bis 10 Kohlenstoffatomen, Alkinyl mit 2 bis 10 Kohlenstoffatomen, Cycloalkyl mit 3 bis 8 Kohlenstoffatomen, Bicycloalkyl mit 4 bis 11 Kohlenstoffatomen, Hydroxyalkyl mit 1 bis 5 Kohlenstoffatomen, Alkoxy mit 1 bis 10 Kohlenstoffatomen, Alkoxyalkyl mit 2 bis 10 Kohlenstoffatomen, Alkoxyalkoxyalkyl mit 3 bis 10 Kohlenstoffatomen, Alkoxyalkoxy mit 2 bis 10 Kohlenstoffatomen, Alkenyloxy mit 2 bis 10 Kohlenstoffatomen, Alkenyloxyalkyl mit 3 bis 10 Kohlenstoffatomen, Alkinyloxy mit 2 bis 10 Kohlenstoffatomen, Alkinyloxyalkyl mit 3 bis 10 Kohlenstoffatomen, Cycloalkoxy mit 3 bis 8 Kohlenstoffatomen, Alkylthio mit 1 bis 10 Kohlenstoffatomen, Alkylthioalkyl mit 2 bis 10 Kohlenstoffatomen, Acylamino mit 1 bis 7 Kohlenstoffatomen, Acylaminoalkyl mit 2 bis 8 Kohlenstoffatomen, Acyloxy mit 1 bis 6 Kohlenstoffatomen, Alkoxycarbonyl mit 2 bis 5 Kohlenstoffatomen, Cycloalkoxycarbonyl mit 4 bis 8 Kohlenstoffatomen, Alkylaminocarbonylamino mit 2 bis 4 Kohlenstoffatomen, Dialkylaminocarbonylamino mit 3 bis 7 Kohlenstoffatomen, (1-Pyrrolidino)-carbonylamino, Cycloalkylaminocarbonylamino mit 4 bis 8 Kohlenstoffatomen, Alkylaminocarbonylaminoalkyl mit 3 bis 9 Kohlenstoffatomen, Dialkylaminocarbonylaminoalkyl mit 4 bis 11 Kohlenstoffatomen, (1-Pyrrolidino)-carbonylaminoethyl, (1-Piperidino)-carbonylaminoethyl, Alkoxycarbonylaminoalkyl mit 3 bis 12 Kohlenstoffatomen, Cycloalkoxycarbonylaminoalkyl mit 5 bis 12 Kohlenstoffatomen, Carbamoylalkyl mit 2 bis 5 Kohlenstoffatomen, Alkylaminocarbonylalkyl mit 3 bis 9 Kohlenstoffatomen, Dialkylaminocarbonylalkyl mit 4 bis 11 Kohlenstoffatomen, (1-Pyrrolidino)-carbonylmethyl, (1-Piperidino)-carbonylmethyl, (1-Piperidino)-carbonylethyl, Cycloalkylaminocarbonylalkyl mit 5 bis 12 Kohlenstoffatomen, Alkylaminocarbonylalkoxy mit 3 bis 10 Kohlenstoffatomen, Dialkylaminocarbonylalkoxy mit 4 bis 10 Kohlenstoffatomen, (1-Piperidinyl)-carbonylmethoxy, Cycloalkylaminocarbonylalkoxy mit 5 bis 11 Kohlenstoffatomen, Cycloalkylaminocarbonylaminoalkyl mit 5 bis 12 Kohlenstoffatomen;
eine Gruppe Naphthyl oder Indenyl, deren eine oder mehrere Bindungen hydriert sein können, welche Gruppen unsubstituiert oder substituiert sein können einfach oder mehrfach durch ein Halogen, eine Gruppe Alkyl, Phenyl, Cyano, Hydroxyalkyl, Hydroxy, Oxo, Alkylcarbonylamino, Alkoxycarbonyl, Thioalkyl, wobei die Alkyle $C_1$-$C_4$ sind; eine Gruppe Pyridyl; Thiadiazolyl, Indolyl, Indazolyl, Imidazolyl, Benzimidazolyl, Chinolyl, Benzotriazol, Benzofuranyl, Benzothienyl, Benzothiazolyl, Benzoisothiazolyl, Isochinolyl, Benzoxazolyl, Benzoxazinyl, Benzodioxinyl, Isoxazolyl, Benzopyranyl, Thiazolyl, Thienyl, Furyl, Pyranyl, Chromenyl, Isobenzofuranyl, Pyrrolyl, Pyrazolyl, Pyrazinyl, Pyrimidinyl, Pyridazinyl, Indolizinyl, Phthalazinyl, Chinazolinyl, Acridinyl, Isothiazolyl, Isochromanyl, Chromanyl, deren eine oder mehrere Doppelbindungen hydriert sein können, welche Gruppen unsubstituiert oder substituiert sein können einfach oder mehrfach durch eine Gruppe Alkyl, Phenyl, Cyano, Hydroxyalkyl, Hydroxy, Alkylcarbonylamino, Alkoxycarbonyl, Thioalkyl, wobei die Alkyle $C_1$-$C_4$ sind; oder auch, wenn T -C=O- bedeutet, -$(CH_2)_q$-Z auch darstellen kann: eine Benzyl-Gruppe, substituiert an -CH- durch ein Hydroxyl, ein $C_1$-$C_4$-Alkoxy, ein $C_1$-$C_4$-Alkyl, und gegebenenfalls substituiert am aromatischen Cyclus durch ein Halogen, ein Trifluormethyl, ein $C_1$-$C_4$-Alkyl, ein Hydroxyl, ein $C_1$-$C_4$-Alkoxy; oder eines ihrer gegebenenfalls vorliegenden Salze mit Mineral-oder organischen Säuren, oder wenn

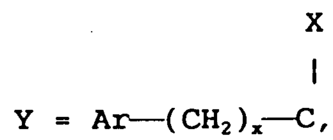

eines ihrer quaternären Ammoniumsalze mit Stickstoff (b) von Piperidin oder ein N-Oxid-Derivat mit demselben Stickstoffatom.

**2.** Verbindung nach Anspruch 1, in optisch reiner Form, der Formel:

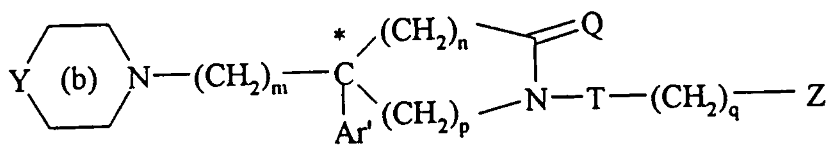

(I*),

worin

- "*" bedeutet, daß das so markierte Kohlenstoffatom die bestimmte absolute (+)- oder (-)-Konfiguration aufweist;
- Y, m, Ar', n, p, Q, T, q und Z wie in Anspruch 1 definiert sind; oder eines ihrer Salze mit Mineral- oder organischen Säuren oder mit dem Stickstoffatom (b) eines ihrer quaternären Ammoniumsalze oder ein N-Oxid-Derivat.

**3.** Verbindung nach einem der Ansprüche 1 oder 2, worin Z darstellt: eine Phenyl-Gruppe, unsubstituiert oder mono- oder disubstituiert durch ein Halogen oder ein Alkoxy; eine Naphthyl-Gruppe.

**4.** Verbindung nach einem der Ansprüche 1 bis 3 der Formel (I) oder (I*)

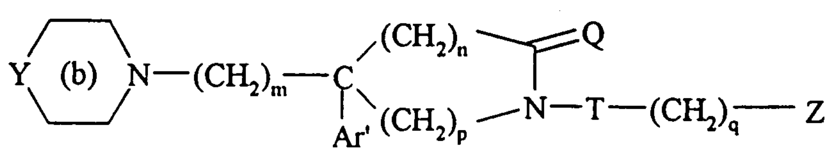

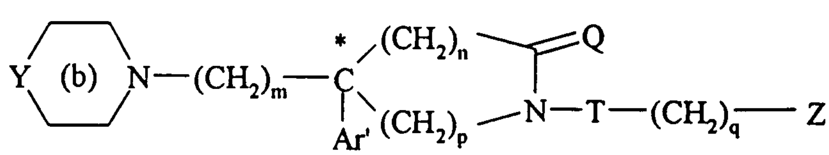

,

worin:

Y bedeutet: eine Gruppe

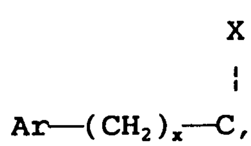

wobei Ar, x, und X wie in Anspruch 1 definiert sind;

- m, Ar', n, p, Q, T, q und Z wie in Anspruch 1 definiert sind;

in Form des quaternären Ammoniumsalzes oder N-Oxid-Derivats, dadurch gekennzeichnet, daß die Gruppe

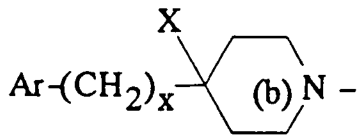

dann dargestellt wird durch die Gruppe

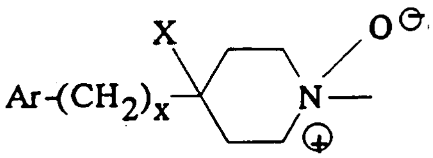 oder 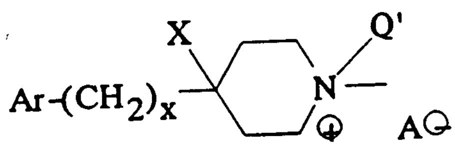 ,

worin:

- Q' eine $C_1$-$C_6$-Alkyl-Gruppe oder eine Benzyl-Gruppe bedeutet; und
- $A^-$ ein Anion darstellt, ausgewählt aus Chlorid, Bromid, Iodid, Acetat, Methansulfonat oder p-Toluolsulfonat.

**5.** Verfahren zur Herstellung von Verbindungen der Formel (I) nach Anspruch 1, dadurch gekennzeichnet, daß:

a) eine Verbindung der Formel:

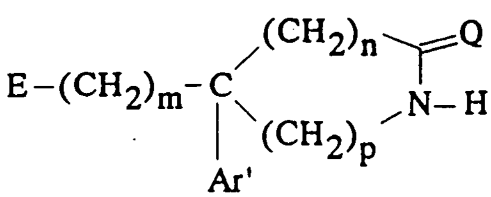 (II),

worin m, Ar', n, p und Q wie in Anspruch 1 definiert sind, und E darstellt: ein Hydroxyl oder gegebenenfalls eine O-geschützte Gruppe oder eine Gruppe:

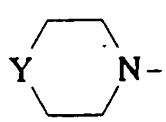 ,

worin Y wie in Anspruch 1 definiert ist; mit der Maßgabe, daß: wenn Y die Gruppe

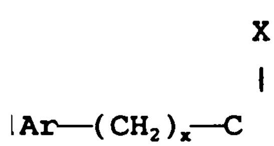

darstellt, wobei X ein Hydroxyl ist, dieses Hydroxyl geschützt werden kann; behandelt wird

- entweder mit einem funktionellen Derivat einer Säure der Formel:

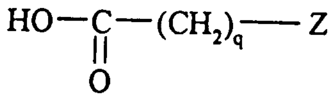

(III),

worin q und Z wie in Anspruch 1 definiert sind, wenn eine Verbindung der Formel (I) hergestellt werden soll, worin T die Bedeutung -CO- hat,

• oder mit einem halogenierten Derivat der Formel:

$$Hal\text{-}(CH_2)_{q+1}\text{-}Z \quad (IV),$$

worin q und Z wie vorstehend definiert sind, und worin Hal ein Halogen darstellt, wenn eine Verbindung der Formel (I) hergestellt werden soll , worin T die Bedeutung $-CH_2-$ hat, wobei die Verbindung der Formel:

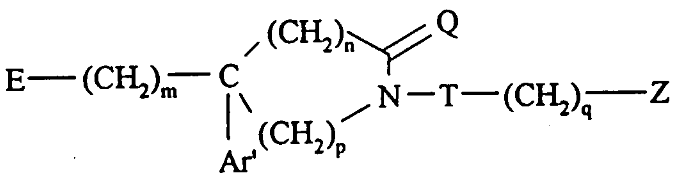

(V),

erhalten wird;

b) anschließend, wenn E eine O-geschützte Gruppe darstellt, die O-Schutzgruppe durch die Einwirkung einer Säure entfernt wird;

c) der so erhaltene Alkohol der Formel:

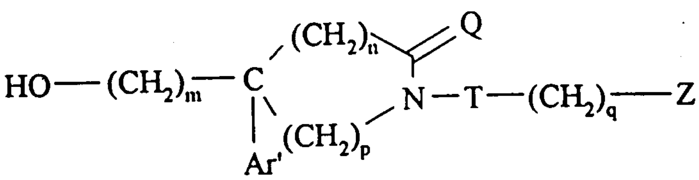

(VI)

mit Methansulfonylchlorid behandelt wird;

d) das so erhaltene Mesylat der Formel:

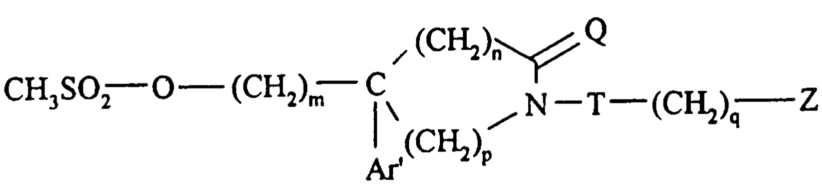

(VII)

umgesetzt wird mit einem sekundären Amin der Formel:

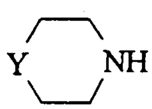

(VIII),

worin Y wie vorstehend definiert ist; und

e) nach dem gegebenenfalls durchgeführten Entschützen des durch X dargestellten Hydroxyls gegebenenfalls das so erhaltene Produkt in eines seiner Salze mit Mineral- oder organischen Säuren oder in eines seiner quaternären Ammoniumsalze mit Stickstoff (b) von Piperidin oder ein N-Oxid-Derivat mit diesem Stickstoffatom übergeführt wird.

**6.** Verbindung der Formel:

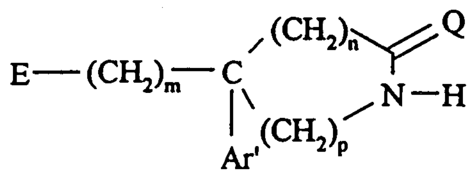

(II),

worin m, Ar', n, p und Q wie in Anspruch 1 definiert sind, und E darstellt: ein Hydroxyl oder gegebenenfalls eine O-geschützte Gruppe oder eine Gruppe:

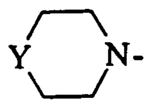

,

worin Y wie in Anspruch 1 definiert ist,

- mit der Maßgabe, daß Q Sauerstoff bedeutet:
  - wenn E = OH oder ($C_1$-$C_6$)Alkoxycarbonyloxy, m = 2 oder 3, Ar' ein durch ein ($C_1$-$C_4$)Alkoxy substituiertes Phenyl darstellt, n = 3, und p = 1;
  - wenn E = ($C_1$-$C_4$)Alkylcarbonyloxy, m = 2, n = 1, und Ar' bedeutet: ein Phenyl, unsubstituiert oder substituiert einfach oder mehrfach durch ein ($C_1$-$C_4$)Alkyl, ein ($C_1$-$C_4$)Alkoxy oder ein Halogen;
- mit der Maßgabe, daß, wenn E = OH oder ein ($C_1$-$C_4$)Alkoxy, m = 2, p = 1, Ar' darstellt: ein Phenyl, unsubstituiert oder substituiert einfach oder mehrfach durch ein ($C_1$-$C_4$)Alkyl, ein ($C_1$-$C_4$)Alkoxy oder ein Halogen, n von 1 verschieden ist;
- mit der Maßgabe, daß, wenn E = OH, Q = 2H, m = 2, n = 1, Ar' von einem unsubstituierten Phenyl verschieden ist; oder eines ihrer Salze mit Mineral- oder organischen Säuren.

**7.** Verbindung der Formel:

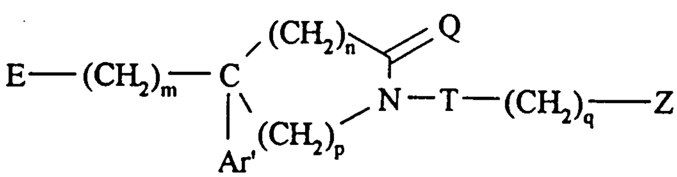

(V),

worin E ein Hydroxyl, eine O-geschützte Gruppe bedeutet, und m, Ar', n, p, Q, T und Z wie in Anspruch 1 definiert sind,

- mit der Maßgabe, daß, wenn E = OH, Q = 2H, m = 2, n = 1, p = 2, T = -$CH_2$-, q = Null, und Z = Phenyl, Ar' von einem unsubstituierten Phenyl verschieden ist; und
- mit der Maßgabe, daß, wenn E = OH, Q = 2H, m = 2, n = 1, p = 1, T = -$CH_2$-, q = Null, und Z = Phenyl, Ar' von einem Phenyl verschieden ist, unsubstituiert oder substituiert einfach oder mehrfach durch ein ($C_1$-$C_4$)Alkyl, ein ($C_1$-$C_4$)Alkoxy oder ein Halogen; und
- mit der Maßgabe, daß, wenn E = OH oder ($C_1$-$C_6$)Alkylcarbonyloxy, Q = 2H, m = 2 oder 3, n = 3, p = 1, T = -$CH_2$-, q = Null, 1, 2 oder 3, und Z eine Aryl-Gruppe bedeutet, Ar' von einem durch ein ($C_1$-$C_4$)Alkoxy substituierten Phenyl verschieden ist; oder eines ihrer Salze mit Mineral- oder organischen Säuren.

**8.** Verbindung nach Anspruch 7 der Formel:

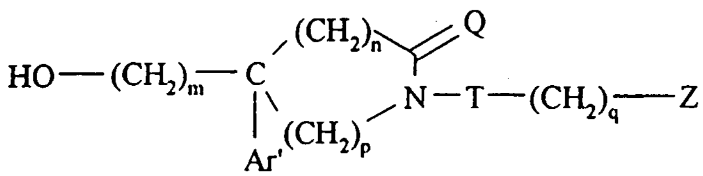

(VI),

worin m, Ar', Q, n, p, T, q und Z wie in Anspruch 1 definiert sind,

- mit der Maßgabe, daß, wenn Q = 2H, m = 2, n = 1, p = 2, T = $-CH_2-$, q = Null, und Z = Phenyl, Ar' von einem unsubstituierten Phenyl verschieden ist; und
- mit der Maßgabe, daß, wenn Q = 2H, m = 2, n = 1, p = 1, T = $-CH_2-$, q = Null, und Z = Phenyl, Ar' von einem Phenyl verschieden ist, unsubstituiert oder substituiert einfach oder mehrfach durch ein $(C_1-C_4)$Alkyl, ein $(C_1-C_4)$Alkoxy oder ein Halogen; und
- mit der Maßgabe, daß, wenn Q = 2H, m = 2 oder 3, n = 3, p = 1, T = $-CH_2-$, q = Null, 1, 2 oder 3, und Z eine Aryl-Gruppe bedeutet, Ar' von einem durch ein $(C_1-C_4)$Alkoxy substituierten Phenyl verschieden ist; oder eines ihrer Salze mit Mineral- oder organischen Säuren.

**9.** Verbindung der Formel:

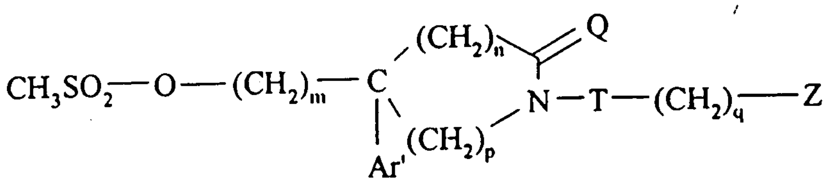

(VII),

worin m, Ar', n, p, Q, T, q und Z wie in Anspruch 1 definiert sind, oder eines ihrer Salze mit Mineral- oder organischen Säuren.

**10.** Verbindung der Formel:

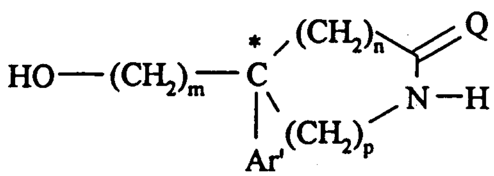

(II*),

worin m, Ar', n und p wie in Anspruch 1 definiert sind, und Q 2 Wasserstoffatome bedeutet, welche Verbindung in optisch reiner Form vorliegt, mit der Maßgabe, daß, wenn m = 2 oder 3, n = 3, und p = 1, Ar' von einem durch ein $C_1-C_4$-Alkoxy substituierten Phenyl verschieden ist.

**11.** Verbindung der Formel:

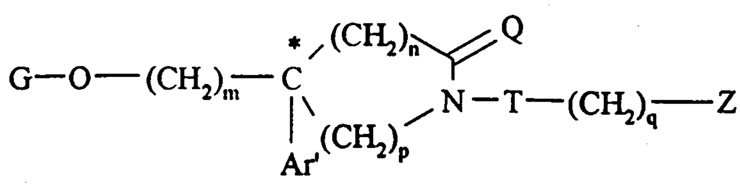

(V*),

worin G Wasserstoff oder eine Methansulfonyl-Gruppe bedeutet, m, Ar', n, p, q, T und Z wie in Anspruch 1 definiert sind, und Q 2 Wasserstoffatome darstellt, welche Verbindung in optisch reiner Form vorliegt, mit der Maßgabe, daß, wenn G Wasserstoff ist, m = 2 oder 3, n = 3, p = 1, und Z eine Aryl-Gruppe bedeutet, Ar' von einem durch ein $C_1$-$C_4$-Alkoxy substituierten Phenyl verschieden ist.

**12.** Pharmazeutische Zusammensetzung, welche als aktiven Bestandteil eine Verbindung nach einem der Ansprüche 1 bis 4 oder eines ihrer pharmazeutisch annehmbaren Salze enthält.

**13.** Pharmazeutische Zusammensetzung nach Anspruch 12, in Einheitsdosierungsform, worin der aktiven Bestandteil mit zumindest einem pharmazeutischen Exzipienten gemischt ist.

**14.** Zusammensetzung nach Anspruch 12, welche 2,5 bis 1000 mg aktiven Bestandteil enthält.

**15.** Verbindungen nach einem der Ansprüche 1 bis 4, worin Ar' und/oder Z eine Phenyl-Gruppe, substituiert einfach oder mehrfach durch ein Chlor- oder Fluoratom, darstellen bzw. darstellt.

**16.** Verfahren nach Anspruch 5, dadurch gekennzeichnet, daß in der Verbindung der Formel (II) E eine Tetrahydropyran-2-yloxy-Gruppe bedeutet.

**17.** Verbindung nach einem der Ansprüche 6 oder 7, worin E eine Tetrahydropyran-2-yloxy-Gruppe bedeutet.

**18.** Verbindung der Formel (II) nach Anspruch 6, ausgewählt aus:

- 5-Tetrahydropyranyloxypropyl-5-(3,4-dichlorphenyl)-piperidon,
- 3-Tetrahydropyranyloxypropyl-3-(3,4-dichlorphenyl)-piperidin, und
- 3(2-Hydroxyethyl)-3-(3,4-dichlorphenyl)-piperidin.

**19.** Optisch reine Verbindung der Formel (II)* nach Anspruch 10, ausgewählt aus:

- (+)-3-(2-Hydroxyethyl)-3-(3,4-dichlorphenyl)-piperidin, und
- (-)-3-(2-Hydroxyethyl)-3-(3,4-dichlorphenyl)-piperidin.

**Patentansprüche für folgenden Vertragsstaat: ES**

**1.** Verfahren zur Herstellung von Verbindungen der Formel (I):

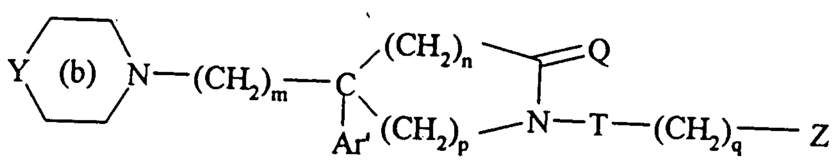

(I),

worin

- Y bedeutet:

  - entweder eine Gruppe Cy-N oder Cy-$CH_2$-N, wobei

    • Cy darstellt: ein Phenyl, unsubstituiert oder substituiert einfach oder mehrfach durch einen Substituenten, ausgewählt aus: einem Halogenatom, einem Hydroxyl, einem $C_1$-$C_4$-Alkoxy, einem $C_1$-$C_4$-Alkyl, einem Trifluormethyl, welche Substituenten gleich oder verschieden sind; eine $C_3$-$C_7$-Cycloalkyl-Gruppe; eine Pyrimidyl-Gruppe oder eine Pyridyl-Gruppe;

  - oder eine Gruppe

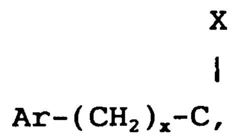

wobei

- Ar bedeutet: ein Phenyl, unsubstituiert oder substituiert einfach oder mehrfach durch einen Substituenten, ausgewählt aus: einem Halogenatom, einem Hydroxyl, einem $C_1$-$C_4$-Alkoxy, einem Trifluormethyl, einem $C_1$-$C_4$-Alkyl, welche Substituenten gleich oder verschieden sind; eine Pyridyl-Gruppe; oder Thienyl-Gruppe;
- x Null oder 1 ist;
- X darstellt: einen Wasserstoff, ein Hydroxyl, ein $C_1$-$C_4$-Alkoxy; ein $C_1$-$C_4$-Acyloxy; ein Carboxy; ein $C_1$-$C_4$-Carbalkoxy; ein Cyano; eine Gruppe $-N(X_1)_2$, wobei die Gruppen $X_1$ unabhängig bedeuten: Wasserstoff, ein $C_1$-$C_4$-Alkyl, ein $C_1$-$C_4$-Hydroxyalkyl, ein $C_1$-$C_4$-Acyl, oder auch $-(X_1)_2$ mit dem Stickstoffatom, an das es gebunden ist, einen Heterocyclus bildet, ausgewählt aus Pyrrolidin, Piperidin oder Morpholin; eine Gruppe $-S-X_2$, wobei $X_2$ Wasserstoff oder eine $C_1$-$C_4$-Alkyl-Gruppe ist;

oder auch X mit dem Kohlenstoffatom, an das es gebunden ist, und mit dem benachbarten Kohlenstoffatom im Heterocyclus eine Doppelbindung bildet;
- m 2 oder 3 ist;
- Ar' bedeutet: ein Phenyl, unsubstituiert oder substituiert einfach oder mehrfach durch einen Substituenten, ausgewählt aus: einem Halogenatom, einem Trifluormethyl, einem $C_1$-$C_4$-Alkoxy, einem $C_1$-$C_4$-Alkyl, welche Substituenten gleich oder verschieden sind; ein Thienyl; ein Benzothienyl; ein Naphthyl oder ein Indolyl;
- n Null, 1, 2 oder 3 ist;
- p 1 oder 2 ist, und wenn p 2 ist, n 1 ist, und Q zwei Wasserstoffatome darstellt;
- Q Sauerstoff oder zwei Wasserstoffatome bedeutet;
- T eine Gruppe ist, ausgewählt aus

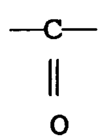 und $—CH_2—$ ;

- q Null, 1, 2 oder 3 ist;
- Z darstellt: ein Phenyl, unsubstituiert oder substituiert durch einen oder mehrere Substituenten, ausgewählt aus: Halogen, CN, OH, $NH_2$, NH-CO-$NH_2$, $NO_2$, $CONH_2$, $CF_3$, $C_1$-$C_{10}$-Alkyl, Alkenyl mit 2 bis 10 Kohlenstoffatomen, Alkinyl mit 2 bis 10 Kohlenstoffatomen, Cycloalkyl mit 3 bis 8 Kohlenstoffatomen, Bicycloalkyl mit 4 bis 11 Kohlenstoffatomen, Hydroxyalkyl mit 1 bis 5 Kohlenstoffatomen, Alkoxy mit 1 bis 10 Kohlenstoffatomen, Alkoxyalkyl mit 2 bis 10 Kohlenstoffatomen, Alkoxyalkoxyalkyl mit 3 bis 10 Kohlenstoffatomen, Alkoxyalkoxy mit 2 bis 10 Kohlenstoffatomen, Alkenyloxy mit 2 bis 10 Kohlenstoffatomen, Alkenyloxyalkyl mit 3 bis 10 Kohlenstoffatomen, Alkinyloxy mit 2 bis 10 Kohlenstoffatomen, Alkinyloxyalkyl mit 3 bis 10 Kohlenstoffatomen, Cycloalkoxy mit 3 bis 8 Kohlenstoffatomen, Alkylthio mit 1 bis 10 Kohlenstoffatomen, Alkylthioalkyl mit 2 bis 10 Kohlenstoffatomen, Acylamino mit 1 bis 7 Kohlenstoffatomen, Acylaminoalkyl mit 2 bis 8 Kohlenstoffatomen, Acyloxy mit 1 bis 6 Kohlenstoffatomen, Alkoxycarbonyl mit 2 bis 5 Kohlenstoffatomen, Cycloalkoxycarbonyl mit 4 bis 8 Kohlenstoffatomen, Alkylaminocarbonylamino mit 2 bis 4 Kohlenstoffatomen, Dialkylaminocarbonylamino mit 3 bis 7 Kohlenstoffatomen, (1-Pyrrolidino)-carbonylamino, Cycloalkylaminocarbonylamino mit 4 bis 8 Kohlenstoffatomen, Alkylaminocarbonylaminoalkyl mit 3 bis 9 Kohlenstoffatomen, Dialkylaminocarbonylaminoalkyl mit 4 bis 11 Kohlenstoffatomen, (1-Pyrrolidino)-carbonylaminoethyl, (1-Piperidino)-carbonylaminoethyl, Alkoxycarbonylaminoalkyl mit 3 bis 12 Kohlenstoffatomen, Cycloalkoxycarbonylaminoalkyl mit 5 bis 12 Kohlenstoffatomen, Carbamoylalkyl mit 2 bis 5 Kohlenstoffatomen, Alkylaminocarbonylalkyl mit 3 bis 9 Kohlenstoffatomen, Dialkylaminocarbonylalkyl mit 4 bis 11 Kohlenstoffatomen, (1-Pyrrolidino)-carbonylmethyl, (1-Piperidino)-carbonylmethyl, (1-Piperidino)-carbonylethyl, Cycloalkylaminocarbonylalkyl mit 5 bis 12 Kohlenstoffatomen, Alkylaminocarbonylalkoxy mit 3 bis 10 Kohlenstoffatomen, Dialkylaminocarbonylalkoxy mit 4 bis

10 Kohlenstoffatomen, (1-Piperidinyl)-carbonylmethoxy, Cycloalkylaminocarbonylalkoxy mit 5 bis 11 Kohlenstoffatomen, Cycloalkylaminocarbonylaminoalkyl mit 5 bis 12 Kohlenstoffatomen;
eine Gruppe Naphthyl oder Indenyl, deren eine oder mehrere Bindungen hydriert sein können, welche Gruppen unsubstituiert oder substituiert sein können einfach oder mehrfach durch ein Halogen, eine Gruppe Alkyl, Phenyl, Cyano, Hydroxyalkyl, Hydroxy, Oxo, Alkylcarbonylamino, Alkoxycarbonyl, Thioalkyl, wobei die Alkyle $C_1$-$C_4$ sind; eine Gruppe Pyridyl; Thiadiazolyl, Indolyl, Indazolyl, Imidazolyl, Benzimidazolyl, Chinolyl, Benzotriazol, Benzofuranyl, Benzothienyl, Benzothiazolyl, Benzoisothiazolyl, Isochinolyl, Benzoxazolyl, Benzoxazinyl, Benzodioxinyl, Isoxazolyl, Benzopyranyl, Thiazolyl, Thienyl, Furyl, Pyranyl, Chromenyl, Isobenzofuranyl, Pyrrolyl, Pyrazolyl, Pyrazinyl, Pyrimidinyl, Pyridazinyl, Indolizinyl, Phthalazinyl, Chinazolinyl, Acridinyl, Isothiazolyl, Isochromanyl, Chromanyl, deren eine oder mehrere Doppelbindungen hydriert sein können, welche Gruppen unsubstituiert oder substituiert sein können einfach oder mehrfach durch eine Gruppe Alkyl, Phenyl, Cyano, Hydroxyalkyl, Hydroxy, Alkylcarbonylamino, Alkoxycarbonyl, Thioalkyl, wobei die Alkyle $C_1$-$C_4$ sind; oder auch, wenn T -C=O- bedeutet, $-(CH_2)_q$-Z auch darstellen kann: eine Benzyl-Gruppe, substituiert an -CH- durch ein Hydroxyl, ein $C_1$-$C_4$-Alkoxy, ein $C_1$-$C_4$-Alkyl, und gegebenenfalls substituiert am aromatischen Cyclus durch ein Halogen, ein Trifluormethyl, ein $C_1$-$C_4$-Alkyl ein Hydroxyl, ein $C_1$-$C_4$-Alkoxy;

oder eines ihrer gegebenenfalls vorliegenden Salze mit Mineral-oder organischen Säuren, oder wenn

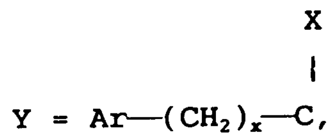

ihrer quaternären Ammoniumsalze mit Stickstoff (b) von Piperidin oder ein N-Oxid-Derivat mit demselben Stickstoffatom; dadurch gekennzeichnet, daß

a) eine Verbindung der Formel:

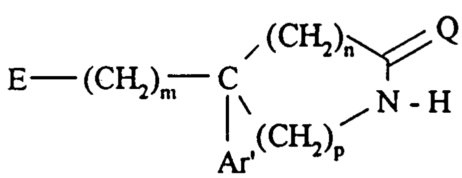

(II),

worin m, Ar', n, p und Q wie vorstehend definiert sind, und E darstellt: ein Hydroxyl oder gegebenenfalls eine O-geschützte Gruppe oder eine Gruppe:

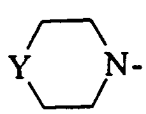

,

worin Y wie vorstehend definiert ist; mit der Maßgabe, daß: wenn Y die Gruppe

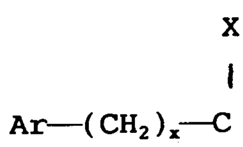

darstellt, wobei X ein Hydroxyl ist, dieses Hydroxyl geschützt werden kann; behandelt wird

- entweder mit einem funktionellen Derivat einer Säure der Formel

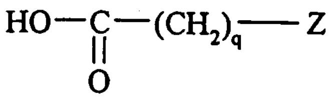

(III),

worin q und Z wie vorstehend definiert sind, wenn eine Verbindung der Formel (I) hergestellt werden soll, worin T die Bedeutung -CO- hat,

• oder mit einem halogenierten Derivat der Formel:

$$Hal\text{-}(CH_2)_{q+1}\text{-}Z \quad (IV),$$

worin q und Z wie vorstehend definiert sind, und worin Hal ein Halogen darstellt, wenn eine Verbindung der Formel (I) hergestellt werden soll, worin T die Bedeutung $-CH_2-$ hat, wobei die Verbindung der Formel:

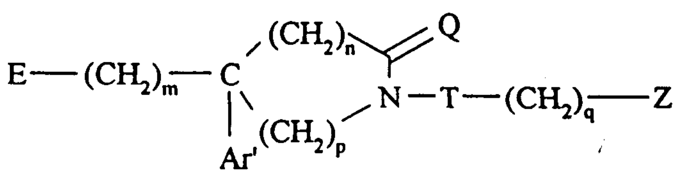

(V),

erhalten wird;

b) anschließend, wenn E eine O-geschützte Gruppe darstellt, die O-Schutzgruppe durch die Einwirkung einer Säure entfernt wird;

c) der so erhaltene Alkohol der Formel:

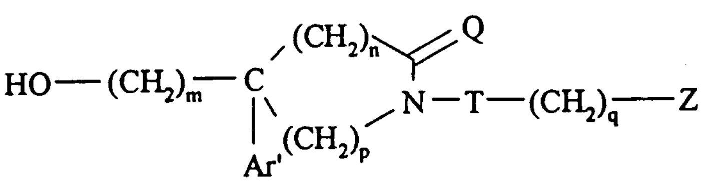

(VI)

mit Methansulfonylchlorid behandelt wird;

d) das so erhaltene Mesylat der Formel:

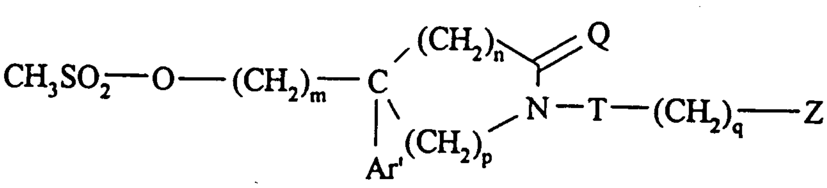

(VII)

umgesetzt wird mit einem sekundären Amin der Formel:

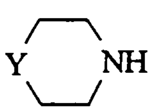

(VIII),

worin Y wie vorstehend definiert ist; und

e) nach dem gegebenenfalls durchgeführten Entschützen des durch X dargestellten Hydroxyls gegebenenfalls das so erhaltene Produkt in eines seiner Salze mit Mineral- oder organischen Säuren oder in eines seiner quaternären Ammoniumsalze mit Stickstoff (b) von Piperidin oder ein N-Oxid-Derivat mit diesem Stickstoffatom übergeführt wird.

2. Verfahren zur Herstellung optisch reiner Verbindungen der Formel:

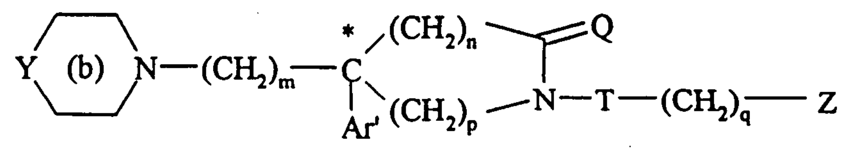

(I*),

worin

- "*" bedeutet, daß das so markierte Kohlenstoffatom die bestimmte absolute (+)- oder (-)-Konfiguration aufweist;
- Y, m, Ar', n, p, Q, T, q und Z wie in Anspruch 1 definiert sind; oder eines ihrer Salze mit Mineral- oder organischen Säuren oder mit dem Stickstoffatom (b) eines ihrer quaternären Ammoniumsalze oder eines N-Oxid-Derivats; dadurch gekennzeichnet, daß diese Verbindungen durch Trennung racemischer Mischungen der Verbindungen der Formel (I), die gemäß dem Verfahren nach Anspruch 1 erhalten werden, durch Kristallisation oder durch Chromatographie isoliert werden, und gegebenenfalls in eines ihrer Salze übergeführt werden.

3. Verfahren zur Herstellung optisch reiner Verbindungen der Formel:

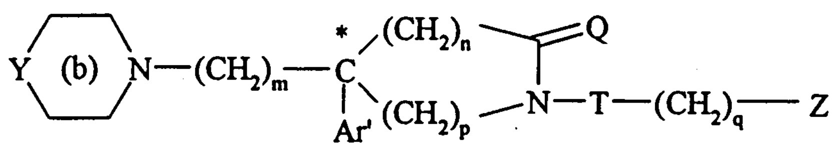

(I*),

worin

- "*" bedeutet, daß das so markierte Kohlenstoffatom die bestimmte absolute (+)- oder (-)-Konfiguration aufweist;
- Y, m, Ar', n, p, Q, T, q und Z wie in Anspruch 1 definiert sind;

dadurch gekennzeichnet, daß:

a) eine Verbindung der Formel

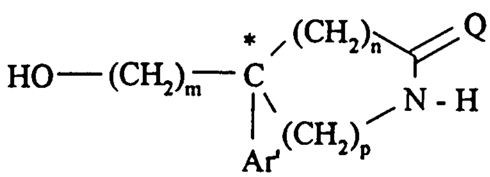

(II*, Q = H),

worin m, Ar', n, p und Q wie oben definiert sind; behandelt wird

• entweder mit einem funktionellen Derivat einer Säure der Formel

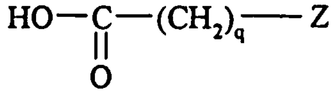

(III),

worin q und Z wie vorstehend definiert sind, wenn eine Verbindung der Formel (I*) hergestellt werden soll, worin T die Bedeutung -CO- hat,

• oder mit einem halogenierten Derivat der Formel:

$$Hal\text{-}(CH_2)_{q+1}\text{-}Z \quad (IV),$$

worin q und Z wie vorstehend definiert sind, und worin Hal ein Halogen und vorzugsweise ein Brom- oder Chloratom darstellt, wenn eine Verbindung der Formel (I*) hergestellt werden soll, worin T die Bedeutung $-CH_2-$ hat, wobei die Verbindung der Formel:

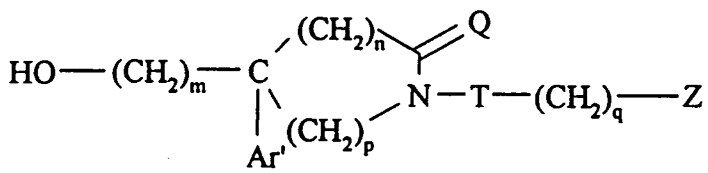

(VI)

erhalten wird;

b) der Alkohol der Formel (VI*) mit Methansulfonylchlorid behandelt wird;
c) das so erhaltene Mesylat der Formel:

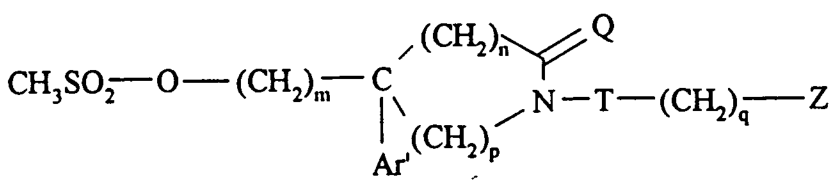

(VII)

umgesetzt wird mit einem sekundären Amin der Formel:

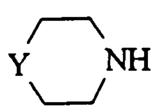

(VIII),

worin Y wie vorstehend definiert ist; und
d) gegebenenfalls das so erhaltene Produkt in eines seiner Salze mit Mineral- oder organischen Säuren oder in eines seiner quaternären Ammoniumsalze mit Stickstoff (b) von Piperidin oder ein N-Oxid-Derivat mit diesem Stickstoffatom übergeführt wird.

**4.** Verfahren nach einem der Ansprüche 1 bis 3, dadurch gekennzeichnet, daß zur Überführung der Verbindungen (I) oder (I*) in eines ihrer quaternären Ammoniumsalze die freien Basen der Verbindungen der Formeln (I) oder (I*), worin

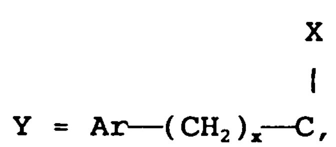

für welche die gegebenenfalls vorliegenden anderen Amin-Funktionen durch eine übliche N-Schutzgruppe N-geschützt werden, umgesetzt werden mit einem Überschuß eines Alkylierungsmittels der Formel:

$$A\text{-}Q',$$

worin A eine Abgangsgruppe ist und wie nachstehend definiert ist, und Q' wie für (I) oder (I*) definiert ist, und die Reaktionsmischung in einem Lösungsmittel, beispielsweise ausgewählt aus Dichlormethan, Chloroform, Aceton oder Acetonitril, auf eine Temperatur zwischen Umgebungstemperatur und der Rückflußtemperatur während einer

bis mehrerer Stunden erhitzt wird, um nach der Behandlung gemäß üblichen Verfahren und nach gegebenenfalls durchgeführtem Entschützen eine Mischung von axialen und äquatorialen Diastereoisomeren von quaternären Ammoniumsalzen der Verbindungen der Formeln (I) oder (I*) zu erhalten, worin: die Gruppe

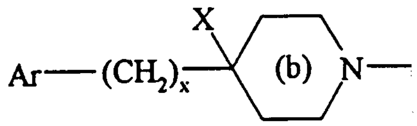

dann dargestellt wird durch die Gruppe

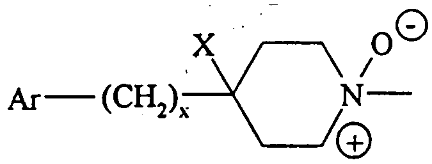

oder

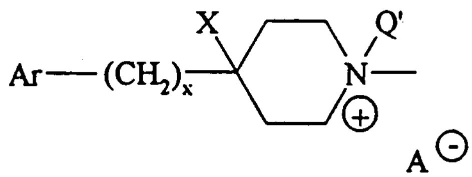

,

worin:

- Q' eine $C_1$-$C_6$-Alkyl-Gruppe oder eine Benzyl-Gruppe bedeutet; und
- A⁻ ein Anion darstellt, ausgewählt aus Chlorid, Bromid, Iodid, Acetat, Methansulfonat oder p-Toluolsulfonat.

**5.** Verfahren nach einem der Ansprüche 1 bis 3, dadurch gekennzeichnet, daß zur Überführung der Verbindungen (I) oder (I*) in N-Oxid-Derivate Verbindungen der Formeln (I) oder (I*), worin

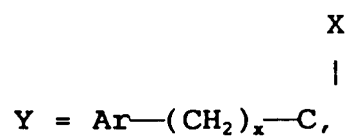

umgesetzt werden mit einem Peroxid-Derivat, beispielsweise m-Chlorperbenzoesäure oder oxygeniertem Wasser, gemäß üblichen Verfahren, um N-Oxid-Derivate der Verbindungen (I) oder (I*) zu erhalten, worin: die Gruppe

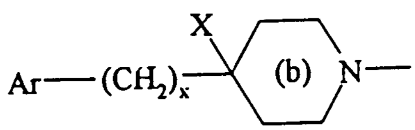

dann dargestellt wird durch die Gruppe

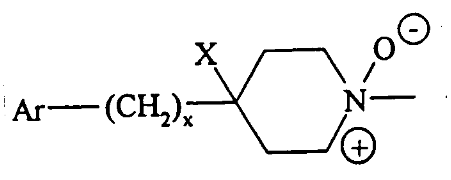

oder

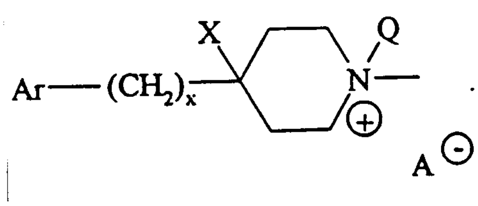

.

**6.** Verfahren zur Herstellung von Verbindungen der Formel:

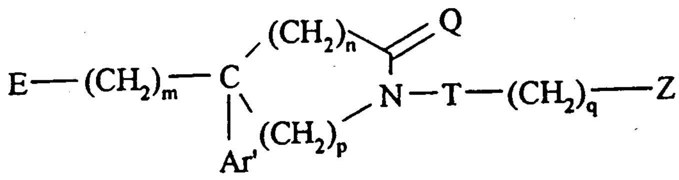 (V),

worin E ein Hydroxyl, eine O-geschützte Gruppe bedeutet, und m, Ar', n, p, Q, T und Z wie in Anspruch 1 definiert sind,

- mit der Maßgabe, daß, wenn E = OH, Q = 2H, m = 2, n = 1, p = 2, T = $-CH_2-$, q = Null, und Z = Phenyl, Ar' von einem unsubstituierten Phenyl verschieden ist; und
- mit der Maßgabe, daß, wenn E = OH, Q = 2H, m = 2, n = 1, p = 1, T = $-CH_2-$, q = Null, und Z = Phenyl, Ar' von einem Phenyl verschieden ist, unsubstituiert oder substituiert einfach oder mehrfach durch ein $(C_1-C_4)$Alkyl, ein $(C_1-C_4)$Alkoxy oder ein Halogen; und
- mit der Maßgabe, daß, wenn E = OH oder $(C_1-C_6)$Alkylcarbonyloxy, Q = 2H, m = 2 oder 3, n = 3, p = 1, T = $-CH_2-$, q = Null, 1, 2 oder 3, und Z eine Aryl-Gruppe bedeutet, Ar' von einem durch ein $(C_1-C_4)$Alkoxy substituierten Phenyl verschieden ist;

oder eines ihrer Salze mit Mineral- oder organischen Säuren, dadurch gekennzeichnet, daß:

a) eine Verbindung der Formel

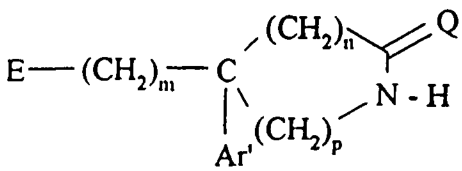 (II),

worin m, Ar', n, p und Q wie vorstehend definiert sind, und E darstellt: ein Hydroxyl oder gegebenenfalls eine O-geschützte Gruppe oder eine Gruppe:

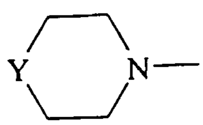 ,

worin Y wie vorstehend definiert ist; mit der Maßgabe, daß: wenn Y die Gruppe

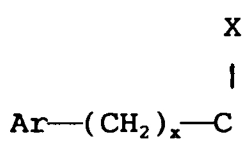

darstellt, wobei X ein Hydroxyl ist, dieses Hydroxyl geschützt werden kann; behandelt wird

- entweder mit einem funktionellen Derivat einer Säure der Formel

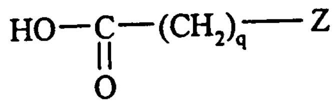

(III),

worin q und Z wie vorstehend definiert sind, wenn eine Verbindung der Formel (V) hergestellt werden soll, worin T die Bedeutung -CO- hat,

- oder mit einem halogenierten Derivat der Formel:

$$Hal\text{-}(CH_2)_{q+1}\text{-}Z \qquad (IV),$$

worin q und Z wie vorstehend definiert sind, und worin Hal ein Halogen darstellt, wenn eine Verbindung der Formel (I) hergestellt werden soll, worin T die Bedeutung $-CH_2-$ hat, wobei die Verbindung der Formel (V) erhalten wird; und

b) nach dem gegebenenfalls durchgeführten Entschützen des durch X dargestellten Hydroxyls gegebenenfalls das so erhaltene Produkt in eines seiner Salze mit Mineral- oder organischen Säuren übergeführt wird.

**7.** Verfahren zur Herstellung von Verbindungen der Formel (VI):

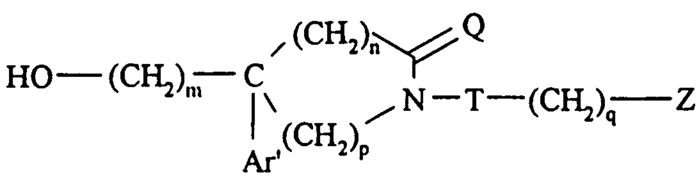

(VI),

worin m, Ar', Q, n, p, T, q und Z wie in Anspruch 1 definiert sind,

- mit der Maßgabe, daß, wenn Q = 2H, m = 2, n = 1, p = 2, T = $-CH_2-$, q = Null, und Z = Phenyl, Ar' von einem unsubstituierten Phenyl verschieden ist; und
- mit der Maßgabe, daß, wenn Q = 2H, m = 2, n = 1, p = 1, T = $-CH_2-$, q = Null, und Z = Phenyl, Ar' von einem Phenyl verschieden ist, unsubstituiert oder substituiert einfach oder mehrfach durch ein $(C_1-C_4)$Alkyl, ein $(C_1-C_4)$Alkoxy oder ein Halogen; und
- mit der Maßgabe, daß, wenn Q = 2H, m = 2 oder 3, n = 3, p = 1, T = $-CH_2-$, q = Null, 1, 2 oder 3, und Z eine Aryl-Gruppe bedeutet, Ar' von einem durch ein $(C_1-C_4)$Alkoxy substituierten Phenyl verschieden ist;

oder eines ihrer Salze mit Mineral- oder organischen Säuren; dadurch gekennzeichnet, daß:

a) eine Verbindung der Formel:

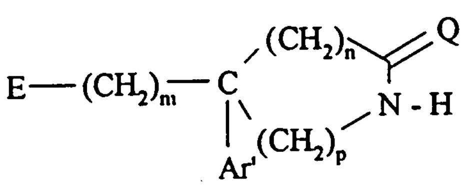

(II),

worin m, Ar', n, p und Q wie vorstehend definiert sind, und E darstellt: ein Hydroxyl oder gegebenenfalls eine O-geschützte Gruppe oder eine Gruppe:

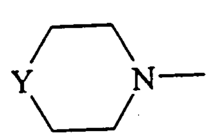

,

worin Y wie vorstehend definiert ist; mit der Maßgabe, daß: wenn Y die Gruppe

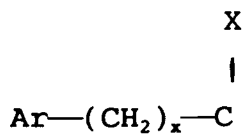

darstellt, wobei X ein Hydroxyl ist, dieses Hydroxyl geschützt werden kann; behandelt wird

- entweder mit einem funktionellen Derivat einer Säure der Formel

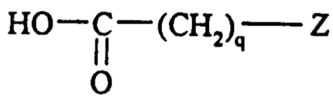 (III),

worin q und Z wie vorstehend definiert sind, wenn eine Verbindung der Formel (VI) hergestellt werden soll, worin T die Bedeutung -CO- hat,
- oder mit einem halogenierten Derivat der Formel:

$$Hal\text{-}(CH_2)_{q+1}\text{-}Z \qquad (IV),$$

worin q und Z wie vorstehend definiert sind, und worin Hal ein Halogen darstellt, wenn eine Verbindung der Formel (VI) hergestellt werden soll, worin T die Bedeutung $-CH_2-$ hat, wobei die Verbindung der Formel:

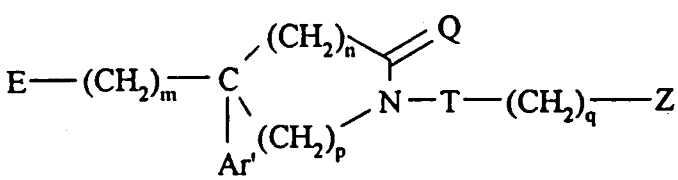 (V),

erhalten wird;

b) anschließend, wenn E eine O-geschützte Gruppe darstellt, die O-Schutzgruppe durch die Einwirkung einer Säure entfernt wird;
c) nach gegebenenfalls durchgeführtem Entschützen des durch X dargestellten Hydroxyls gegebenenfalls das so erhaltene Produkt in eines seiner Salze mit Mineral- oder organischen Säuren übergeführt wird.

**8.** Verfahren zur Herstellung von Verbindungen der Formel (VII):

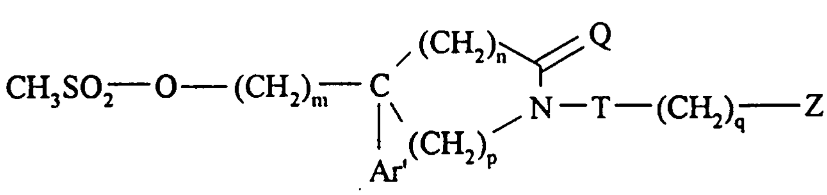 (VII),

worin m, Ar', n, p, Q, T, q und Z wie in Anspruch 1 definiert sind, oder eines ihrer Salze mit Mineral- oder organischen Säuren;
dadurch gekennzeichnet, daß

a) eine Verbindung der Formel:

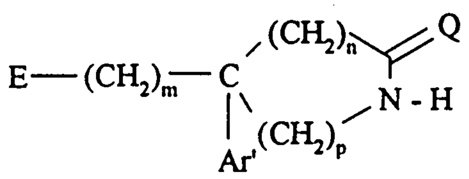

(II),

worin m, Ar', n, p und Q wie vorstehend definiert sind, und E darstellt: ein Hydroxyl oder gegebenenfalls eine O-geschützte Gruppe oder eine Gruppe:

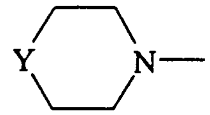

,

worin Y wie vorstehend definiert ist; mit der Maßgabe, daß: wenn Y die Gruppe

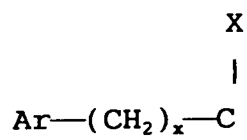

darstellt, wobei X ein Hydroxyl ist, dieses Hydroxyl geschützt werden kann; behandelt wird

- entweder mit einem funktionellen Derivat einer Säure der Formel

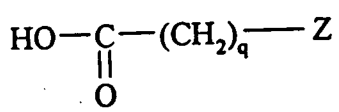

(III),

worin q und Z wie vorstehend definiert sind, wenn eine Verbindung der Formel (VII) hergestellt werden soll, worin T die Bedeutung -CO- hat,

- oder mit einem halogenierten Derivat der Formel:

$$Hal\text{-}(CH_2)_{q+1}\text{-}Z \quad (IV),$$

worin q und Z wie vorstehend definiert sind, und worin Hal ein Halogen darstellt, wenn eine Verbindung der Formel (VII) hergestellt werden soll, worin T die Bedeutung $-CH_2-$ hat, wobei die Verbindung der Formel:

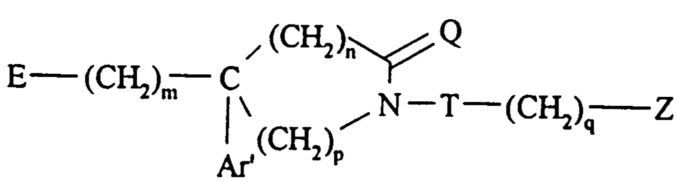

(V),

erhalten wird;

b) anschließend, wenn E eine O-geschützte Gruppe darstellt, die O-Schutzgruppe durch die Einwirkung einer

Säure entfernt wird;

c) der so erhaltene Alkohol der Formel:

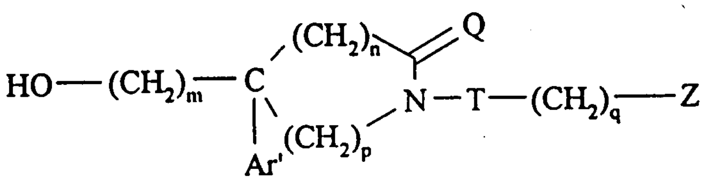

(VI)

mit Methansulfonylchlorid behandelt wird; und

d) nach gegebenenfalls durchgeführtem Entschützen des durch X dargestellten Hydroxyls gegebenenfalls das so erhaltene Produkt in eines seiner Salze mit Mineral- oder organischen Säuren übergeführt wird.

**9.** Verfahren zur Herstellung von Verbindungen der Formel (II*):

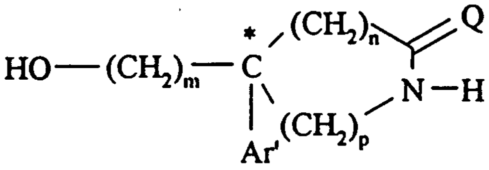

(II*),

worin

- "*" bedeutet, daß das so markierte Kohlenstoffatom die bestimmte absolute (+)- oder (-)-Konfiguration aufweist;
- m, Ar', n und p wie in Anspruch 1 definiert sind;
- Q 2 Wasserstoffatome bedeutet; oder eines ihrer Salze mit Mineral- oder organischen Säuren, dadurch gekennzeichnet, daß diese Verbindungen durch Trennung racemischer Mischungen der Verbindungen der Formel (II), durch Kristallisation oder durch Chromatographie isoliert werden, und gegebenenfalls in eines ihrer Salze übergeführt werden. 10. Verfahren zur Herstellung von Verbindungen der Formel:

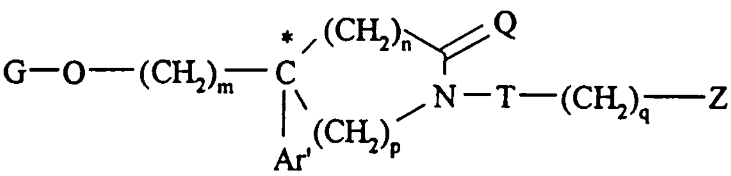

(V*, Q = H),

worin

- "*" bedeutet, daß das so markierte Kohlenstoffatom die bestimmte absolute (+)- oder (-)-Konfiguration aufweist;
- m, Ar', n und p wie in Anspruch 1 definiert sind;
- Q Wasserstoff darstellt;
- G Wasserstoff oder eine Methansulfonyl-Gruppe bedeutet;

dadurch gekennzeichnet, daß:

a) eine Verbindung der Formel:

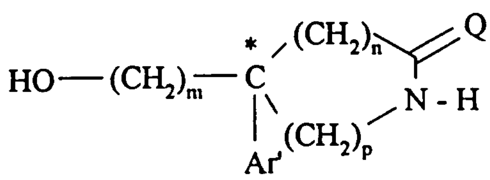

(II*, Q=H),

worin m, Ar', n, p und Q wie oben definiert sind; behandelt wird

- entweder mit einem funktionellen Derivat einer Säure der Formel

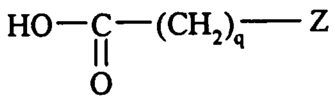

(III),

worin q und Z wie vorstehend definiert sind, wenn eine Verbindung der Formel (V*) hergestellt werden soll, worin T die Bedeutung -CO- hat,
- oder mit einem halogenierten Derivat der Formel:

$$Hal\text{-}(CH_2)_{q+1}\text{-}Z \quad (IV),$$

worin q und Z wie vorstehend definiert sind, und worin Hal ein Halogen darstellt, wenn eine Verbindung der Formel (V*) hergestellt werden soll, worin T die Bedeutung $-CH_2-$ hat, wobei die Verbindung der Formel (V*) erhalten wird, worin G Wasserstoff bedeutet, die gegebenenfalls mit Methansulfonylchlorid behandelt wird, um eine Verbindung der Formel (V*) zu erhalten, worin G eine Methansulfonyl-Gruppe ist; und

b) das so erhaltene Produkt in eines seiner Salze mit Mineral- oder organischen Säuren übergeführt wird. 11. Verfahren nach einem der Ansprüche 1, 6, 7 oder 8, dadurch gekennzeichnet, daß in der Verbindung der Formel (II) E eine Tetrahydropyran-2-yloxy-Gruppe bedeutet. 12. Verfahren nach Anspruch 9 zur Herstellung einer Verbindung der Formel (II*), worin:

- m = 2;
- Ar' = 3,4-Dichlorphenyl;
- n = 2;
- p = 1;

in optisch reiner Form (+) oder (-),

dadurch gekennzeichnet, daß diese Verbindungen durch Trennung racemischer Mischungen der Verbindungen der Formel (II), durch Kristallisation oder durch Chromatographie isoliert werden, und gegebenenfalls in eines ihrer Salze übergeführt werden. 13. Verfahren zur Herstellung einer pharmazeutischen Zusammensetzung, dadurch gekennzeichnet, daß als aktiver Bestandteil eine Verbindung der Formel (I) oder (I*), hergestellt durch das Verfahren nach einem der Ansprüche 1 bis 5, mit einem pharmazeutisch annehmbaren Träger gemischt wird.

**Patentansprüche für folgenden Vertragsstaat: GR**

1. Verbindung der Formel:

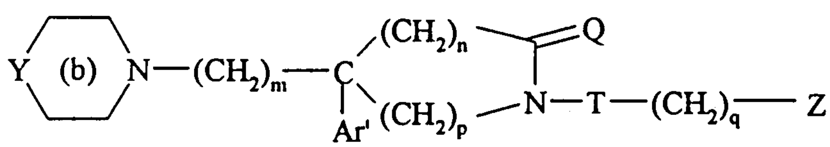

(I),

worin

- Y bedeutet:

  - entweder eine Gruppe Cy-N oder Cy-$CH_2$-N, wobei

    • Cy darstellt: ein Phenyl, unsubstituiert oder substituiert einfach oder mehrfach durch einen Substituenten, ausgewählt aus: einem Halogenatom, einem Hydroxyl, einem $C_1$-$C_4$-Alkoxy, einem $C_1$-$C_4$-Alkyl, einem Trifluormethyl, welche Substituenten gleich oder -verschieden sind; eine $C_3$-$C_7$-Cycloalkyl-Gruppe; eine Pyrimidyl-Gruppe oder eine Pyridyl-Gruppe;

  - oder eine Gruppe

X
|
$Ar-(CH_2)_x-C,$

  wobei

    • Ar bedeutet: ein Phenyl, unsubstituiert oder substituiert einfach oder mehrfach durch einen Substituenten, ausgewählt aus: einem Halogenatom, einem Hydroxyl, einem $C_1$-$C_4$-Alkoxy, einem Trifluormethyl, einem $C_1$-$C_4$-Alkyl, welche Substituenten gleich oder verschieden sind; eine Pyridyl-Gruppe; eine Thienyl-Gruppe;
    • x Null oder 1 ist;
    • X darstellt: einen Wasserstoff, ein Hydroxyl, ein $C_1$-$C_4$-Alkoxy; ein $C_1$-$C_4$-Acyloxy; ein Carboxy; ein $C_1$-$C_4$-Carbalkoxy; ein Cyano; eine Gruppe -$N(X_1)_2$, wobei die Gruppen $X_1$ unabhängig bedeuten: Wasserstoff, ein $C_1$-$C_4$-Alkyl, ein $C_1$-$C_4$-Hydroxyalkyl, ein $C_1$-$C_4$-Acyl, oder auch -$(X_1)_2$ mit dem Stickstoffatom, an das es gebunden ist, einen Heterocyclus bildet, ausgewählt aus Pyrrolidin, Piperidin oder Morpholin; eine Gruppe -S-$X_2$, wobei $X_2$ Wasserstoff oder eine $C_1$-$C_4$-Alkyl-Gruppe ist;

oder auch X mit dem Kohlenstoffatom, an das es gebunden ist, und mit dem benachbarten Kohlenstoffatom im Heterocyclus eine Doppelbindung bildet;
- m 2 oder 3 ist;
- Ar' bedeutet: ein Phenyl, unsubstituiert oder substituiert einfach oder mehrfach durch einen Substituenten, ausgewählt aus: einem Halogenatom, einem Trifluormethyl, einem $C_1$-$C_4$-Alkoxy, einem $C_1$-$C_4$-Alkyl, welche Substituenten gleich oder verschieden sind; ein Thienyl; ein Benzothienyl; ein Naphthyl oder ein Indolyl;
- n Null, 1, 2 oder 3 ist;
- p 1 oder 2 ist, und wenn p 2 ist, n 1 ist, und Q zwei Wasserstoffatome darstellt;
- Q Sauerstoff oder zwei Wasserstoffatome bedeutet;
- T eine Gruppe ist, ausgewählt aus

—C— und —$CH_2$— ;
‖
O

- q Null, 1, 2 oder 3 ist;
- Z darstellt: ein Phenyl, unsubstituiert oder substituiert durch einen oder mehrere Substituenten, ausgewählt aus: Halogen, CN, OH, $NH_2$, NH-CO-$NH_2$, $NO_2$, $CONH_2$, $CF_3$, $C_1$-$C_{10}$-Alkyl, Alkenyl mit 2 bis 10 Kohlenstoffatomen, Alkinyl mit 2 bis 10 Kohlenstoffatomen, Cycloalkyl mit 3 bis 8 Kohlenstoffatomen, Bicycloalkyl mit 4 bis 11 Kohlenstoffatomen, Hydroxyalkyl mit 1 bis 5 Kohlenstoffatomen, Alkoxy mit 1 bis 10 Kohlenstoffatomen, Alkoxyalkyl mit 2 bis 10 Kohlenstoffatomen, Alkoxyalkoxyalkyl mit 3 bis 10 Kohlenstoffatomen, Alkoxyalkoxy mit 2 bis 10 Kohlenstoffatomen, Alkenyloxy mit 2 bis 10 Kohlenstoffatomen, Alkenyloxyalkyl mit 3 bis 10 Kohlenstoffatomen, Alkinyloxy mit 2 bis 10 Kohlenstoffatomen, Alkinyloxyalkyl mit 3 bis 10 Kohlenstoffatomen, Cycloalkoxy mit 3 bis 8 Kohlenstoffatomen, Alkylthio mit 1 bis 10 Kohlenstoffatomen, Alkylthioalkyl mit 2 bis 10 Kohlenstoffatomen, Acylamino mit 1 bis 7 Kohlenstoffatomen, Acylaminoalkyl mit 2 bis 8 Kohlenstoffatomen,

Acyloxy mit 1 bis 6 Kohlenstoffatomen, Alkoxycarbonyl mit 2 bis 5 Kohlenstoffatomen, Cycloalkoxycarbonyl mit 4 bis 8 Kohlenstoffatomen, Alkylaminocarbonylamino mit 2 bis 4 Kohlenstoffatomen, Dialkylaminocarbonylamino mit 3 bis 7 Kohlenstoffatomen, (1-Pyrrolidino)-carbonylamino, Cycloalkylaminocarbonylamino mit 4 bis 8 Kohlenstoffatomen, Alkylaminocarbonylaminoalkyl mit 3 bis 9 Kohlenstoffatomen, Dialkylaminocarbonylaminoalkyl mit 4 bis 11 Kohlenstoffatomen, (1-Pyrrolidino)-carbonylaminoethyl, (1-Piperidino)-carbonylaminoethyl, Alkoxycarbonylaminoalkyl mit 3 bis 12 Kohlenstoffatomen, Cycloalkoxycarbonylaminoalkyl mit 5 bis 12 Kohlenstoffatomen, Carbamoylalkyl mit 2 bis 5 Kohlenstoffatomen, Alkylaminocarbonylalkyl mit 3 bis 9 Kohlenstoffatomen, Dialkylaminocarbonylalkyl mit 4 bis 11 Kohlenstoffatomen, (1-Pyrrolidino)-carbonylmethyl, (1-Piperidino)-carbonylmethyl, (1-Piperidino)-carbonylethyl, Cycloalkylaminocarbonylalkyl mit 5 bis 12 Kohlenstoffatomen, Alkylaminocarbonylalkoxy mit 3 bis 10 Kohlenstoffatomen, Dialkylaminocarbonylalkoxy mit 4 bis 10 Kohlenstoffatomen, (1-Piperidinyl)-carbonylmethoxy, Cycloalkylaminocarbonylalkoxy mit 5 bis 11 Kohlenstoffatomen, Cycloalkylaminocarbonylaminoalkyl mit 5 bis 12 Kohlenstoffatomen;
eine Gruppe Naphthyl oder Indenyl, deren eine oder mehrere Bindungen hydriert sein können, welche Gruppen unsubstituiert oder substituiert sein können einfach oder mehrfach durch ein Halogen, eine Gruppe Alkyl, Phenyl, Cyano, Hydroxyalkyl, Hydroxy, Oxo, Alkylcarbonylamino, Alkoxycarbonyl, Thioalkyl, wobei die Alkyle $C_1$-$C_4$ sind; eine Gruppe Pyridyl; Thiadiazolyl, Indolyl, Indazolyl, Imidazolyl, Benzimidazolyl, Chinolyl, Benzotriazol, Benzofuranyl, Benzothienyl, Benzothiazolyl, Benzoisothiazolyl, Isochinolyl, Benzoxazolyl, Benzoxazinyl, Benzodioxinvl, Isoxazolyl, Benzopyranyl, Thiazolyl, Thienyl, Furyl, Pyranyl, Chromenyl, Isobenzofuranyl, Pyrrolyl, Pyrazolyl, Pyrazinyl, Pyrimidinyl, Pyridazinyl, Indolizinyl, Phthalazinyl, Chinazolinyl, Acridinyl, Isothiazolyl, Isochromanyl, Chromanyl, deren eine oder mehrere Doppelbindungen hydriert sein können, welche Gruppen unsubstituiert oder substituiert sein können einfach oder mehrfach durch eine Gruppe Alkyl, Phenyl, Cyano, Hydroxyalkyl, Hydroxy, Alkylcarbonylamino, Alkoxycarbonyl, Thioalkyl, wobei die Alkyle $C_1$-$C_4$ sind; oder auch, wenn T -C=O- bedeutet, -$(CH_2)$q-Z auch darstellen kann: eine Benzyl-Gruppe, substituiert an -CH- durch ein Hydroxyl, ein $C_1$-$C_4$-Alkoxy, ein $C_1$-$C_4$-Alkyl, und gegebenenfalls substituiert am aromatischen Cyclus durch ein Halogen, ein Trifluormethyl, ein $C_1$-$C_4$-Alkyl, ein Hydroxyl, ein $C_1$-$C_4$-Alkoxy; oder eines ihrer gegebenenfalls vorliegenden Salze mit Mineral-oder organischen Säuren, oder wenn

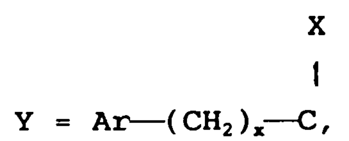

eines ihrer quaternären Ammoniumsalze mit Stickstoff (b) von Piperidin oder ein N-Oxid-Derivat mit demselben Stickstoffatom.

2. Verbindung nach Anspruch 1, in optisch reiner Form, der Formel:

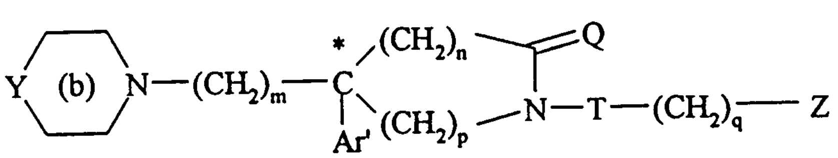
(I*),

worin

- "*" bedeutet, daß das so markierte Kohlenstoffatom die bestimmte absolute (+)- oder (-)-Konfiguration aufweist;
- Y, m, Ar', n, p, Q, T, q und Z wie in Anspruch 1 definiert sind; oder eines ihrer Salze mit Mineral- oder organischen Säuren oder mit dem Stickstoffatom (b) eines ihrer quaternären Ammoniumsalze oder ein N-Oxid-Derivat.

3. Verbindung nach einem der Ansprüche 1 oder 2, worin Z darstellt: eine Phenyl-Gruppe, unsubstituiert oder mono- oder disubstituiert durch ein Halogen oder ein Alkoxy; eine Naphthyl-Gruppe.

4. Verbindung nach einem der Ansprüche 1 bis 3 der Formel (I) oder (I*)

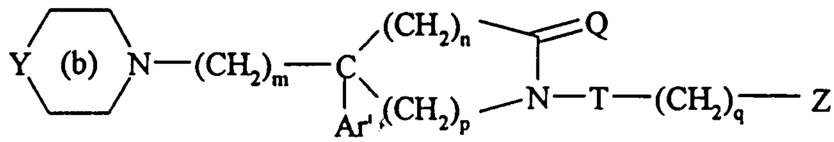

(I)

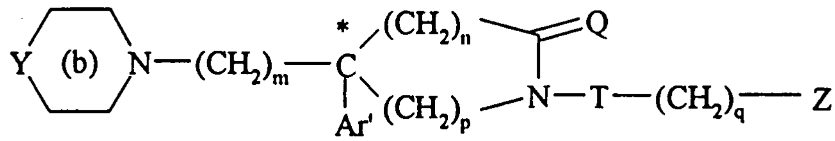

(I*)

,

worin:

Y bedeutet: eine Gruppe

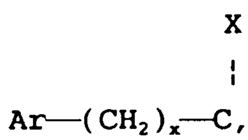

wobei Ar, x, und X wie in Anspruch 1 definiert sind;
- m, Ar', n, p, O, T, q und Z wie in Anspruch 1 definiert sind;

in Form des quaternären Ammoniumsalzes oder N-Oxid-Derivats, dadurch gekennzeichnet, daß die Gruppe

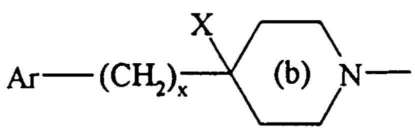

dann dargestellt wird durch die Gruppe

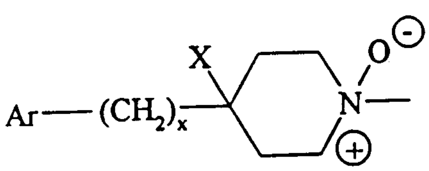

oder

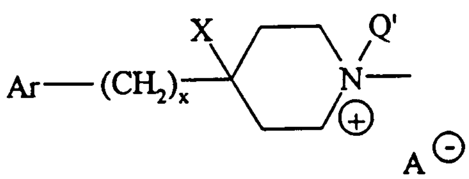

,

worin:

- Q' eine $C_1$-$C_6$-Alkyl-Gruppe oder eine Benzyl-Gruppe bedeutet; und
- $A^-$ ein Anion darstellt, ausgewählt aus Chlorid, Bromid, Iodid, Acetat, Methansulfonat oder p-Toluolsulfonat.

**5.** Verfahren zur Herstellung von Verbindungen der Formel (I) nach Anspruch 1, dadurch gekennzeichnet, daß:

a) eine Verbindung der Formel:

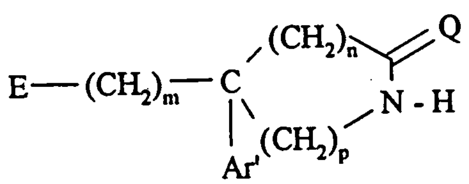

(II),

worin m, Ar', n, p und Q wie in Anspruch 1 definiert sind, und E darstellt: ein Hydroxyl oder gegebenenfalls eine O-geschützte Gruppe oder eine Gruppe:

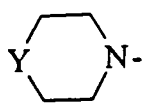

,

worin Y wie in Anspruch 1 definiert ist; mit der Maßgabe, daß: wenn Y die Gruppe

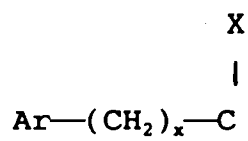

darstellt, wobei X ein Hydroxyl ist, dieses Hydroxyl geschützt werden kann; behandelt wird

- entweder mit einem funktionellen Derivat einer Säure der Formel:

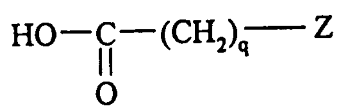

(III),

worin q und Z wie in Anspruch 1 definiert sind, wenn eine Verbindung der Formel (I) hergestellt werden soll, worin T die Bedeutung -CO- hat,

- oder mit einem halogenierten Derivat der Formel:

$$Hal\text{-}(CH_2)_{q+1}\text{-}Z \qquad (IV),$$

worin q und Z wie vorstehend definiert sind, und worin Hal ein Halogen darstellt, wenn eine Verbindung der Formel (I) hergestellt werden soll, worin T die Bedeutung $-CH_2-$ hat, wobei die Verbindung der Formel:

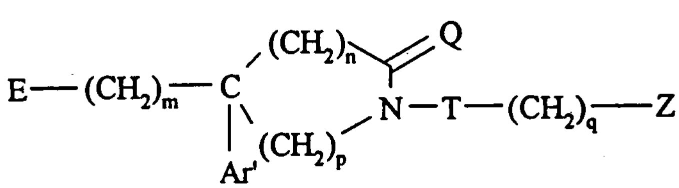

(V),

erhalten wird;

b) anschließend, wenn E eine O-geschützte Gruppe darstellt, die O-Schutzgruppe durch die Einwirkung einer Säure entfernt wird;

c) der so erhaltene Alkohol der Formel:

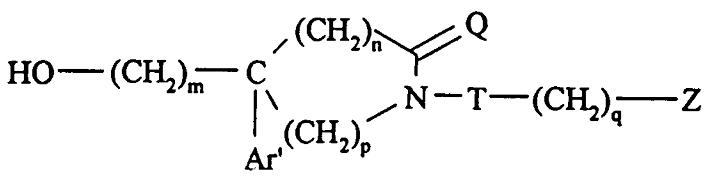

(VI)

mit Methansulfonylchlorid behandelt wird;

d) das so erhaltene Mesylat der Formel:

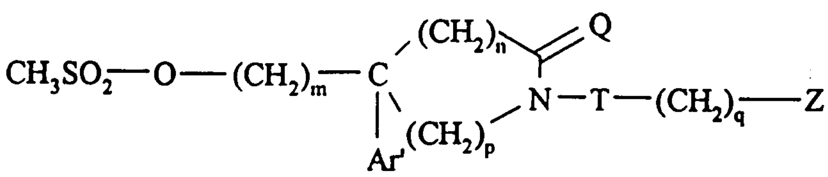

(VII)

umgesetzt wird mit einem sekundären Amin der Formel:

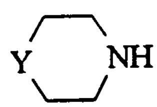

(VIII),

worin Y wie vorstehend definiert ist; und

e) nach dem gegebenenfalls durchgeführten Entschützen des durch X dargestellten Hydroxyls gegebenenfalls das so erhaltene Produkt in eines seiner Salze mit Mineral- oder organischen Säuren oder in eines seiner quaternären Ammoniumsalze mit Stickstoff (b) von Piperidin oder ein N-Oxid-Derivat mit diesem Stickstoffatom übergeführt wird.

**6.** Verbindung der Formel:

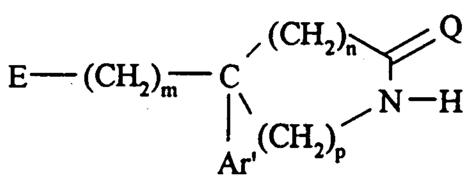

(II),

worin m, Ar', n, p und Q wie in Anspruch 1 definiert sind, und E darstellt: ein Hydroxyl oder gegebenenfalls eine O-geschützte Gruppe oder eine Gruppe:

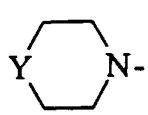

,

worin Y wie in Anspruch 1 definiert ist,

- mit der Maßgabe, daß Q Sauerstoff bedeutet:

  - wenn E = OH oder ($C_1$-$C_6$)Alkoxycarbonyloxy, m = 2 oder 3, Ar' ein durch ein ($C_1$-$C_4$)Alkoxy substituiertes Phenyl darstellt, n = 3, und p = 1;
  - wenn E = ($C_1$-$C_4$)Alkylcarbonyloxy, m = 2, n = 1, und Ar' bedeutet: ein Phenyl, unsubstituiert oder substituiert einfach oder mehrfach durch ein ($C_1$-$C_4$)Alkyl, ein ($C_1$-$C_4$)Alkoxy oder ein Halogen;

• mit der Maßgabe, daß, wenn E = OH oder ein ($C_1$-$C_4$)Alkoxy, m = 2, p = 1, Ar' darstellt: ein Phenyl, unsubstituiert oder substituiert einfach oder mehrfach durch ein ($C_1$-$C_4$)Alkyl, ein ($C_1$-$C_4$)Alkoxy oder ein Halogen, n von 1 verschieden ist;

• mit der Maßgabe, daß, wenn E = OH, Q = 2H, m = 2, n = 1, Ar' von einem unsubstituierten Phenyl verschieden ist; oder eines ihrer Salze mit Mineral- oder organischen Säuren.

**7.** Verbindung der Formel:

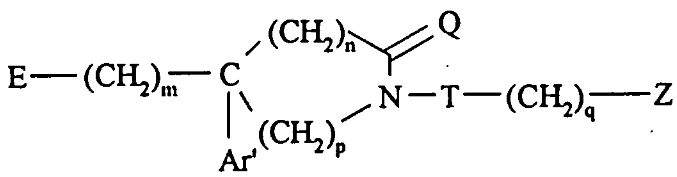

(V),

worin E ein Hydroxyl, eine O-geschützte Gruppe bedeutet, und m, Ar', n, p, Q, T und Z wie in Anspruch 1 definiert sind,

• mit der Maßgabe, daß, wenn E = OH, Q = 2H, m = 2, n = 1, p = 2, T = -$CH_2$-, q = Null, und Z = Phenyl, Ar' von einem unsubstituierten Phenyl verschieden ist; und
• mit der Maßgabe, daß, wenn E = OH, Q = 2H, m = 2, n = 1, p = 1, T = -$CH_2$-, q = Null, und Z = Phenyl, Ar' von einem Phenyl verschieden ist, unsubstituiert oder substituiert einfach oder mehrfach durch ein ($C_1$-$C_4$)Alkyl, ein ($C_1$-$C_4$)Alkoxy oder ein Halogen; und
• mit der Maßgabe, daß, wenn E = OH oder ($C_1$-$C_6$)Alkylcarbonyloxy, Q = 2H, m = 2 oder 3, n = 3, p = 1, T = -$CH_2$-, q = Null, 1, 2 oder 3, und Z eine Aryl-Gruppe bedeutet, Ar' von einem durch ein ($C_1$-$C_4$)Alkoxy substituierten Phenyl verschieden ist; oder eines ihrer Salze mit Mineral- oder organischen Säuren.

**8.** Verbindung nach Anspruch 7 der Formel:

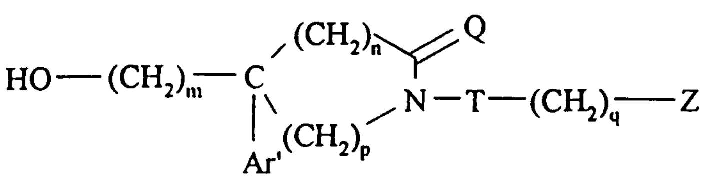

(VI),

worin m, Ar', Q, n, p, T, q und Z wie in Anspruch 1 definiert sind,

• mit der Maßgabe, daß, wenn Q = 2H, m = 2, n = 1, p = 2, T = -$CH_2$-, q = Null, und Z - Phenyl, Ar' von einem unsubstituierten Phenyl verschieden ist; und
• mit der Maßgabe, daß, wenn Q = 2H, m = 2, n = 1, p = 1, T = -$CH_2$-, q = Null, und Z = Phenyl, Ar' von einem Phenyl verschieden ist, unsubstituiert oder substituiert einfach oder mehrfach durch ein ($C_1$-$C_4$)Alkyl, ein ($C_1$-$C_4$)Alkoxy oder ein Halogen; und
• mit der Maßgabe, daß, wenn Q = 2H, m = 2 oder 3, n = 3, p = 1, T = -$CH_2$-, q = Null, 1, 2 oder 3, und Z eine Aryl-Gruppe bedeutet, Ar' von einem durch ein ($C_1$-$C_4$)Alkoxy substituierten Phenyl verschieden ist;

oder eines ihrer Salze mit Mineral- oder organischen Säuren.

**9.** Verbindung der Formel:

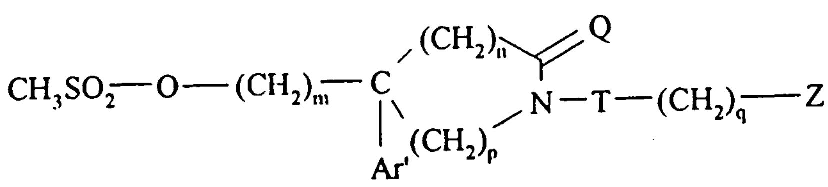

(VII),

worin m, Ar', n, p, Q, T, q und Z wie in Anspruch 1 definiert sind, oder eines ihrer Salze mit Mineral- oder organischen Säuren.

**10.** Verbindung der Formel:

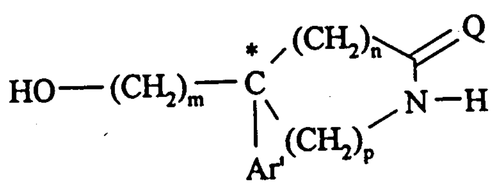

(II*),

worin m, Ar', n und p wie in Anspruch 1 definiert sind, und Q 2 Wasserstoffatome bedeutet, welche Verbindung in optisch reiner Form vorliegt, mit der Maßgabe, daß, wenn m = 2 oder 3, n = 3, und p = 1, Ar' von einem durch ein $C_1$-$C_4$-Alkoxy substituierten Phenyl verschieden ist.

**11.** Verbindung der Formel:

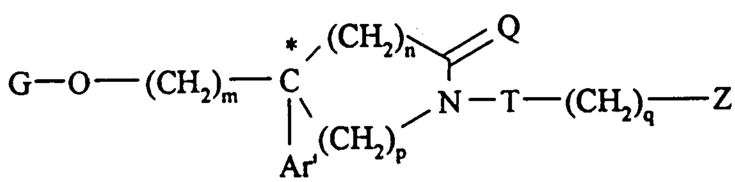

(V*),

worin G Wasserstoff oder eine Methansulfonyl-Gruppe bedeutet, m, Ar', n, p, q, T und Z wie in Anspruch 1 definiert sind, und Q 2 Wasserstoffatome darstellt, welche Verbindung in optisch reiner Form vorliegt, mit der Maßgabe, daß, wenn G Wasserstoff ist, m = 2 oder 3, n = 3, p = 1, und Z eine Aryl-Gruppe bedeutet, Ar' von einem durch ein $C_1$-$C_4$-Alkoxy substituierten Phenyl verschieden ist.

**12.** Verfahren zur Herstellung einer pharmazeutischen Zusammensetzung, dadurch gekennzeichnet, daß als aktiver Bestandteil eine Verbindung nach einem der Ansprüche 1 bis 4 mit einem pharmazeutisch annehmbaren Träger gemischt wird.

**13.** Verbindungen nach einem der Ansprüche 1 bis 4, worin Ar' und/oder Z eine Phenyl-Gruppe, substituiert einfach oder mehrfach durch ein Chlor- oder Fluoratom, darstellen bzw. darstellt.

**14.** Verfahren nach Anspruch 5, dadurch gekennzeichnet, daß in der Verbindung der Formel (II) E eine Tetrahydropyran-2-yloxy-Gruppe bedeutet.

**15.** Verbindung nach einem der Ansprüche 6 oder 7, worin E eine Tetrahydropyran-2-yloxy-Gruppe bedeutet.

**16.** Verbindung der Formel (II) nach Anspruch 6, ausgewählt aus:

- 5-Tetrahydropyranyloxypropyl-5-(3,4-dichlorphenyl)-piperidon,
- 3-Tetrahydropyranyloxypropyl-3-(3,4-dichlorphenyl)-piperidin, und
- 3-(2-Hydroxyethyl)-3-(3,4-dichlorphenyl)-piperidin.

**17.** Optisch reine Verbindung der Formel (II)* nach Anspruch 10, ausgewählt aus:

- (+)-3-(2-Hydroxyethyl)-3-(3,4-dichlorphenyl)-piperidin, und
- (-)-3-(2-Hydroxyethyl)-3-(3,4-dichlorphenyl)-piperidin.